# EUROPEAN PATENT APPLICATION

(11) **EP 1 803 814 A1**
(43) Date of publication of application: **04.07.2007**
(21) Application number: 05028501.4
(22) Date of filing: 27.12.2005
(51) Int. Cl.: C12N 15/10, C12N 15/13, C07K 16/30, C40B 40/08, C40B 50/06

(54) **Method of improving the antibody selection capacity in phage-display library**

(71) Applicant: SIGMA-TAU Industrie Farmaceutiche Riunite S.p.A., 00144 Roma (IT)
(72) Inventor: Minenkova, Olga, 00040 Montecompatri (RM) (IT); Pavoni, Emiliano, 00046 Grottaferrata (RM) (IT)

(57) **Abstract**

It is described a method of improving the antibody selection capacity in phage-display library, in which said improvement is obtained through the reduction of the expression levels of the antibodies produced in said library.

## Description

The present invention relates to a method for improving the antibody selection capacity in phage-display library, in which said improvement is obtained through the reduction of the expression levels of the antibodies produced in said library.

### Field of the invention

Recombinant DNA technology provides a cheap and useful alternative to monoclonal antibody production. Display of recombinant antibodies on bacteriophage capsid, known as phage-display, allows generation of human antibody libraries for selection of specific binders, thus providing antibodies useful for a therapy, which does not induce a harmful immune response in patients.

Display of recombinant antibodies on bacteriophage capsid also facilitates affinity maturation of antibodies through construction of mutant antibody libraries, giving clones with a higher affinity.

The possibility of finding high-affinity binders in a recombinant antibody library characterizes its quality, which depends on several factors like library size, diversity and source of immunoglobulin genes.

It is known that various lymphoid tissues from immunized or non-immunized donors, such as peripheral blood lymphocytes, spleen and bone marrow and even metastasized or drained lymph node tissue from individuals affected by tumors, may serve as a source of specific antibody repertoire.

Although naive antibody libraries are more diverse and lead to isolation of broad antibody specificities, it is reasonable to suggest that construction of a recombinant antibody library from Ig repertoire of a patient affected by specific disease can provide antibody fragments of higher binding affinity against particular antigens.

Several published studies describe construction of recombinant antibody libraries from tumor-associated lymph nodes (Clin. Exp. Immunol. 1997 109(1):166-74; Int. J. Mol. Med. 2004 14(4):729-35; World J. Gastroenterol. 2004 10(18):2619-23). These studies are based on the general idea that lymph node tissue from cancer patients are infiltrated with activated B cells, which may serve as source of tumor-specific antibodies.

It is quite difficult to obtain metastasized or drained lymph nodes from breast cancer patients as fresh surgical material. According to recent medical practice, the surgeon removes only a sentinel lymph node or a small cluster of nodes (sentinel node and those closest to it), thus performing less invasive surgery and reducing side effects, instead of removing dozen of lymph according previous surgery technique. After sentinel lymph node dissection, practically the entire node is studied for presence of micrometastasis or single cancer cells. Therefore, in breast cancer surgery the metastasized node is practically unavailable as discarded surgical material.

The evidence that tumor-infiltrating B lymphocyte-derived antibodies may recognize tumor cells was shown by producing human hybridomas, obtained from TIL, able to secrete tumor-specific antibodies (Lancet. 1982 1(8262):11-4; Br. J. Cancer, 1983 47(1):135-45); by B cell expansion of TIL from human tumor biopsies (Cancer Immunol. Immunother. 1994 38(4):225-32); by B cell expansion of melanoma-derived TIL and following cloning of the scFv antibody from single B cell clone with specific melanoma reactivity (Cancer Res. 1995 55:3584-91); and by subcutaneous transplantation of human lung cancer tissue in immunodeficient mice producing human antibodies derived from TIL-B, which recognized two tumor-specific proteins (Cancer Invest. 2000;18(6):530-6; Cancer Res. 2002 62(6): 1751-6), thus suggesting a specific function of TIL-B in the tumor.

Recently, cervical carcinoma (Cancer Immunol. Immunother. 2001 50(10):523-32) and a rare type of breast cancer, classified as medullary carcinoma (MCB), characterized by lymphoplasmacytic infiltrates and correlated with improved prognosis and patient survival (Cancer Res. 2001 61(21):7889-99; Proc. Natl. Acad. Sci. U.S.A. 2001 98(22):12659-64), were investigated to understand the nature of tumor-infiltrated B lymphocytes (TIL-B) by using also phage-display methods.

In the work of Hansen and colleagues (Proc. Natl. Acad. Sci. U.S.A. 2001 98(22): 12659-64; J. Immunol. 2002 169(5):2701-11) a Fab antibody library, derived from medullary carcinoma TIL-B, was panned on frozen tissue sections of MCB. In this work, it is reported that no antibodies against a cancer-specific antigen were selected (only antibodies against the cytoskeletal □-actin protein were selected).

In the work of Coronella and others (Cancer Res. 2001 61(21):7889-99) the TIL-derived antibody library was panned against two purified antigens, Her2/neu and p53, and on the MCB cell line. Also in this work the authors failed to identify tumor-specific antibodies from the library, suggesting that the neoantigen, which elicits antigen-driven maturation of B-cells inside the tumor mass, either was not present on the cell surface or was expressed at low levels. And finally, one year later, this group identified 3 antibodies recognizing tumor cells from this type of library (J. Immunol. 2002 169:1829-36).

Additionally, Hoogenboom's group (Int. J. Cancer 2001 93:832-40) has constructed two Fab libraries derived from TIL and from drained lymph node from colorectal cancer patient. Although a large panel of antibodies reactive with primary tumors was obtained from these immune repertoires, none of these antibodies discriminated between tumor and normal cells, none were reactive with cell-surface antigens.

Random analysis of IgG heavy chain sequences revealed strong oligoclonal immune response in TIL-B. In comparison with peripheral blood lymphocytes, where clonality is very low, tumor-draining lymph nodes contains more limiting repertoire, containing about 7% of clonal sequences out of total IgG. TIL-B present extremely restricted antibody repertoire. IgG sequences from TIL-B belonging to clonal groups range between 18%-68% (Cancer Immunol. Immunother. 2003 52:715-738).

In spite of the fact that TIL-B present very limiting antibody repertoire and the molecular structure of variable gene regions of TIL-derived antibodies reveals evidence of antigen-driven humoral immune responses in studied tumors, selection of tumor-specific antibodies from TIL-derived libraries often failed or demonstrated a very limited success.

With regard to TIL-B cell expansion and production of tumor specific antibodies by immortalized tumor-infiltrated lymphocytes mentioned above, B cells transformed with EBV have (i) limited capacity of growth; (ii) limited capacity of antibody production; (iii) problems of stability; and finally, (iv) laboratory personnel using this technique run the danger of EB virus infection.

In spite of the not very encouraging results obtained by other research groups, the applicant continued the study the recombinant antibody libraries derived from TIL-B.

### Description of the invention

We have now found that it is possible to modify the phagemid vectors known in the art for improving the capacity of the phage-display system for the selection and maturation of recombinant antibodies. An example of phagemid vectors known in the art can be found in "Antibody Engineering - A practical approach (McCafferty, J. Hoogenboom, H. & Chiswell D., eds), pp.325, Oxford University Press, 1996)".

It is therefore an object of the present invention a method for improving the capacity of the phage-display system for the selection and maturation of recombinant antibodies, in which said improvement comprises the following steps:
(i) introduction in a phagemid vector of elements that reduce the expression levels of the antibodies produced, in which said reduction of the expression of the antibodies produced is obtained:
   by introducing a suppressed stop codon inside the leader peptide gene (as described in Example 1); or
   by introducing a suppressed stop codon inside the antibody gene (for example using a method described in Sambrook J, Fritsch EF, Maniatis T. Molecular cloning: A laboratory manual. Cold Spring Harbor Laboratory Press, Cold Spring Harbor, 1989); or
   by introducing low-efficient or repressible promoters directing the expression of recombinant antibodies in the phagemid vector [Biochem. J. 1970, 118:741-746; or in J. Bacteriol. 1987, 169(10):4457-62; Science. 1975, 187(4171):27-35)]; or
   by using suitable inhibitors for promoters [an example of suitable inhibitors can be found in J. Bacteriol. 1979, 138(1):40-7; or in Proc. Natl. Acad. Sci. U.S.A. 1997, 94(24):12914-9)] in the phagemid vectors known in the art; and / or
(ii) fusion of single-chain antibody genes only to carboxy-terminal part of pIII protein (described in Example 1); and/or
(iii) use of the leader peptide gene of the alkaline phosphatase [(the genuine periplasmic protein of *E.coli*, which guarantees the efficient processing, transport and assembly of antibodies on phage particle in *E.coli* expression system) (described in Example 1)].

It is a further object of the present invention a phagemid vector, having improved capacity of selection and/or maturation of recombinant antibodies, characterized in that it contains elements that reduce the expression levels of the antibodies produced and/or has an improved efficiency of display of the antibody produced, in which said improved capacity is obtained:
(i) by introducing a suppressed stop codon inside the leader peptide gene; or by introducing a suppressed stop codon inside the antibody gene; or by introducing low-efficient or repressible promoters in the phagemid vector; or by using suitable inhibitors for promoters in the phagemid vectors known in the art, as mentioned above; and/or
(ii) by fusing the single-chain antibody genes only to carboxy-terminal part of pIII protein; and/or
(iii) by the use of the leader peptide gene of the alkaline phosphatase of *E. coli.*

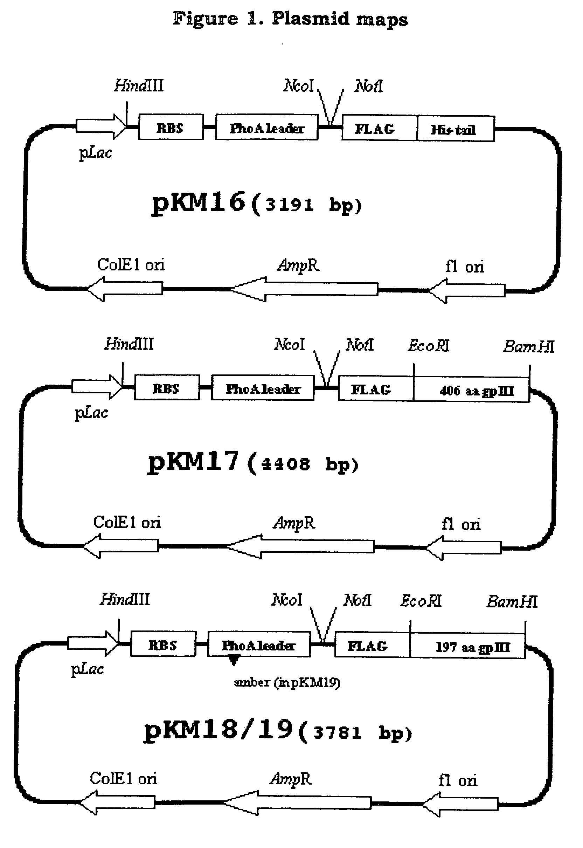

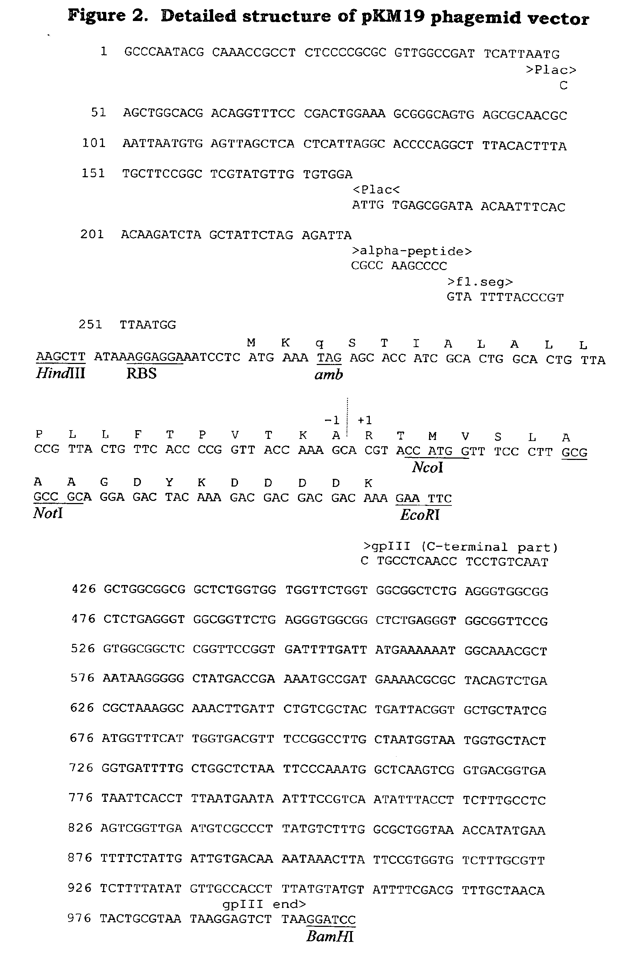

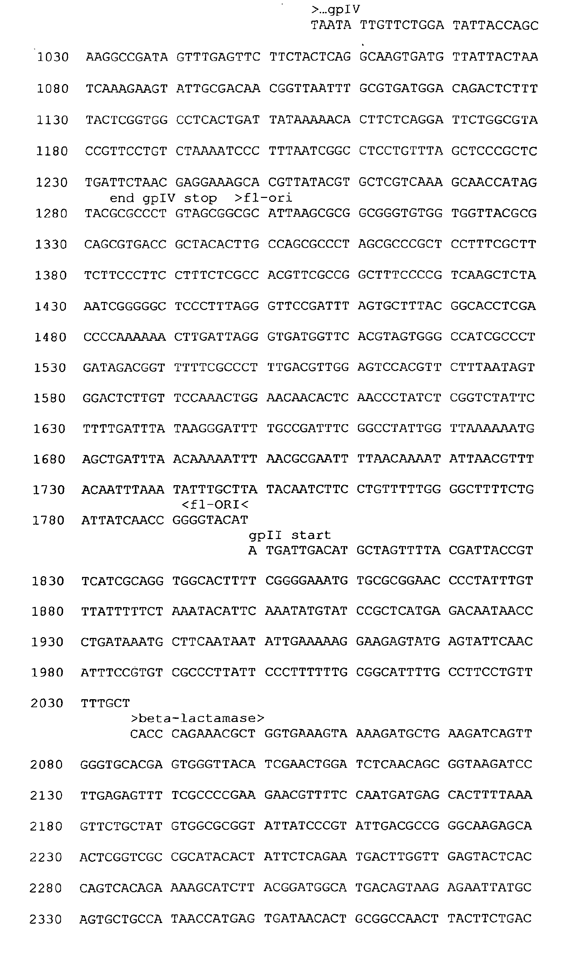

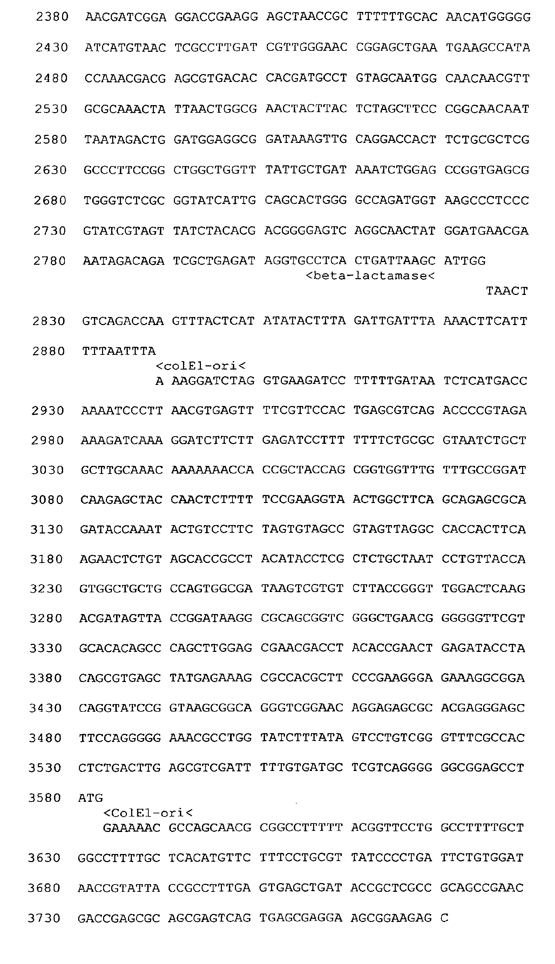

It is a further object of the present invention a phagemid vector, pKM 19, having the nucleotide sequence of SEQ ID NO: 1; the plasmid maps shown in Figure 1; and the detailed structure of modification reported in Figure 2.

This vector is designed for the display of recombinant antibodies in single-chain format on the surface of filamentous phage.

It is a further object of the present invention a phage display-antibody library which is constructed by using the phagemid vector having improved capacity of selection and/or maturation of recombinant antibodies above mentioned.

It is a further object of the present invention a phage-display antibody library which is constructed using the phagemid vector having improved capacity of selection and/or maturation of recombinant antibodies, in which said phage-display antibody library is selected from the group comprising:
ScFvB87; ScFvB95; ScFvB96; scFvmix; or scFvEC23.
It is a further object of the present invention an antibody obtained using a phage-display antibody library which is constructed by using the phagemid vector having improved capacity of selection and/or maturation of recombinant antibodies.
It is a further object of the present invention an antibody, obtained using a phage-display antibody library selected from the group comprising: ScFvB87; ScFvB95; ScFvB96; scFvmix; or scFvEC23; in which said library is constructed using the phagemid vector having improved capacity of selection and/or maturation of recombinant antibodies;
in which said antibody recognize an antigen selected from the group comprising: ED-B protein domain of fibronectin, MUC1 tumor antigen, CEA (carcino-embrionic antigen), or MCF7 breast carcinoma;
in which said antibody is selected from the group comprising:
EDE1 scFv antibody, recognizing the ED-B protein domain of fibronectin, having the nucleotide sequence of SEQ ID NO: 2, and the amino acid sequence of SEQ ID NO: 3;
EDE3 scFv antibody, recognizing the ED-B protein domain, having the nucleotide sequence of SEQ ID NO: 4, and the amino acid sequence of SEQ ID NO: 5;
EDE5 scFv antibody, recognizing the ED-B protein domain, having the nucleotide sequence of SEQ ID NO: 6, and the amino acid sequence of SEQ ID NO:7;
EDB5 scFv antibody, recognizing the ED-B protein domain, having the nucleotide sequence of SEQ ID NO: 8, and the amino acid sequence of SEQ ID NO:9;
ME1 scFv antibody, recognizing the MUC1 tumor antigen, having the nucleotide sequence of SEQ ID NO: 10, and the amino acid sequence of SEQ ID NO: 11;
ME2 scFv antibody, recognizing the MUC1 tumor antigen, having the nucleotide sequence of SEQ ID NO: 12, and the amino acid sequence of SEQ ID NO: 13;
MB5 scFv antibody, recognizing the MUC1 tumor antigen, having the nucleotide sequence of SEQ ID NO: 14, and the amino acid sequence of SEQ ID NO: 15;
CB3 scFv antibody, recognizing the CEA (carcino-embrionic antigen), having the nucleotide sequence of SEQ ID NO: 16, and the amino acid sequence of SEQ ID NO: 17;
CB37 scFv antibody, recognizing the CEA antigen, having the nucleotide sequence of SEQ ID NO: 18, and the amino acid sequence of SEQ ID NO: 19;
CB41 scFv antibody, recognizing the CEA antigen, having the nucleotide sequence of SEQ ID NO: 20, and the amino acid sequence of SEQ ID NO: 21;
CB53 scFv antibody, recognizing the CEA antigen, having the nucleotide sequence of SEQ ID NO: 22, and the amino acid sequence of SEQ ID NO:23;
B96/11L scFv antibody, recognizing the MCF7 breast carcinoma cells, having the nucleotide sequence of SEQ ID NO: 24, and the amino acid sequence of SEQ ID NO:25;
mix7 scFv antibody, recognizing the MCF7 breast carcinoma cells, having the nucleotide sequence of SEQ ID NO: 26, and the amino acid sequence of SEQ ID NO: 27;
mix8 scFv antibody, recognizing the MCF7 breast carcinoma cells, having the nucleotide sequence of SEQ ID NO: 28, and the amino acid sequence of SEQ ID NO: 29;
mix11 scFv antibody, recognizing the MCF7 breast carcinoma cells, having the nucleotide sequence of SEQ ID NO: 30, and the amino acid sequence of SEQ ID NO: 31;
mix12 scFv antibody, recognizing the MCF7 breast carcinoma cells, having the nucleotide sequence of SEQ ID NO: 32, and the amino acid sequence of SEQ ID NO: 33;
mix17 scFv antibody, recognizing the MCF7 breast carcinoma cells, having the nucleotide sequence of SEQ ID NO: 34, and the amino acid sequence of SEQ ID NO: 35;
mix23 scFv antibody, recognizing the MCF7 breast carcinoma cells, having the nucleotide sequence of SEQ ID NO: 36, and the amino acid sequence of SEQ ID NO: 37;
mix25 scFv antibody, recognizing the MCF7 breast carcinoma cells, having the nucleotide sequence of SEQ ID NO: 38, and the amino acid sequence of SEQ ID NO:39;
mix39 scFv antibody, recognizing the MCF7 breast carcinoma cells, having the nucleotide sequence of SEQ ID NO: 40, and the amino acid sequence of SEQ ID NO: 41.

It is a further object of the present invention a method of producing an antigen or antigen fragment, in which said method is characterized by the following steps:
a) Purification of suitable lymphocytes, if any;
b) RNA extraction and cDNA synthesis;
c) Amplification of the antibody genes using known oligonucleotide primers;
d) "in vitro" assembly of the antibody genes;
e) Generation of antibody-displayed libraries using the phagemid vector having improved capacity of selection and/or maturation of recombinant antibodies according to the present invention;
f) Phage amplification;
g) Selection of new antibody specificities against purified protein antigen, comprising purified proteins from natural sources or recombinant proteins obtained by correspondent gene amplification, cloning and protein production;
h) Production of the selected antibodies in soluble form using a suitable plasmid vector known in the art;
i) Identification of specific peptide antigens by selection of peptide from random peptide library or from peptide library derived from DNA encoding target protein gene through selection by using soluble antibody fragments;
j) Alternatively, after step "f" (phage amplification), selection of new antibody specificities by using complex multi-component biological structures, like living cells, as described in example 2;
k) Repeat step "h" (Production of the selected antibodies in soluble form using a suitable plasmid vector known in the art);
l) Identification of new disease-specific targets, for example, new tumor surface antigens, by using novel anti-tumor antibodies recognizing tumor cells through: (i) immunoprecipitation of unknown target proteins from tumor cell extracts (Antibodies. A laboratory manual. Ed Harlow, David Lane, Cold Spring Harbor, NY: Cold Spring Harbor Laboratory Press, 1988); or (ii) developing the immunoreactions with tumor cell extract, separated by two-dimensional PAGE (Proteins and proteomics: A laboratory manual. Richard J. Simpson, pp.705, Science 2002) and transferred onto nitrocellulose membrane (Sambrook J, Fritsch EF, Maniatis T. Molecular cloning: A laboratory manual. Cold Spring Harbor Laboratory Press, Cold Spring Harbor, 1989);
m) Protein sequencing and identification of new antigens.

The antigen obtained according the methods reported above can be useful for producing vaccines, diagnostic reagents or in the research field.

It is a further object of the present invention the DNA, which encodes for the antibodies above mentioned.

It is a further object of the present invention a vector for the expression of the antibody which contains the DNA, which encodes for the antibodies above mentioned.

It is a further object of the present invention a cell tranfected with the vector of the invention for the expression of the antibody, which contains the DNA, which encodes for the antibodies above mentioned.

It is a further object of the present invention a process for the production of the recombinant antibody above mentioned, which comprises the cultivation of the cell tranfected with the vector for the expression of the antibody, which contains the DNA, which encodes for said antibodies, and isolation of the antibodies produced.

It is a further object of the present invention a pharmaceutical composition comprising as active ingredient one or more recombinant antibody above mentioned, and optionally one or more excipients or diluents pharmaceutically acceptable and well known in the art.

It is a further object of the present invention a pharmaceutical composition comprising as active ingredient one or more disease-specific antigen above mentioned, and optionally one or more excipients or diluents pharmaceutically acceptable and well known in the art.

It is a further object of the present invention a diagnostic reagent, comprising one or more recombinant antibody above mentioned, useful for the diagnosis of infective, inflammatory, tumoral, metabolic, autoimmune or allergic diseases.

It is a further object of the present invention a diagnostic reagent, comprising one or more specific antigen above mentioned, useful for the diagnosis of infective, inflammatory, tumoral, metabolic, autoimmune or allergic diseases.

It is a further object of the present invention the use of a phagemid vector according to the present invention, for the preparation of recombinant antibody libraries;
in which:
said library is selected from the group comprising synthetic or semisynthetic antibody libraries or mutated antibody gene libraries for affinity maturation of antibodies;
said libraries are useful for producing antibodies specific for a selected antigen;
said antibodies can be used for the selection of specific antigen;
said antibodies are in single or double-format.

It is a further object of the present invention the use of the recombinant antibodies above mentioned for preparing a medicament for the therapy of infective, inflammation, tumoral, metabolic and autoimmune or allergic diseases, in human and animals.

It is a further object of the present invention the use of the recombinant antibodies above mentioned for preparing diagnostic reagents for infective, inflammatory, tumoral, metabolic, autoimmune or allergic diseases.

It is a further object of the present invention the use of the recombinant antibodies above mentioned for preparing reagents for research use.

It is a further object of the present invention the use of the recombinant antibodies above mentioned for the identification of novel disease-specific antigens.

It is a further object of the present invention the use of the novel disease-specific antigens above mentioned, for preparing vaccines for the prevention or therapy of infective, inflammation, tumoral, metabolic, autoimmune or allergic diseases.

It is a further object of the present invention the use of the novel disease-specific antigens above mentioned, for preparing diagnostic reagents for infective, inflammation, tumoral, metabolic, autoimmune or allergic diseases.

It is a further object of the present invention the use of the novel disease-specific antigens above mentioned, for preparing reagents useful in the research field.

### Detailed description of the drawings

**Figure 1**. It is schematically described the pKM16 plasmid useful for the production of soluble antibodies in scFv format and pKM17, pKM18 and pKM 19 plasmids useful for production of phage-displayed antibodies. These plasmids direct antibody expression under control of pLac promoter. The unique *Nco*I and *Not*I cloning sites allow insertion of an antibody gene to express single-chain antibodies with a leader peptide of the bacterial periplasmic enzyme, alkaline phosphatase (PhoA leader). Plasmid pKM17 and plasmids pKM18 and pKM19 encode entire protein pIII (406 aa) or only carboxy-terminal part of pIII (197 aa), respectively. Plasmid pKM 19 contains amber codon in PhoA leader.
**Figure 2.** It is described the detailed structure of modification of pKM19 phagemid vector. The specific modification made are reported in the figure and described in the text.
**Figure 3.** Soluble scFv production by using pKM16 plasmid. Three independent clones obtained by cloning scFv anti-CEA gene in pKM16 were tested for soluble scFv production (gel lines 1-3). Periplasmic protein fractions were purified from bacteria by freeze and thaw method. The protein size marker is included (line 1). Western blot membrane was developed with an anti-FLAG AP-conjugated secondary antibody. Bands corresponding to soluble scFv antibodies (expected molecular weight 25.4 kDa) migrate between 24.5 and 35.9 kDa bands.
**Figure 4.** Display efficiency of pKM17 and pKM18 plasmids. Single clones obtained by cloning the anti-CEA scFv gene in two plasmids, were assayed by ELISA. Irrelevant GST protein was included as negative control. Data reported are the average values of assays performed in duplicate.
**Figure 5.** Selection on SP2-GST protein. Reactivity of phage pools derived from first and second round of panning. GST protein, presenting as protein carrier in the selection process is included as a negative control. Data reported are the average values of assays performed in duplicate.
**Figure 6.** Affinity selection of maturated anti-CEA scFv antibody from mutated library. In this assay the positive immunoreactions were developed by an anti-FLAG HRP-conjugated secondary antibody in order to moderate positive signals and make visible growing reactivity in selection process. Data reported are the average values of assays performed in duplicate.
**Figure 7.** V(D)J analysis of TIL-derived antibody genes. A. SMART cDNAs derived from 10 different tumor samples (patients B84, B85, B87, B89, B90, B91, B92, B93, B95, B96), from normal breast, normal testis and lymphocytes from four healthy donors (L1, L2, L3, L4), were used, as template for amplification of V(D)J antibody regions. cDNAs were normalized by amplification of □-actin housekeeping gene. V(D)J fragments were amplified well from all templates excluding normal testis cDNA. B. The same PCR products were fractionated by PAGE giving a higher resolution for DNA bands.
**Figure 8.** Antibody subclass distributions. PCR-amplified normal breast and B84 cDNA samples not showing oligoclonal bands in V(D)J test, have prevalence of IgA bands in comparison to IgG1 and IgG2 (left panel), while three samples diving strong oligoclonal bands in previous test, have IgG1 or both IgG band prevalence in comparison with IgA (right panel).
**Figure 9.** Amino acid sequences of variable regions of 30 random clones obtained by cloning □-chain antibody genes derived from B92 and B93 cDNAs. Peptide sequence is reported in single-letter code. Identical amino acids a in similar clones are represented by a dash.
**Figure 10.** Selection on ED-B, MUC1 and CEA proteins. Reactivity of phage pools derived from second and third rounds of panning in comparison with original libraries were tested. GST is included as a negative control. Additional negative control, protein D possessing 6His tail as a target protein used in the selection was used in case of ED-B panning. Data reported are the average values of assays performed in duplicate.
**Figure 11.** ELISA reactivity of single phage clones displayed scFv antibodies. Data reported are the average values of assays performed in duplicate. An anti-SP2 irrelevant phage antibody is included as negative control.
**Figure 12.** Cell-based panning. Reactivity of phage pools derived from fourth and fifth rounds of panning in comparison with original libraries were tested Data reported are the average values of assays performed in triplicate.
**Figure 13.** Cell-ELISA reactivity of single phage clones. Data reported are the average values of assays performed in triplicate. Cell developing with irrelevant anti-SP2 antibody is included as negative control.
**Figure 14.** Fluorescent staining of breast carcinoma MCF7 and normal breast epithelium MCF10-2A fixed cells by phage-displayed scFv antibodies (mix17 (A), mix7 (B)).
**Figure 15.** A. Fluorescent staining of breast carcinoma cells MCF7, ScBr3 expressing MUC1 tumor antigen and normal breast epithelium cells MCF10-2A by using phage-displayed anti-MUC1 MB5 scFv antibody; B. Staining of colorectal adenocarcinoma cells LoVo expressing CEA by phage-displayed anti-CEA CB37 scFv antibody. Staining of negative control MCF10-2A cells is included.

The following non limiting examples further illustrates the invention.

### EXAMPLE 1

### Construction of novel pKM 19 phagemid vector for the display of single-chain antibodies on filamentous phage.

### Introduction

A library of recombinant antibodies constructed from natural immunoglobulin repertoire contains millions of various antibody molecules having particular capacity to recognize and bind enormous number of antigens. Therefore, all antibodies interacting with bacterial proteins may affect to bacterial growth or be toxic for host bacteria used for antibody expression.

Recently applicant has developed a high affinity single-chain anti-CEA antibody E8, by maturation of an original anti-CEA MA39 antibody selected from the ETH-2 scFv synthetic library (Methods Enzymol. 2000, 326:480-505). A maturation library was constructed by cloning mutated scFv gene in pDN332, a vector known in the art (J. Biol. Chem. 1998, 273(34):21769-76). Several phage clones with increased reactivity were selected, however, the expression of the soluble maturated antibody in bacteria failed. It was discovered that new mutated genes of single chain antibodies contain TAG and TGA codons in 70% of phage clones sequenced. Suppression of TAG codon in a suppressing strain DH5□F' and TGA by natural suppression (suppression of nonsense mutation with native, not mutant tRNA) (Microbiol Rev. 1989 53(3):273-98) allowed the selection of a highly reactive phage-displayed antibody, impossible to produce in soluble form without additional manipulations, aimed at substituting the stop codons in antibody gene sequence.

Accumulation of stop codons in antibody genes during selection process indicated a strong biological bias against production of the anti-CEA antibody in bacteria.

In the present work a novel phagemid vector, pKM19, providing a low antibody expression level was developed. This vector counterbalances the probability for the antibodies, which are neutral and harmful to bacterial host, to be selected from the library.

In the present work an amber codon positioned in a sequence encoding for a phosphatase alkaline leader peptide used in this construction that leads to a relatively lower expression level of recombinant antibody in the suppressing bacteria harboring this plasmid, was introduced. This facilitates avoidance of abundant protein production, which would result in rapid acquisition of different mutations, reducing antibody expression levels (i.e., stop codons, frame-shifts) where harmful antibodies interfere with vital bacterial functions, allowing such antibodies to be selected from the library. In spite of the low expression level of recombinant antibodies, functional tests demonstrate that (i) the present level of antibody production is sufficient to produce highly reactive phage antibodies giving a saturated signal in ELISA test, (ii) specific antibodies can be easily isolated, already after two selection rounds, from scFv library constructed from peripheral blood lymphocytes of the patient who had antibodies against target protein.

Use of pKM 19 vector for maturation of anti-CEA antibody significantly improved selection results. None of the 19 sequenced cloned with increased affinity had any stop codon within antibody genes and could easily be recloned in pKM16 for soluble antibody production, whereas 70% of clones contained such mutations when the classic phagemid system was used in analogous work.

The pKM19 vector allows the cloning of scFv fragments as N-terminal fusion of deleted gene III protein. The pIII is a protein of 406 aa residues, composed of two functional domains [Virology. 1984; 132(2):445-55]. N-terminal domain is required for bacteriophage infectivity, while C-terminal domain is incorporated into the virion and is responsible for generation of unit-length particles. Commonly used phage-display vectors for scFv lead to incorporation into phage particle of the entire pIII fused to antibody fragment, while in the case of pComb3 plasmid utilized for Fab display [Proc. Natl. Acad. Sci. U.S.A. 1991; 88(18) : 7978-82], the antibody fragment is fused to the carboxyl-terminal half of the pIII. Infectivity of such recombinant phages is obtained during their propagation, since superinfection with a helper phage provides the native gene III protein. According to present data, fusion of the single-chain antibody to the C-terminal part of pIII improved display of antibody in comparison with the entire protein fusion.

In bacteria harboring pKM19 vector, after synthesis of the recombinant protein, the PhoA leader peptide is cleaved off by leader peptidase upon membrane translocation and scFv-pIII is assembled into the phage particle. In this way, the entire cleavage site of the alkaline phosphatase, genuine periplasmic protein of *E.coli,* is preserved to guarantee efficient and correct processing. As the result, the mature protein contains two additional amino acids at the N-terminus of scFv. In this system, for the subsequent production of soluble antibodies needed to reclone the antibody gene in the appropriate plasmid, thus, on this step the additional amino acids can be conserved or eliminated according to specific requirements.

In conclusion, novel pKM 19 phagemid is useful for selection of recombinant scFv antibodies against desired targets as well as for their affinity maturation. The plasmid guarantees efficient display and allows reduction of biological bias for "difficult" antibodies in the delicate step of initial selection. Moreover, this vector is particularly useful for affinity maturation of antibodies, since high expression levels may increase avidity of phage particles displaying Ab, leading to selection of antibodies with only modest affinity.

### METHODS

### Bacterial strains

Bacterial strain DH5□F' *(supE44* □*lac*U169 (□80 *lacZ*□M15) *hsdR*17 *rec*A1 *endAlgyrA96 thi-1 rel*A1 F' [*traD36 proAB⁺ lacI*^{q}*lacZ□M15*]) was used for soluble and phage antibody production.

### Construction of plasmids

The pC89 plasmid [J. Mol. Biol. 1991 222(2):301-10] was amplified by inverse PCR with the KM161, KM162 oligonucleotides, containing *Hind*III and *Not*I sites (underlined) (KM 161 5'-GAGGAAGCTTCCATTAAACGGGTAAAATAC-3'; KM 162 5'-TGCAATGGCGGCCGCTAATATTGTTCTGGATATTACCAGC-3'). In inverse PCR a *Taq* polymerase mixture with *Pfu* DNA polymerase was used to increase fidelity of DNA synthesis. Twenty-five cycles of amplification (95°C-30sec, 55°C-30sec, 72°C-20min) were done. The PCR product was digested with *Hind*III and *Not*I endonucleases and ligated with a KM163-KM164 oligonucleotide duplex encoding FLAG peptide and His-tail (KM 163 5'-AGCTTCCTC ATG TAG GCG GCC GCA GGA GAC TAC AAA GAC GAC GAC GAC AAA CAC CAC CAT CAC CAC CAT TAA-3'; KM 164 5'- GGCC TTA ATG GTG GTG ATG GTG GTG TTT GTC GTC GTC GTC TTT GTA GTC TCC TGC GGC CGC CTA CAT GAGGA-3'). The cloned DNA duplex contained an internal *Not*I site, upstream of FLAG peptide encoding sequence, while the *Not*I site, used for cloning of the duplex, was not restored. The resulting pKM15 plasmid was newly digested with *Hind*III, *Not*I endonucleases and ligated with KM175-KM176 duplex encoding the leader sequence and the first two amino acids of the PhoA bacterial protein, containing the *Nco*I cloning site (KM 175 5'-AGC TTA TAA AGG AGG AAA TCC TCA TGA AAC AGA GCA CCA TCG CAC TGG CAC TGT TAC CGT TAC TGT TCA CCC CGG TTA CCA AAG CAC GTA CCA TGG TTT CCC TTGC-3'; KM 176 5'-GGC CGC AAG GGA AAC CAT GGT ACG TGC TTT GGT AAC CGG GGT GAA CAG TAA CGG TAA CAG TGC CAG TGC GAT GGT GCT CTG TTT CAT GAG GAT TTC CTC CTT TATA-3'). This new pKM16 plasmid was destined for soluble single-chain antibody production (Figure 1).

Plasmid pKM16 was amplified by inverse PCR with the KM181, KM182 oligonucleotides, presenting *EcoR*I and *BamH*I restriction sites, respectively (KM 181 5'- GTG GTG ATG GAA TTC TTT GTC GTC GTC GTC TTT GTA GTC-3'; KM 182 5'- CAC CAT TAA GGA TCC TAA TAT TGT TCT GGA TAT TAC CAG C-3'). Full-length gene III or 3' part of the gene encoding the last 197 aa of the pIII were amplified by using the oligonucleotides KM183-KM185 or KM184-KM185 containing *BamH*I and *EcoR*I sites (underlined) and ligated into digested pKM16, giving plasmid pKM17 or pKM18, respectively (KM 183 5'- TC TAT TCT GAA TTC GCT GAA ACT GTT GAA AGT TGT TTA GC-3'; KM 184 5'- GC CAA TCG GAA TTC CTG CCT CAA CCT CCT GTC AAT GCT-3'; KM185 5'- GAA CTG GGA TCC TTA AGA CTC CTT ATT ACG CAG TAT G-3').

A fragment of the pKM 18 plasmid encoding the leader sequence was PCR-amplified with KM186-KM180 primers, introducing an amber mutation in PhoA leader peptide gene (KM 186 5'- ACC CGT AAG CTT ATA AAG GAG GAA ATC CTC ATG AAA TAG AGC ACC ATC GC-3'; KM 180 5'- TAG CCC CCT TAT TAG CGT TTG-3'). The resulting PCR product was digested with *Hind*III and *Not*I and cloned into pKM 18, digested with *Hind*III and *Not*I and purified from agarose, to construct pKM 19.

### Soluble antibody production

A single colony was inoculated into 50 mL of LB containing 100 □g/mL Ap and 2% glucose. Culture was grown at 37°C for 2-3 h up to O.D.=0.8. Cells recovered by centrifugation were resuspended in 50 mL of LB with Ap and 1 mM IPTG and incubated overnight at 30- 32°C. Cell pellet was resuspended in 500 □L of PBS. After three cycles of freeze and thaw, cell debris was pelleted by centrifugation. The resulting supernatant was used for ELISA or for Western blot.

### Purification of lymphocytes from peripheral blood and cDNA synthesis

The lymphocytes were isolated from 10 mL of fresh peripheral blood with anticoagulant using Ficoll-Paque Plus (Amersham Pharmacia Biotech, Sweden) according to manufacturer's instructions. mRNA was isolated from lymphocytes by using Dynabeads mRNA DIRECT Kit (Dynal, Norway). One □g of the poly(A)⁺ RNA from the lymphocytes was used to synthesize full-length cDNA by using SMART cDNA Library Construction Kit (Clontech, Palo Alto, CA).

### scFv library construction

The antibody gene repertoire was amplified using a set of primers designed for amplification of VH and VL antibody domains, while entire scFv fragments were assembled *in vitro* as it was described in [Pope, A.R., Embleton, M.J. & Mernaugh R. (1996) Construction and use of antibody gene repertoires. In: Antibody Engineering - A practical approach (McCafferty, J., Hoogenboom, H. & Chiswell D., eds), pp.325, Oxford University Press]. The latter were then amplified by PCR with appropriate extension primers, incorporating *Nco*I, *Not*I restriction sites, and allowing the cloning of scFv genes into a pKM 19 vector. The resulting PCR products were purified on 1% low-melting agarose gel (NuSieve 3:1 agarose, Rockland, ME), cut with *Nco*I/*Not*I and inserted into digested plasmid. The transformed library contained 1.77x10⁷ independent clones with full-length scFv insert.

### Construction of mutated anti-CEA scFv library

The maturation library for the anti-CEA scFv was constructed as following. Briefly, mutated scFv gene fragments were generated by PCR amplification with primers: KM 144-KM 143 (KM 143, 5'-GTCATCGTCGGAATCGTCATCTGC-3'; KM 144, 5'-TGTGCGAAAAGTAATGAGTTTCTTTTTGACTACTGGGGC-3') and KM148-KM145 (KM148, 5'-CTATTGCCTACGGCAGCCGCTGGA-3'; KM145, 5'-TCCGCCGAATACCACATAGGGCAACCACGGATAAGAGGAGTTACAGTAATAGT CAGCC-3') introducing random mutations in CDR3 regions of heavy or light chains with low frequency. Each underlined base of KM144 and KM145 oligonucleotides was replaced with mixture of G/A/T/C with a frequency of 10%. Missing scFv antibody gene parts were amplified with KM148-KM157 and KM158-KM143 primers for HC and LC, respectively (KM157 5'-TTT CGC ACA GTA ATA TAC GG-3'; KM158 5'-TAT GTG GTA TTC GGC GGA-3').

In order to reconstruct the entire gene, the corresponding fragments were combined and amplified in PCR-like process without oligonucleotide primers.

The resulting product was utilized to amplify entire gene with external primers KM148, KM143. The final DNA fragment was agarose-purified, digested with restriction enzymes *Nco*I and *Not*I*,* and ligated with digested plasmid pKM19. The resulting library contained 2.2x10⁶ mutated antibody clones.

### Results

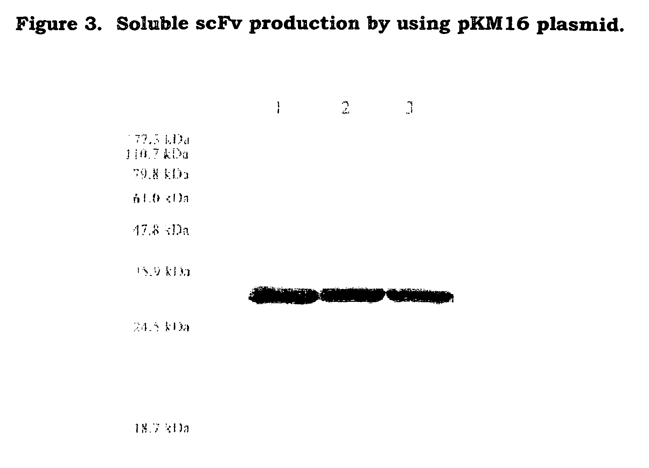

The pKM16 plasmid (Figure 1) for production of soluble antibodies in scFv configuration was constructed as described above. This plasmid directs protein expression under control of pLac promoter. The unique *Nco*I and *Not*I cloning sites allow insertion of an antibody gene to express single-chain antibodies with a leader peptide of the bacterial periplasmic enzyme, alkaline phosphatase (AP), and with the first two amino acids of the mature AP protein, at the antibody's amino-terminus; and FLAG/His-tail at carboxyl-terminus of antibody. In order to confirm the plasmid's practical qualities, a gene of a single-chain antibody of known specificity, the anti-CEA MA39, was amplified by PCR and cloned into pKM16 vector. Then, freeze-thaw purified periplasmic proteins was analyzed in Western blot developed with an anti-FLAG secondary antibody (Figure 3). Single-chain antibody bands migrated as proteins with expected molecular weight. N-terminal protein sequencing by Edman degradation confirmed correct processing of the leader peptide.

### Phagemids for the display of scFv antibody.

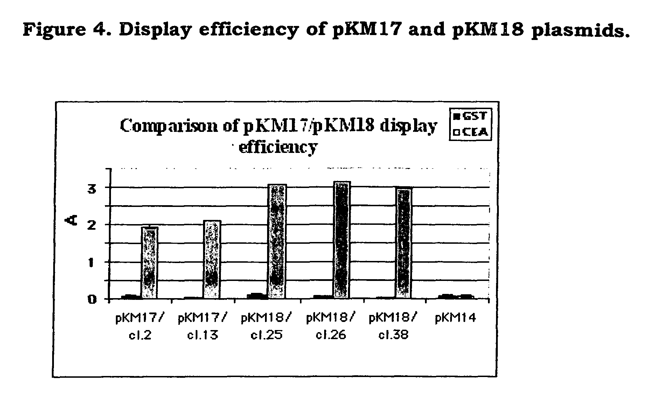

The pKM17 and pKM18 plasmids (Figure 1) allow display of antibody fragment on phage particle by fusion to entire pIII or only to carboxy-terminal domain of the protein, respectively. A functional test was performed by cloning the anti-CEA single-chain gene into these plasmids. ELISA reactivity of the anti-CEA single clones indicates that bacteria harboring any of the plasmids are able to produce phage particles displaying functional antibodies (Figure 4). However, since the signals in ELISA are higher, the pKM 18 clones have an advantage in display of the antibody produced.

### Generation of scFv antibody-displayed library and isolation of binding specificities using new pKM19 plasmid

The pKM 19 plasmid, a derivative of pKM 18, harboring amber codon in leader sequence gene was used for generation of scFv library to study whether low production of fused antibodies allows efficient selection of a specific antibody against a target molecule.

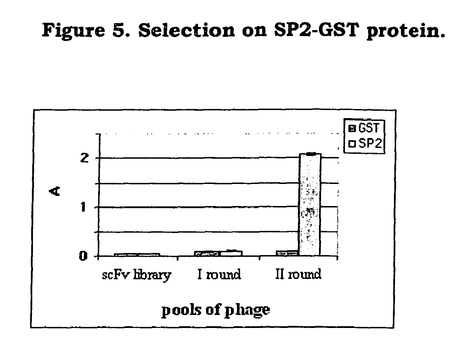

An scFv antibody library was constructed from human peripheral blood lymphocytes as described in Materials and Methods. The library was selected against GST fusion of a 168 aa-long *Streptoccocus pneumoniae* polypeptide (SP2, E. Beghetto, manuscript in preparation), which was reactive with the blood sample utilized for the scFv library construction, as described in Example 2. A selection procedure was designed to create a high concentration of the target protein in small incubation volume, by using biotinylated protein for panning and streptavidin-coated Dynabeads for isolation of bound phage. After completion of two panning rounds, the reactivity of the phage pools was tested in ELISA (Figure 5). The phage pool, already after second round of affinity selection, was highly reactive with the fusion protein and negative with GST (present as protein carrier in the selection and used as negative control in ELISA). Finally, a number of positive clones was isolated and sequenced to confirm correct scFv sequence.

### Maturation of anti-CEA scFv antibody by using pKM19 vector

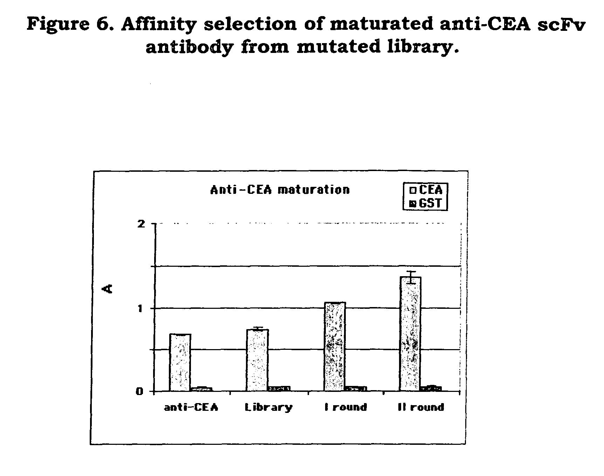

Affinity selection from mutated library was carried out as described in Example 2. After two rounds of selection the pools of phage were tested for their reactivity against CEA protein (Figure 6). Nineteen random clones from pool of phage after second round of affinity selection with enriched anti-CEA reactivity, were sequenced. None of these clones contained stop codons.

### EXAMPLE 2

### Library construction and antibody selection

### Introduction

Identification of tumor-specific recombinant antibodies from display libraries derived from lymph nodes of cancer patients is described in Clin. Exp. Immunol. 1997 109(1):166-74; Int. J. Mol. Med. 2004 14(4):729-35; World J. Gastroenterol. 2004 10(18):2619-23. However, it was quite difficult to obtain metastasized lymph nodes from breast cancer patients as fresh surgical material. According to recent medical practice the surgeon removes only a sentinel lymph node or a small cluster of nodes (sentinel node and those closest to it), instead of removing dozen of lymph nodes and analyzing all of them looking for cancer, thus performing less invasive surgery and reducing side effects. After sentinel lymph node dissection, practically the entire node is studied for presence of micrometastasis or single cancer cells. Therefore, in breast cancer surgery the metastasized node is practically unavailable as discarded surgical material. It was demonstrated in this study that tumor-specific antibodies potentially useful in therapy of cancer, can be efficiently selected against known tumor antigens, as well as against entire tumor cells, from phage display library, derived from pieces of tumor tissue removed in tumor surgery and obtained by using novel pKM 19 vector having improved capacity for selection and maturation of recombinant antibodies.

It was also shown that B-lymphocytes, purified from peripheral blood sample of a breast cancer patient, as small as 10 mL, were sufficient to construct a phage-displayed antibody library giving rise to various tumor-specific antibodies by using a novel phagemid vector, pKM19, having improved capacity for selection and maturation of recombinant antibodies. The possibility of obtaining such antibodies also from tumor-infiltrating lymphocytes was also investigated. Ten random breast carcinoma samples (all of not rare MBC histological type), obtained from patients of 49-79 years old, were examined by PCR amplification of specific antibody gene regions. This analysis has revealed that 7 out of 10 samples had molecular features typical of a rare medullary carcinoma, characterized by strong lymphoplasmacytic infiltrates and favorable prognosis. The prominence of IgG antibody expression in comparison with IgA subclass correlated in these 7 samples with the oligoclonality of the hypervariable region of heavy chain antibodies, suggesting a specific immune response to tumor-derived antigens. Clonality of tumor-derived antibodies was confirmed by sequencing analysis. Higher frequency of somatic mutations within CDRs *versus* FRs within VH of the same specificity (*P*=0.0002) indicated that tumor-infiltrated B-cells locally produce restricted IgG repertoire with evidence of antigen-driven maturation.

A panel of tumor-specific antibodies derived from described libraries, which were reactive with ED-B domain, MUC1, CEA and MCF7 breast carcinoma cells used in respective selections, was identified. It is interesting that in performing cell-based selection without subtractive panning step on normal breast epithelium, in contrast with numerous previously described selection protocols [Int J Mol Med. 2004 14(4):729-35; World J Gastroenterol. 2004 10(18):2619-23; Int J Oncol. 2000 16(1):187-95; Cancer Res. 1999 59(11):2718-23; Biochem Biophys Res Cmmun. 2001 280(2):548-52], only one selected scFv out of 10 was not tumor-specific and recognized normal breast cells as well. Analyzing sequences of □ chain derived from TILs of B92 and B93 patients, it was found that the antibody repertoire was very restricted. Random sequence analysis confirmed oligoclonality of the TIL-derived antibodies. Therefore, it is likely that our moderate-size libraries of reflect naturally occurring antibody responses provided by lymphocytes infitrated in tumor tissue rather then the creation of a vast antibody repertoire by antibody chain shuffling.

In conclusion, our results indicate that naturally occurring immune responses to tumor-related antigens exist in a majority of patients with breast cancer, and not only in hystologically defined MCB. Tumor samples from 70% of patients, obtained as surgical material, can be exploited as appropriate source for construction of recombinant phage display libraries enriched for tumor-specific antibodies. Isolation of a panel of tumor-specific scFvs through selection by using desirable protein targets, as well as living breast carcinoma cells by using novel phagemid vector pKM19 having improved capacity for selection and maturation of recombinant antibodies, shows this approach to be very promising for development of therapeutic antibodies.

### METHODS

### Tissue and blood samples

Specimens of breast carcinoma and fresh peripheral blood from breast cancer patients (B81-B96, EC23) were obtained from M. G. Vannini Hospital, Rome. All the human biological samples were obtained through informed consent.

### Cell lines

The breast carcinoma cell lines MCF-7 (ATCC Number: HTB-22), MDA-MB-468 (ATCC Number: HTB-132) were maintained in DMEM/F12 supplemented with 5% FBS and used for cell-based panning or for analysis of isolated scFv antibodies. Human fibroblasts Hs68 (ATCC number CRL-1635) and immortal breast epithelial cells MCF 10-2A (ATCC number CRL-10781) [Cancer Res. 1990 50(18):6075-86] were propagated according to manufacturer's instructions, and used as negative controls in ELISA tests.

### Purified tumor antigen proteins

Human CEA protein, purified from human fluids, was purchased from Trichem (#10695, Trichem, West Chester, PA).

Biotinylated recombinant ED-B domain of fibronectin was obtained in

Sigma-Tau S.p.A. (Pomezia, Rome).

Recombinant MUC1 protein was obtained in several steps. Two overlapping oligonucleotides KM358 5'-ACT TCA GCT CCG GAC ACC CGT CCG GCT CCG GGT TCC ACC GCT CCG CCG GCT CAC GGT GTC-3' and KM359 5'-CGG AGC CGG ACG GGT GTC CGG AGC TGA AGT GAC ACC GTG AGC CGG CGG AGC GGT GGA ACC-3' encoded for 20-aa MUC1 repeat, were assembled in PCR-like process, in which 25 cycles of PCR amplification were performed with 0,2 pM/□L of KM358 and KM359.

High-weight DNA band was then cut from agarose gel and ligated with a short adapter, obtained by annealing a KM328 5'-CT AGT TCG TCG GGT TCG TCG GGA-3' oligonucleotide and a phospharylated one: KM329 5'-TCC CGA CGA ACC CGA CGA A-3'. The resulting DNA fragment was purified from adapter excess, phosphorylated and cloned into digested and dephospharylated pGEX-SN [Int J Cancer. 2003 106(4):534-44], derived from pGEX-3X plasmid [Gene 1988 67:31-40]. GST-fused MUC1 recombinant protein, containing a 107-aa MUC1 sequence, containing 5.3 repeats, was purified according to standard methods [Gene 1988 67:31-40].

### Purification of lymphocytes from peripheral blood

The lymphocytes were isolated from 10 mL of fresh peripheral blood mixed with anticoagulant by using Ficoll-Paque Plus (Amersham Pharmacia Biotech, Sweden) according to manufacturer's instructions. mRNA was isolated from lymphocytes by using Dynabeads mRNA DIRECT Kit (Dynal, Norway).

### RNA extraction and cDNA synthesis

Tumor specimens of about 200 mg from breast carcinoma patients were obtained as surgical discard samples and immediately frozen in liquid nitrogen. Total RNA was prepared by Total RNA Isolation System (Promega, Madison, WI) and purified to poly A⁺ RNA using PolyATract mRNA Isolation Systems (Promega). Five hundred ng of poly(A)⁺ RNA from breast carcinomas or 1 □g of the poly(A)⁺ RNA from the lymphocytes were used to synthesize full-length cDNAs by using SMART cDNA library construction kit (Clontech,

Palo Alto, CA).

### Analysis of antibody gene expression by PCR

The hypervariable V(D)J antibody region was amplified by PCR from cDNA templates by using site-specific primers 5'-GGACACGGCT(G/C)TGTATTACTG-3' and 5'-GCTGAGGAGACGGTGACC-3' designed in designed in a study by Hansen and colleagues [Proc Natl Acad Sci U S A 2001 98(22):12659-64]. IgG1, IgG2 and IgA subclass determination was done as described in [J Immunol. 2002 169(5):2701-11] by individually combining constant region-specific primers for IgG1, IgG2 and IgA genes (CG1d, CG2a and CA1, respectively) with a set of variable heavy chain primers: VH135, VH3a, VH3f, VH4, VH4b. These primers were designed for construction of human Fab libraries [Barbas CF III, Burton DR (1994) Monoclonal antibodies from combinatorial libraries. Cold Spring Harbor Laboratory Course Manual.].

### ScFv library construction

Antibody gene repertoire was amplified using set of primers designed for amplification of VH and VL antibody domains [ Pope, A.R., Embleton, M.J. & Mernaugh R. (1996) Construction and use of antibody gene repertoires. In: Antibody Engineering - A practical approach (McCafferty, J., Hoogenboom, H. & Chiswell D., eds), pp.325, Oxford University Press] and scFv fragments were assembled *in vitro* as described earlier [Pope AR et al., 1996]. The scFv fragments were then amplified by PCR with appropriate extension primers, incorporating *Nco*I*, Not*I restriction sites, permitting the cloning of the scFv genes into pKM 19 vector. The resulting PCR products were purified on a 1% low- melting agarose gel (NuSieve 3:1 agarose, Rockland, ME). The DNA fragments were digested with *Nco*I/*Not*I and inserted into pKM□□□vector. The ligated DNA was used to transform competent bacterial cells DH5□F' *(supE44* □*lac*U169 (□80 *lacZ*□M15) *hsdR17 recA1 endA1gyrA96 thi-1 relA1* F' *[traD36 proAB⁺ lacI*^{q}*lacZ*□M15]) by electroporation. The transformed cells were plated on 20 agar dishes (o 15 cm), containing LB agar, 100 □g/mL ampicillin and 1% glucose. After overnight incubation at 37°C, bacterial colonies were scraped from the plates and resuspended in LB, containing 10% of glycerol. Aliquots of this cell suspension were stored at -80°C and used for phage amplification.

### Phage amplification

Forty □L of scraped bacterial cells were incubated in 40 mL of LB containing ampicillin and 1% glucose up to O.D.=0.2. The bacteria were collected by centrifuging and resuspended in 40 mL of LB with ampicillin without glucose. About 6x10⁹ pfu of helper M13K07 were added to each mL of cell suspension, incubated for 15 min at 37°C without agitation and a further 2 h in a shaker. Kanamycin was added to final concentration 20 □g/mL and cells were incubated ON at 32°C. Phage was purified according to standard PEG/NaCl precipitation [Sambrook J, Fritsch EF, Maniatis T. Molecular cloning: A laboratory manual. Cold Spring Harbor Laboratory Press, Cold Spring Harbor, 1989].

### Cell-based selection of antibodies from phage-displayed library

MCF-7 semi-confluent cells (about 2x10⁷) were rinsed 3 times with PBS buffer and incubated with 2 mL of 2 mM EDTA in PBS for 15 min at 37°C. Ten mL of PBS containing 10 mM MgCl₂ were added to the cells, they were accurately removed by pipetting. The cells were collected by centrifuging, washed once with 10 mL of PBS/MgCl₂ and finally resuspended in 1 mL of freshly prepared blocking buffer: 4% non-fat dry milk, 0.05% Tween 20, 5x10¹¹ pfu of f1 UV-killed phage. The cells were blocked for 30 min at RT on rotating wheel, then collected and incubated for 1 h at 37°C on the wheel with about 5x10¹¹ TU of freshly amplified scFv antibody library in 1 mL of blocking buffer. The cells were washed 5 times with PBS/Tween. The bound phage was eluted by adding 400 □L of 0.1 M HCl, pH 2.2 (adjusted by glycine). Cell suspension was incubated with elution solution for 10 min at RT, neutralized by 40 □L of 2M Tris-HCl, pH 9.6 and used for infection of bacterial cells. The bacteria were plated on two LB agar dishes (ø 15 cm), containing 100 □g/mL ampicillin and 1% glucose. Scraped bacteria were used for phage amplification.

### Affinity selection on purified protein targets.

CEA and MUC1 were biotinylated as described in [Harlow E. & Lane D. Antibody: A laboratory manual. Cold Spring Harbor Laboratory Press, Cold Spring Harbor, 1988]. About 5x10¹¹ TU of freshly amplified scFv antibody libraries were preincubated with 50 □L of AD202 bacterial extract in blocking buffer for 30 min at 37 °C. Twenty 0 of a biotinylated protein were added to the reaction mixture and incubated for another h at 37 °C under gentle agitation. The bound phage was captured by using streptavidin-coated Dynabeads M-280 (112.05, Dynal, Oslo, Norway) according to manufacturer's instructions, washed 5-10 times with PBS/Tween, then, eluted and amplified as above.

### ELISA experiments

The cells were grown in 96-well plate until almost confluent. After discarding the growth medium, 100 □L of freshly prepared 4% paraformadehyde (#15710, Electron Microscopy Science, Hatfield, PA) in PBS were rapidly added for 10 min. The fixing solution was removed by pipetting and cells were incubated with blocking buffer (5% milk, 0.05% Tween 20 in PBS) for 30 min at RT. PEG purified phage in blocking buffer (1:1) was added to cells and incubated for 1 h at 37 °C under gentle agitation. The cells were washed 3 times and an anti-M13 HRP-conjugated antibody (Pharmacia) was used for developing the reaction. All assays were done in triplicate.

### RESULTS

### Characterization of the lymphoplasmatic cell infiltrate in breast tumor samples

Ten tumor specimens from breast cancer patients (aged 47-79 years) for presence and nature of TIL-B by PCR amplification of V(D)J antibody segments (CDR3) and by comparison of representation of IgG and IgA antibody classes were examined.

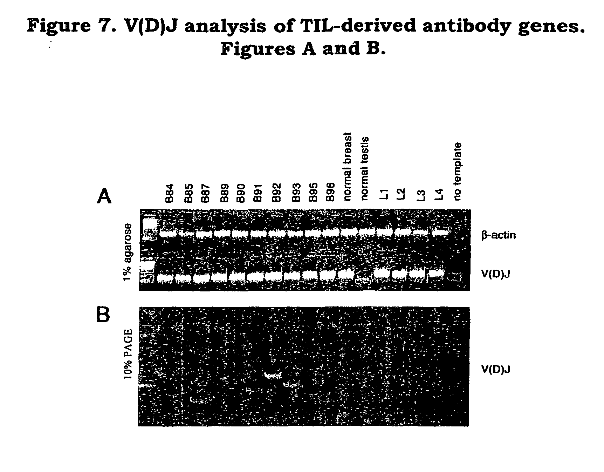

The expression patterns of the antibody fragment genes was analyzed by semi-quantitative PCR from SMART cDNA template. The panel of cDNAs from ten breast carcinomas was normalized, and samples of normal breast, normal testis and peripheral blood lymphocytes from healthy donors by PCR amplification of a housekeeping gene, □-actin (Figure 7A). Hypervariable heavy chain antibody regions (V(D)J) were amplified as described in Materials and Methods. After analysis by agarose gel electrophoresis, the same PCR products were fractionated by high resolving 10% PAGE (Figure 7B). Applying this technique, it was found that 7 out of 10 tumor-deriving samples contain various numbers of discrete bands, characterizing oligoclonality of the immune response in these patients, while the well-amplified normal breast and peripheral lymphocyte DNA fragments do not contain visible bands. Observed oligoclonality of the immunoglobulins does not correlate with the age of the patients.

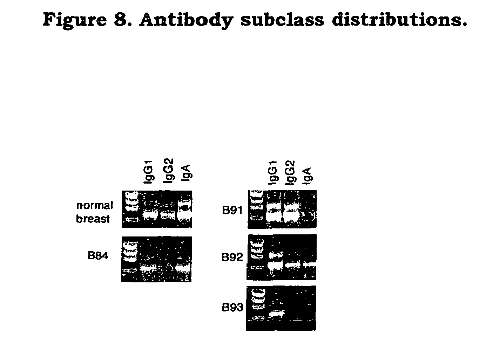

In order to analyze the antibody subclass distributions, the immunoglobulin genes from breast carcinoma cDNAs and normal breast, were amplified by using subclass-specific primers. In agreement with previous assay, the normal breast and 3 cDNA tumor samples, not containing oligoclonal bands in PCR-amplified V(D)J regions, have a prevalence of the IgA band in comparison with IgG1 and IgG2 bands in second test. In contrast, samples showing oligoclonality in first assay, contain IgG1 or both IgG1 and IgG2 as dominant antibody bands. Figure 8 shows four more characteristic examples along with normal breast sample.

### Oligoclonality of TIL-B derived antibodies in breast cancer patients was confirmed by sequencing

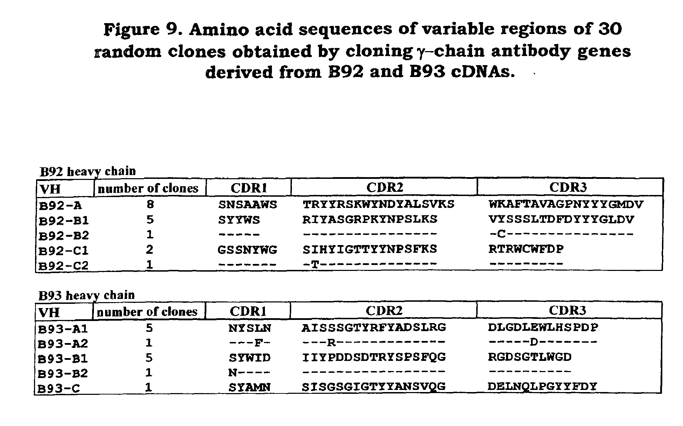

Two cDNA samples (B92, B93) giving strongest single bands in V(D)J test were chosen for sequencing analysis. The nucleotide sequences of 17 and 13 randomly picked clones containing 0 antibody genes deriving from B92 and B93 cDNA, respectively, were determined and their amino acid sequences were deduced. All 30 clones encoded in-frame correct organized heavy chains. More frequently isolated antibodies (B92A and B93A) contained V(D)J regions of a length corresponding to the strong bands observed in Figure 7B (lines with B92 and B93 samples) (Figure 9), thus indicating that both PCR amplification with variable heavy chain primers and the cloning step do not introduce any particular bias interfering with heavy chain frequencies in the constructed library.

Six somatic mutations identified in antibody fragments isolated several times are localized in variable CDRs of □ chain of the same specificity, while only one mutation is found out of variable regions (*P*=0.0002). Therefore, oligoclonality of antibody repertoire derived from tumor tissue is a natural immune response occurring within tumor tissue driven by tumor antigens, and not an artifact introduced by PCR amplification.

### Library construction

Four scFv antibody libraries were constructed using seven cDNAs as template, characterized by oligoclonality of the immune response (see list of libraries in Table 1). Only library scFvEC23 was constructed from peripheral blood lymphocytes, obtained from a single patient with advanced stage of breast cancer.

**Table 1 ScFv-antibody library list.**

| Library | Source of Ig genes | Patient (age) | Library complexity |
|---|---|---|---|
| ScFvB87 | TIL | B87 (55) | 4.7x10⁵ |
| ScFvB95 | TIL | B95 (73) | 1.1x10⁷ |
| ScFvB96 | TIL | B96 (72) | 2.6x10⁷ |
| Sc Fvmix | TIL | B85 (47), B91 (70), B92 (79), B93 (66) | 2.4x10⁷ |
| scFvEC23 | PBL | EC23 (65) | 1.8 x 10⁷ |

### Selection of specific anti-tumor antibodies from phage display libraries generated from TIL-B and PBL.

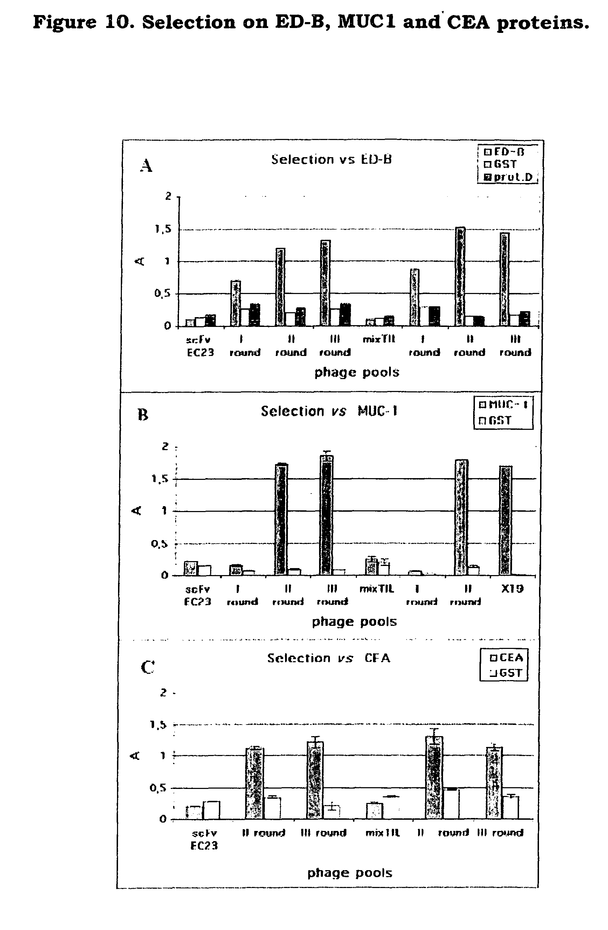

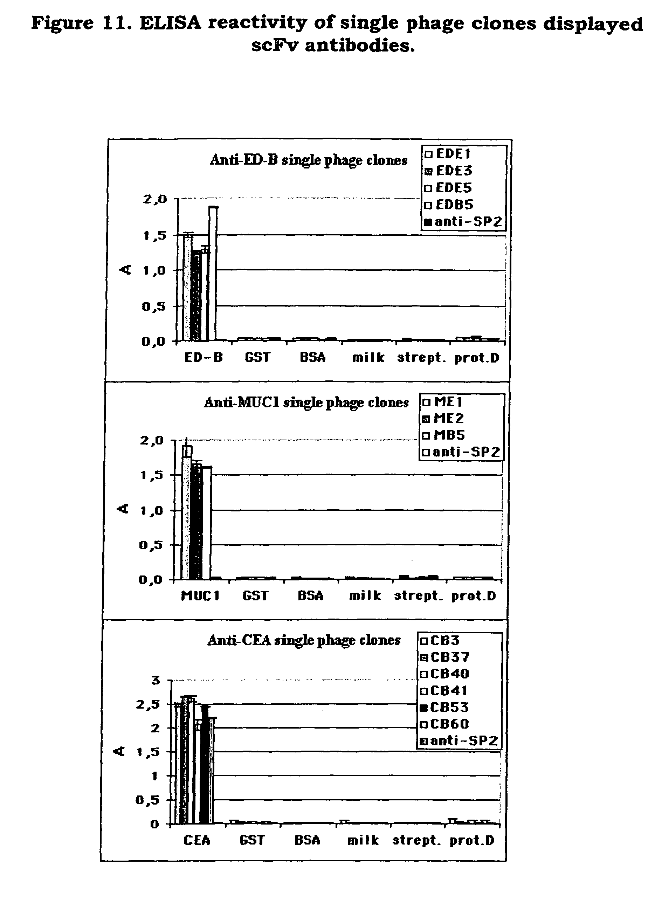

The possibility of selecting specific antibody fragments from phage libraries against common cancer antigens available in our lab, including ED-B domain of fibronectin [EMBO J. 1987 6(8):2337-42], MUC1 [Cancer Res. 1992 52(22):6365-70; Hum Pathol. 1995 26(4):432-9], and CEA [J. Clin. Lab. Anal. 5: 344-366; Semin Cancer Biol. 1999 9:67-81; Cancer Res. 2002 62:5049-5057], was examined directly. Under conditions described in Materials and Methods a mixture of four TIL-derived scFv antibody-displayed libraries (mixTIL) and the scFvEC23 library were panned separately against three protein targets in several rounds. In every case, the pools of phage were already positive against the selecting antigen after second and third rounds of panning (Figure 10). Randomly picked clones were tested for binding reactivity against the antigens. Results of the test of random phage clones from third round phage pools are summarized in Table 2. Positive clones were analyzed by fingerprinting using *Hae*III and *Alu*I double digestion and unique antibody clones were sequenced. Figure 11 represents ELISA of single scFv-phages selected on purified antigens.

**Table 2. Result of selections through the use of three purified tumor antigens.**

| Target antigen | Library | Positive clones/ tested clones | Isolated anti-body genes |
|---|---|---|---|
| ED-B | mixTIL | 10/10 | 1 |
| | scFvEC23 | 10/10 | 3 |
| MUC1 | mixTIL | 2/16 | 1 |
| | scFvEC23 | 6/8 | 2 |
| CEA | mixTIL | 17/20 | 4 |
| | scFvEC23 | 15/20 | 3 |

### Cell-based selection of tumor-specific antibodies

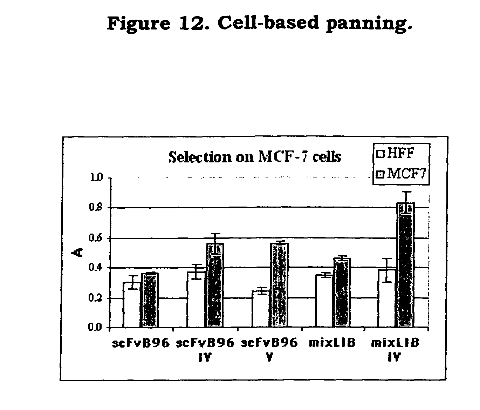

Functionality of a single TIL-derived library (scFvB96) was tested by selecting breast cancer-specific antibodies through cell-based panning on MCF-7 breast carcinoma cell line. Another four libraries, including scFvB87, scFvB95, scFvmix, scFV23, were pooled together (mixLIB) and panned on the same type of cells. Four or five selection rounds on MCF-7 cells were necessary for mixLIB or scFvB96 libraries, respectively, in order to enrich the phage pools for specific cell binders (Figure 12). Then, randomly picked clones were analyzed for entire scFv antibody presence. The full-length scFv-phage clones were tested by cell-based ELISA, and analyzed by fingerprinting, and various positive clones were sequenced. Amino acid sequences were deduced from DNA sequences, confirming correct, in-frame antibody structures. Clone analysis data are summarized in Table 3. Nucleotide and amino acid sequences of all selected antibodies are presented, Seq. Id. N° 2 - Seq. Id. N° 41.

**Table 3 Result of selection on intact/living human breast carcinoma MCF7 cells.**

| | **MCF-7 selection** | |
|---|---|---|
| Library | scFvB96 | mixLIB |
| Selection round | 5 | 4 |
| Full-length scFv/ tested clones | 12/40 | 30/40 |
| Positive clones/ full-length tested clones | 5/12 | 22/30 |
| Isolated anti-body genes | 2 | 8 |

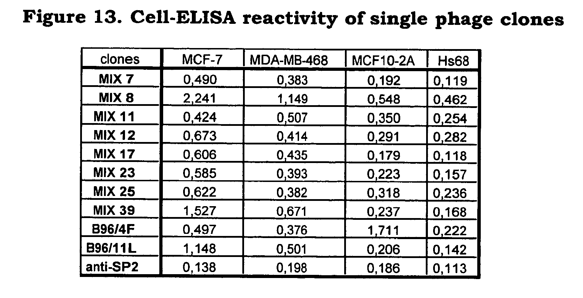

The reactivity and specificity of cell-selected antibodies were verified by ELISA on both breast carcinoma cell lines: MCF-7, MDA-MB-468, and normal cells, as negative controls: MCF 10-2A (human breast epithelium), Hs68 (human fibroblasts) (Figure 13). Among 10 different antibodies belonging to 7 specificity groups (mix7, mix12, mix25 have the same heavy chain sequence and different light chains; mix8 and mix39 have similar sequences with minor differences), 9 are specific for breast carcinoma cells, while only B96/4F scFv bound normal epithelial cells as well.

### Fluorescent staining of tumor cells

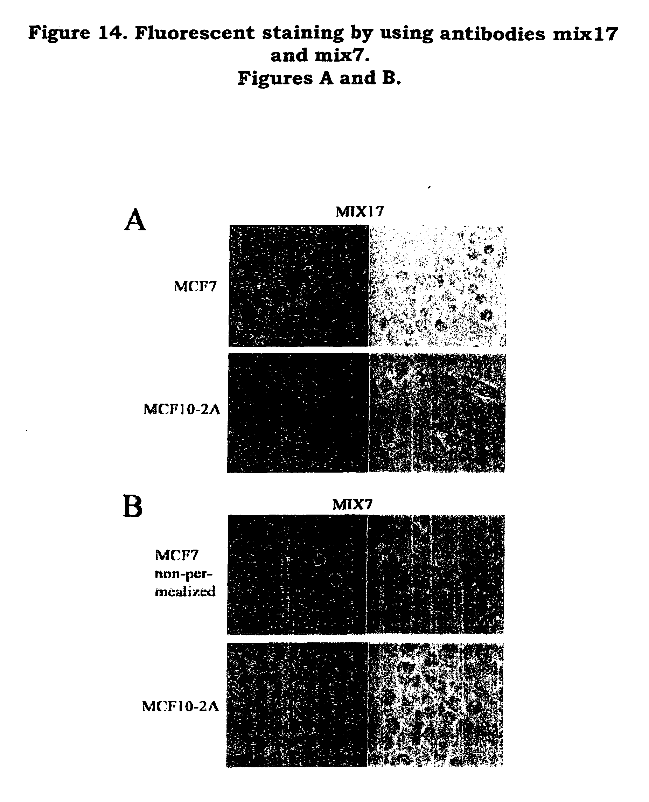

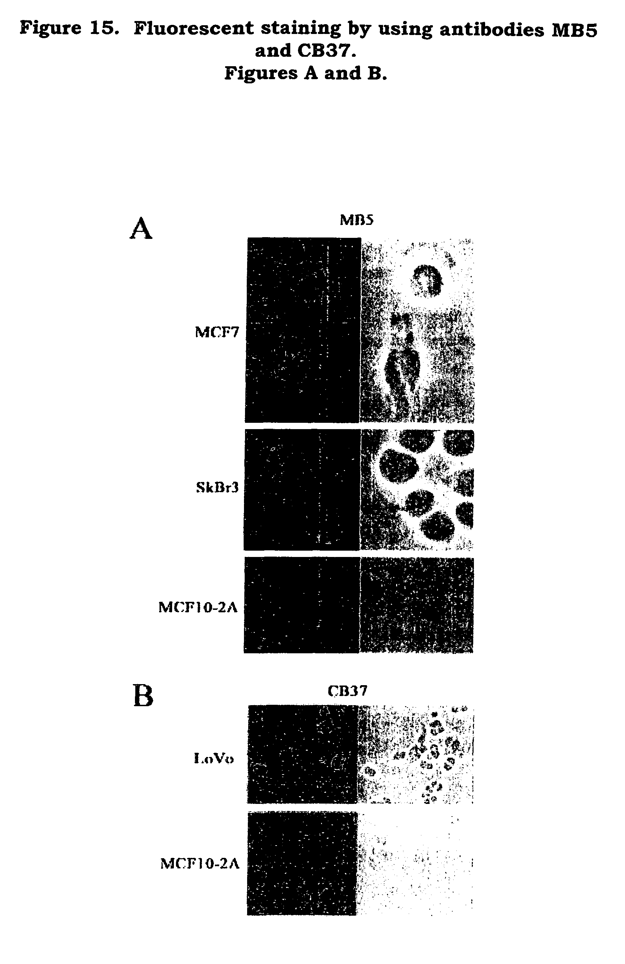

Binding specificities of several clones comprising anti-MCF7 antibodies MIX17 and MIX7 (Figure 14), anti-Muc1 antibody MB5 and anti-CEA CB37 (Figure 15) were assayed by immunofluorescent staining of tumor cells directly with scFvs antibodies displayed on the phage. MIX17 scFv recognizes major part of non-permealized MCF7 breast carcinoma cells in this experiment, while MIX7 stains low percentage of cells, probably in certain phase of the cell growth. MB5 antibody intensively stains MCF7 cells, known for high MUC1 expression and reacts well also with another breast carcinoma cell line, SkBr3. No background staining for normal breast epithelium was observed.

## Claims

1. Method for improving the capacity of the phage-display system for the selection and maturation of recombinant antibodies, in which said improvement is obtained according to the following steps:
(i) introduction in a phagemid vector of elements that reduce the expression levels of the antibodies produced, in which said reduction of the expression of the antibodies produced is obtained by introducing a suppressed stop codon inside the leader peptide gene; or by using inhibitors for promoters in the phagemid vectors; or by introducing a suppressed stop codon inside the antibody gene; or by introducing low-efficient or repressible promoters in the phagemid vector; and/or
(ii) fusion of single-chain antibody genes only to carboxy-terminal part of pIII protein; and/or
(iii) use of the leader peptide gene of the alkaline phosphatase of *E. coli.*

2. Phagemid vector having improved capacity of selection and/or maturation of recombinant antibodies, **characterized in that** it contains elements that reduce the expression levels of the antibodies produced and/or has an improved efficiency of display of the antibody produced.

3. The phagemid vector of claim 2, in which the reduction of the expression levels of the antibodies produced and/or the improved efficiency of display of the antibody produced is obtained: (i) by introducing a suppressed of the stop codon inside the leader peptide gene; or by introducing a suppressed stop codon inside the antibody gene; or by introducing low-efficient or repressible promoters in the phagemid vector; or by using inhibitors for promoters in the phagemid vectors; and/or (ii) by the fusion of single-chain antibody genes only to carboxy-terminal part of pIII protein; and/or (iii) by the use of the leader peptide gene of the alkaline phosphatase of *E. coli.*

4. The phagemid vector of claims 2-3, having the nucleotide sequence of SEQ ID NO: 1.

5. A phage display library constructed using the phagemid vector of claims 2-4.

6. A phage display library of claim 5, selected from the group comprising:
ScFvB87; ScFvB95; ScFvB96; scFvmix; or scFvEC23.

7. A recombinant antibody, in a single or double-chain format, obtained using the phage display library of claim 5 or 6.

8. Antibody of claim 7, which recognize an antigen selected from the group comprising: ED-B protein domain of fibronectin, MUC 1 tumor antigen, CEA antigen, MCF7 breast carcinoma.

9. Antibody of claim 7 or 8, selected from the group comprising:
EDE1 scFv antibody, recognizing the ED-B protein domain of fibronectin, having the nucleotide sequence of SEQ ID NO: 2, and the amino acid sequence of SEQ ID NO: 3;
EDE3 scFv antibody, recognizing the ED-B protein domain, having the nucleotide sequence of SEQ ID NO: 4, and the amino acid sequence of SEQ ID NO: 5;
EDE5 scFv antibody, recognizing the ED-B protein domain, having the nucleotide sequence of SEQ ID NO: 6, and the amino acid sequence of SEQ ID NO:7;
EDB5 scFv antibody, recognizing the ED-B protein domain, having the nucleotide sequence of SEQ ID NO: 8, and the amino acid sequence of SEQ ID NO:9;
ME1 scFv antibody, recognizing the MUC1 tumor antigen, having the nucleotide sequence of SEQ ID NO: 10, and the amino acid sequence of SEQ ID NO: 11;
ME2 scFv antibody, recognizing the MUC1 tumor antigen, having the nucleotide sequence of SEQ ID NO: 12, and the amino acid sequence of SEQ ID NO: 13;
MB5 scFv antibody, recognizing the MUC1 tumor antigen, having the nucleotide sequence of SEQ ID NO: 14, and the amino acid sequence of SEQ ID NO: 15;
CB3 scFv antibody, recognizing the CEA (carcino-embrionic antigen), having the nucleotide sequence of SEQ ID NO: 16, and the amino acid sequence of SEQ ID NO: 17;
CB37 scFv antibody, recognizing the CEA antigen, having the nucleotide sequence of SEQ ID NO: 18, and the amino acid sequence of SEQ ID NO: 19;
CB41 scFv antibody, recognizing the CEA antigen, having the nucleotide sequence of SEQ ID NO: 20, and the amino acid sequence of SEQ ID NO: 21;
CB53 scFv antibody, recognizing the CEA antigen, having the nucleotide sequence of SEQ ID NO: 22, and the amino acid sequence of SEQ ID NO:23;
B96/11L scFv antibody, recognizing the MCF7 breast carcinoma cells, having the nucleotide sequence of SEQ ID NO: 24, and the amino acid sequence of SEQ ID NO:25;
mix7 scFv antibody, recognizing the MCF7 breast carcinoma cells, having the nucleotide sequence of SEQ ID NO: 26, and the amino acid sequence of SEQ ID NO: 27;
mix8 scFv antibody, recognizing the MCF7 breast carcinoma cells, having the nucleotide sequence of SEQ ID NO: 28, and the amino acid sequence of SEQ ID NO: 29;
mix11 scFv antibody, recognizing the MCF7 breast carcinoma cells, having the nucleotide sequence of SEQ ID NO: 30, and the amino acid sequence of SEQ ID NO: 31;
mix12 scFv antibody, recognizing the MCF7 breast carcinoma cells, having the nucleotide sequence of SEQ ID NO: 32, and the amino acid sequence of SEQ ID NO: 33;
mix17 scFv antibody, recognizing the MCF7 breast carcinoma cells, having the nucleotide sequence of SEQ ID NO: 34, and the amino acid sequence of SEQ ID NO: 35;
mix23 scFv antibody, recognizing the MCF7 breast carcinoma cells, having the nucleotide sequence of SEQ ID NO: 36, and the amino acid sequence of SEQ ID NO: 37;
mix25 scFv antibody, recognizing the MCF7 breast carcinoma cells, having the nucleotide sequence of SEQ ID NO: 38, and the amino acid sequence of SEQ ID NO:39;
mix39 scFv antibody, recognizing the MCF7 breast carcinoma cells, having the nucleotide sequence of SEQ ID NO: 40, and the amino acid sequence of SEQ ID NO: 41.

10. Method of producing an antigen or antigen fragment **characterized by** the following steps:
a. Purification of the lymphocytes, if any;
b. RNA extraction and cDNA synthesis;
c. Amplification of the antibody genes using known oligonucleotide primers;
d. "in vitro" assembly of the antibody genes;
e. Generation of antibody-displayed libraries using the phagemid vector of claims 2-4;
f. Phage amplification;
g. Selection of new antibody specificities against purified protein antigen, comprising purified proteins from natural sources or recombinant proteins obtained by correspondent gene amplification, cloning and protein production;
h. Production of the selected antibodies in soluble form using a plasmid vector known in the art;
i. Identification of specific peptide antigens by selection of peptide from random peptide library or from peptide library derived from DNA encoding target protein gene through selection by using soluble antibody fragments;
j. Alternatively, after step "f", selection of new antibody specificities by using complex multi-component biological structures, like living cells;
k. Repeat step "h";
l. Identification of new disease-specific targets, for example, new tumor surface antigens, by using novel anti-tumor antibodies recognizing tumor cells through: (i) immunoprecipitation of unknown target proteins from tumor cell extracts; or (ii) developing the immunoreactons with tumor cell extract, separated by two-dimensional PAGE and transferred onto nitrocellulose membrane;
m. Protein sequencing and identification of new antigens.

11. The antigen of the method of claim 10, useful for producing vaccines, diagnostic reagents or in the research field.

12. DNA which encodes for the antibodies of claims 7-9.

13. Vector for the expression of the antibody which contains the DNA which encodes for the antibodies of claims 7-9.

14. Cell tranfected with the vector for the expression of the antibody which contains the DNA which encodes for the antibodies of claims 7-9.

15. Process for the production of the recombinant antibody of claims 7-9, which comprises the cultivation of the cell tranfected with the vector for the expression of the antibody which contains the DNA which encodes for said antibodies, and isolation of the antibodies produced.

16. Pharmaceutical composition comprising as active ingredient one or more recombinant antibody of claims 7-9, and optionally one or more excipients or diluents pharmaceutically acceptable.

17. Pharmaceutical composition comprising as active ingredient one or more disease-specific antigen of the method of claim 10, and optionally one or more excipients or diluents pharmaceutically acceptable.

18. Diagnostic reagent, comprising one or more recombinant antibody of claims 7-9, useful for the diagnosis of infective, inflammatory, tumoral, metabolic, autoimmune or allergic diseases.

19. Diagnostic reagent, comprising one or more specific antigen of the method of claim 10, useful for the diagnosis of infective, inflammatory, tumoral, metabolic, autoimmune or allergic diseases.

20. Use of the phagemid vector of claims 2-4, for the preparation of recombinant antibody libraries, in which:
said library is selected from the group comprising synthetic or semi-synthetic antibody libraries or mutated antibody gene libraries for affinity maturation of antibodies;
said libraries are useful for producing antibodies specific for a selected antigen;
said antibodies can be used for the selection of specific antigen;
said antibodies are in single or double-chain format.

21. Use of the recombinant antibodies of claims 7-9, for preparing a medicament for the therapy of infective, inflammatory, tumoral, metabolic and autoimmune or allergic diseases.

22. Use of the recombinant antibodies of claims 7-9, for preparing diagnostic reagents for infective, inflammatory, tumoral, metabolic, autoimmune or allergic diseases.

23. Use of the recombinant antibodies of claims 7-9, for preparing reagents for research use.

24. Use of the recombinant antibodies of claims 7-9, for the identification of novel disease-specific antigens.

25. Use of the novel disease-specific antigen of the method of claim 10, for preparing vaccines for the prevention or therapy of infective, inflammatory, tumoral, metabolic, autoimmune or allergic diseases.

26. Use of the novel disease-specific antigen of the method of claim 10, for preparing diagnostic reagents for infective, inflammatory, tumoral, metabolic, autoimmune or allergic diseases.

27. Use of the novel disease-specific antigen of the method of claim 10, for preparing reagents useful in the research field.


SEQUENCE LISTING

<110>  SIGMA-TAU INDUSTRIE FARMACEUTICHE RIUNITE S.p.A.

<120>  Method of improving the antibody selection capacity in
       phage-display library.

<130>  515-EP

<160>  41

<170>  PatentIn version 3.3

<210>  1
<211>  3770
<212>  DNA
<213>  E. coli

<400>  1
gcccaatacg caaaccgcct ctccccgcgc gttggccgat tcattaatgc agctggcacg      60

acaggtttcc cgactggaaa gcgggcagtg agcgcaacgc aattaatgtg agttagctca     120

ctcattaggc accccaggct ttacacttta tgcttccggc tcgtatgttg tgtggaattg     180

tgagcggata acaatttcac acaagatcta gctattctag agattacgcc aagccccgta     240

ttttacccgt ttaatggaag cttataaagg aggaaatcct catgaaatag agcaccatcg     300

cactggcact gttaccgtta ctgttcaccc cggttaccaa agcacgtacc atggtttccc     360

ttgcggccgc aggagactac aaagacgacg acgacaaaga attcctgcct caacctcctg     420

tcaatgctgg cggcggctct ggtggtggtt ctggtggcgg ctctgagggt ggcggctctg     480

agggtggcgg ttctgagggt ggcggctctg agggtggcgg ttccggtggc ggctccggtt     540

ccggtgattt tgattatgaa aaaatggcaa acgctaataa gggggctatg accgaaaatg     600

ccgatgaaaa cgcgctacag tctgacgcta aaggcaaact tgattctgtc gctactgatt     660

acggtgctgc tatcgatggt ttcattggtg acgtttccgg ccttgctaat ggtaatggtg     720

ctactggtga ttttgctggc tctaattccc aaatggctca agtcggtgac ggtgataatt     780

cacctttaat gaataatttc cgtcaatatt taccttcttt gcctcagtcg gttgaatgtc     840

gcccttatgt ctttggcgct ggtaaaccat atgaattttc tattgattgt gacaaaataa     900

acttattccg tggtgtcttt gcgtttcttt tatatgttgc cacctttatg tatgtatttt     960

cgacgtttgc taacatactg cgtaataagg agtcttaagg atcctaatat tgttctggat    1020

attaccagca aggccgatag tttgagttct tctactcagg caagtgatgt tattactaat    1080

caaagaagta ttgcgacaac ggttaatttg cgtgatggac agactctttt actcggtggc    1140

ctcactgatt ataaaaacac ttctcaggat tctggcgtac cgttcctgtc taaaatccct    1200

ttaatcggcc tcctgtttag ctcccgctct gattctaacg aggaaagcac gttatacgtg    1260

ctcgtcaaag caaccatagt acgcgccctg tagcggcgca ttaagcgcgg cgggtgtggt 1320

ggttacgcgc agcgtgaccg ctacacttgc cagcgcccta gcgcccgctc ctttcgcttt 1380

cttcccttcc tttctcgcca cgttcgccgg ctttccccgt caagctctaa atcgggggct 1440

ccctttaggg ttccgattta gtgctttacg gcacctcgac cccaaaaaac ttgattaggg 1500

tgatggttca cgtagtgggc catcgccctg atagacggtt tttcgccctt tgacgttgga 1560

gtccacgttc tttaatagtg gactcttgtt ccaaactgga acaacactca accctatctc 1620

ggtctattct tttgatttat aagggatttt gccgatttcg gcctattggt taaaaaatga 1680

gctgatttaa caaaaattta acgcgaattt taacaaaata ttaacgttta caatttaaat 1740

atttgcttat acaatcttcc tgtttttggg gcttttctga ttatcaaccg gggtacatat 1800

gattgacatg ctagttttac gattaccgtt catcgcaggt ggcacttttc ggggaaatgt 1860

gcgcggaacc cctatttgtt tatttttcta aatacattca aatatgtatc cgctcatgag 1920

acaataaccc tgataaatgc ttcaataata ttgaaaaagg aagagtatga gtattcaaca 1980

tttccgtgtc gcccttattc ccttttttgc ggcattttgc cttcctgttt ttgctcaccc 2040

agaaacgctg gtgaaagtaa aagatgctga agatcagttg ggtgcacgag tgggttacat 2100

cgaactggat ctcaacagcg gtaagatcct tgagagtttt cgccccgaag aacgttttcc 2160

aatgatgagc acttttaaag ttctgctatg tggcgcggta ttatcccgta ttgacgccgg 2220

gcaagagcaa ctcggtcgcc gcatacacta ttctcagaat gacttggttg agtactcacc 2280

agtcacagaa aagcatctta cggatggcat gacagtaaga gaattatgca gtgctgccat 2340

aaccatgagt gataacactg cggccaactt acttctgaca acgatcggag gaccgaagga 2400

gctaaccgct tttttgcaca acatggggga tcatgtaact cgccttgatc gttgggaacc 2460

ggagctgaat gaagccatac caaacgacga gcgtgacacc acgatgcctg tagcaatggc 2520

aacaacgttg cgcaaactat taactggcga actacttact ctagcttccc ggcaacaatt 2580

aatagactgg atggaggcgg ataaagttgc aggaccactt ctgcgctcgg cccttccggc 2640

tggctggttt attgctgata aatctggagc cggtgagcgt gggtctcgcg gtatcattgc 2700

agcactgggg ccagatggta agccctcccg tatcgtagtt atctacacga cggggagtca 2760

ggcaactatg gatgaacgaa atagacagat cgctgagata ggtgcctcac tgattaagca 2820

ttggtaactg tcagaccaag tttactcata tatactttag attgatttaa aacttcattt 2880

ttaatttaaa aggatctagg tgaagatcct ttttgataat ctcatgacca aaatccctta 2940

acgtgagttt tcgttccact gagcgtcaga ccccgtagaa aagatcaaag gatcttcttg 3000

agatcctttt tttctgcgcg taatctgctg cttgcaaaca aaaaaaccac cgctaccagc 3060

ggtggtttgt ttgccggatc aagagctacc aactcttttt ccgaaggtaa ctggcttcag 3120

cagagcgcag ataccaaata ctgtccttct agtgtagccg tagttaggcc accacttcaa 3180

gaactctgta gcaccgccta catacctcgc tctgctaatc ctgttaccag tggctgctgc 3240

cagtggcgat aagtcgtgtc ttaccgggtt ggactcaaga cgatagttac cggataaggc 3300

gcagcggtcg ggctgaacgg ggggttcgtg cacacagccc agcttggagc gaacgaccta 3360

caccgaactg agatacctac agcgtgagct atgagaaagc gccacgcttc ccgaagggag 3420

aaaggcggac aggtatccgg taagcggcag ggtcggaaca ggagagcgca cgagggagct 3480

tccaggggga aacgcctggt atctttatag tcctgtcggg tttcgccacc tctgacttga 3540

gcgtcgattt ttgtgatgct cgtcaggggg gcggagccta tggaaaaacg ccagcaacgc 3600

ggccttttta cggttcctgg ccttttgctg gccttttgct cacatgttct ttcctgcgtt 3660

atcccctgat tctgtggata accgtattac cgcctttgag tgagctgata ccgctcgccg 3720

cagccgaacg accgagcgca gcgagtcagt gagcgaggaa gcggaagagc 3770

<210> 2
<211> 791
<212> DNA
<213> E. coli

<220>
<221> CDS
<222> (1)..(789)

<400> 2
cag gtg cag ctg cag gag tct ggg gct gag gtg aag aag cct ggg gcc 48
Gln Val Gln Leu Gln Glu Ser Gly Ala Glu Val Lys Lys Pro Gly Ala
1               5                   10                  15

tca gtg aag gtc tcc tgc aag gct tct gga tac acc ttc acc ggc tac 96
Ser Val Lys Val Ser Cys Lys Ala Ser Gly Tyr Thr Phe Thr Gly Tyr
            20                  25                  30

tat atg cac tgg gtg cga cag gcc cct gga caa ggg ctt gag tgg atg 144
Tyr Met His Trp Val Arg Gln Ala Pro Gly Gln Gly Leu Glu Trp Met
        35                  40                  45

gga tgg atc aac cct aac agt ggt ggc aca aac tat gca cag aag ttt 192
Gly Trp Ile Asn Pro Asn Ser Gly Gly Thr Asn Tyr Ala Gln Lys Phe
    50                  55                  60

cag ggc agg gtc acc atg acc agg gac acg tcc atc agc aca gcc tac 240
Gln Gly Arg Val Thr Met Thr Arg Asp Thr Ser Ile Ser Thr Ala Tyr
65                  70                  75                  80

atg gag ctg agc agg ctg aga tct gac gac acg gcc gtg tat tac tgt 288
Met Glu Leu Ser Arg Leu Arg Ser Asp Asp Thr Ala Val Tyr Tyr Cys
                85                  90                  95

gcg aga gat tcg cca caa aat tgt act aat ggt gta tgc cac cgg ggg 336
Ala Arg Asp Ser Pro Gln Asn Cys Thr Asn Gly Val Cys His Arg Gly

                100                 105                 110

agt cat gtc cac tac tac ggt atg gac gtc tgg ggc caa ggc acc ctg        384
Ser His Val His Tyr Tyr Gly Met Asp Val Trp Gly Gln Gly Thr Leu
        115                 120                 125

gtc acc gtc tct tca ggt ggg ggc ggt tca ggc gga ggt ggc tct ggc        432
Val Thr Val Ser Ser Gly Gly Gly Gly Ser Gly Gly Gly Gly Ser Gly
    130                 135                 140

ggt ggc gga tcg cag tct gcc ctg act cag cct gcc tcc gcg gcc ggg        480
Gly Gly Gly Ser Gln Ser Ala Leu Thr Gln Pro Ala Ser Ala Ala Gly
145                 150                 155                 160

tct cct gga cag tca gtc acc atc tcc tgc act gga acc agc agt gat        528
Ser Pro Gly Gln Ser Val Thr Ile Ser Cys Thr Gly Thr Ser Ser Asp
                165                 170                 175

gtt ggt ggt tat aac tat gtc tcc tgg tac caa cag cac cca ggc aaa        576
Val Gly Gly Tyr Asn Tyr Val Ser Trp Tyr Gln Gln His Pro Gly Lys
            180                 185                 190

gcc ccc aaa ctc atg att tat gac gtc aat aag cgg ccc tca ggg gtc        624
Ala Pro Lys Leu Met Ile Tyr Asp Val Asn Lys Arg Pro Ser Gly Val
        195                 200                 205

cct gat cgc ttc tct gcc tcc aag tct ggc aac acg gcc tcc ctg acc        672
Pro Asp Arg Phe Ser Ala Ser Lys Ser Gly Asn Thr Ala Ser Leu Thr
    210                 215                 220

gtc tct ggg ctc cag gct gac gat gag gct gat tac tac tgc gct tca        720
Val Ser Gly Leu Gln Ala Asp Asp Glu Ala Asp Tyr Tyr Cys Ala Ser
225                 230                 235                 240

tat gca ggc acc tac agt tat gtc ttc gga act ggg acc cag ctc acc        768
Tyr Ala Gly Thr Tyr Ser Tyr Val Phe Gly Thr Gly Thr Gln Leu Thr
                245                 250                 255

gtt tta ggt gcg gcc gca gga ga                                         791
Val Leu Gly Ala Ala Ala Gly
            260


<210>  3
<211>  263
<212>  PRT
<213>  E. coli

<400>  3

Gln Val Gln Leu Gln Glu Ser Gly Ala Glu Val Lys Lys Pro Gly Ala
1               5                   10                  15


Ser Val Lys Val Ser Cys Lys Ala Ser Gly Tyr Thr Phe Thr Gly Tyr
            20                  25                  30


Tyr Met His Trp Val Arg Gln Ala Pro Gly Gln Gly Leu Glu Trp Met
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Asp Ser Pro Gln Asn Cys Thr Asn Gly Val Cys His Arg Gly
            100                 105                 110

Ser His Val His Tyr Tyr Gly Met Asp Val Trp Gly Gln Gly Thr Leu
        115                 120                 125

Val Thr Val Ser Ser Gly Gly Gly Gly Ser Gly Gly Gly Gly Ser Gly
    130                 135                 140

Gly Gly Gly Ser Gln Ser Ala Leu Thr Gln Pro Ala Ser Ala Ala Gly
145                 150                 155                 160

Ser Pro Gly Gln Ser Val Thr Ile Ser Cys Thr Gly Thr Ser Ser Asp
                165                 170                 175

Val Gly Gly Tyr Asn Tyr Val Ser Trp Tyr Gln Gln His Pro Gly Lys
            180                 185                 190

Ala Pro Lys Leu Met Ile Tyr Asp Val Asn Lys Arg Pro Ser Gly Val
        195                 200                 205

Pro Asp Arg Phe Ser Ala Ser Lys Ser Gly Asn Thr Ala Ser Leu Thr
    210                 215                 220

Val Ser Gly Leu Gln Ala Asp Asp Glu Ala Asp Tyr Tyr Cys Ala Ser
225                 230                 235                 240

Tyr Ala Gly Thr Tyr Ser Tyr Val Phe Gly Thr Gly Thr Gln Leu Thr
                245                 250                 255

Val Leu Gly Ala Ala Ala Gly
            260

<210>  4
<211>  786
<212>  DNA
<213>  E. coli

<220>
<221> CDS
<222> (1)..(786)

<400> 4
gag gtg cag ctg ttg cag tct ggg gcc gag gtg aag aag cct ggg gcc 48
Glu Val Gln Leu Leu Gln Ser Gly Ala Glu Val Lys Lys Pro Gly Ala
1               5                   10                  15

tca gtg aag gtc tcc tgc aag gct tct gga tac acc ttc acc ggc tac 96
Ser Val Lys Val Ser Cys Lys Ala Ser Gly Tyr Thr Phe Thr Gly Tyr
            20                  25                  30

tat atg cac tgg gtg cga cag gcc cct gga caa ggg ctt gag tgg atg 144
Tyr Met His Trp Val Arg Gln Ala Pro Gly Gln Gly Leu Glu Trp Met
        35                  40                  45

gga tgg atc aac cct aac agt ggt ggc aca aac tat gca cag aag ttt 192
Gly Trp Ile Asn Pro Asn Ser Gly Gly Thr Asn Tyr Ala Gln Lys Phe
    50                  55                  60

cag ggc agg gtc acc atg acc agg gac acg tcc atc agc aca gcc tac 240
Gln Gly Arg Val Thr Met Thr Arg Asp Thr Ser Ile Ser Thr Ala Tyr
65                  70                  75                  80

atg gag ctg agc agg ctg aga tct gac gac acg gcc gtg tat tac tgt 288
Met Glu Leu Ser Arg Leu Arg Ser Asp Asp Thr Ala Val Tyr Tyr Cys
                85                  90                  95

gcg aga gat tcg cca caa aat tgt act aat ggt gta tgc cac cgg ggg 336
Ala Arg Asp Ser Pro Gln Asn Cys Thr Asn Gly Val Cys His Arg Gly
            100                 105                 110

agt cat gtc cac tac tac ggt atg gac gtc tgg ggc cag gga acc ctg 384
Ser His Val His Tyr Tyr Gly Met Asp Val Trp Gly Gln Gly Thr Leu
        115                 120                 125

gtc acc gtc tcc tca ggt ggg ggc ggt tca ggc gga ggt ggc tct ggc 432
Val Thr Val Ser Ser Gly Gly Gly Gly Ser Gly Gly Gly Gly Ser Gly
    130                 135                 140

ggt ggc gga tcg cag tct gcc ctg act cag cct gcc tcc gcg gcc ggg 480
Gly Gly Gly Ser Gln Ser Ala Leu Thr Gln Pro Ala Ser Ala Ala Gly
145                 150                 155                 160

tgt ctt gga cag tca gtc acc atc tcc tgc act gga acc agc agt gat 528
Cys Leu Gly Gln Ser Val Thr Ile Ser Cys Thr Gly Thr Ser Ser Asp
                165                 170                 175

gtt ggt ggt tat aaa tat gtc tcc tgg tac caa cag cac cca ggc aaa 576
Val Gly Gly Tyr Lys Tyr Val Ser Trp Tyr Gln Gln His Pro Gly Lys
            180                 185                 190

gcc ccc aaa ctc atg att tat gac gtc aat aag cgg ccc tca ggg gtc 624
Ala Pro Lys Leu Met Ile Tyr Asp Val Asn Lys Arg Pro Ser Gly Val
        195                 200                 205

cct gat cgc ttc ttt gcc tcc aag tct ggc aac acg gcc tcc ctg acc 672
Pro Asp Arg Phe Phe Ala Ser Lys Ser Gly Asn Thr Ala Ser Leu Thr

210                 215                 220

gtc tct ggg ctc cag gct gac gat gag gct gat tac tac tgc gct tca         720
Val Ser Gly Leu Gln Ala Asp Asp Glu Ala Asp Tyr Tyr Cys Ala Ser
225                 230                 235                 240

tat gca ggc acc tac agt tat gtc ttc gga act ggg acc cag ctc acc         768
Tyr Ala Gly Thr Tyr Ser Tyr Val Phe Gly Thr Gly Thr Gln Leu Thr
                245                 250                 255

gtt tta ggt gcg gcc gca                                                 786
Val Leu Gly Ala Ala Ala
            260

<210>  5
<211>  262
<212>  PRT
<213>  E. coli

<400>  5

Glu Val Gln Leu Leu Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Asp Ser Pro Gln Asn Cys Thr Asn Gly Val Cys His Arg Gly
            100                 105                 110

Ser His Val His Tyr Tyr Gly Met Asp Val Trp Gly Gln Gly Thr Leu
        115                 120                 125

Val Thr Val Ser Ser Gly Gly Gly Gly Ser Gly Gly Gly Gly Ser Gly
    130                 135                 140

Gly Gly Gly Ser Gln Ser Ala Leu Thr Gln Pro Ala Ser Ala Ala Gly
145                 150                 155                 160

Cys Leu Gly Gln Ser Val Thr Ile Ser Cys Thr Gly Thr Ser Ser Asp
                165                 170                 175

Val Gly Gly Tyr Lys Tyr Val Ser Trp Tyr Gln Gln His Pro Gly Lys
            180                 185                 190

Ala Pro Lys Leu Met Ile Tyr Asp Val Asn Lys Arg Pro Ser Gly Val
        195                 200                 205

Pro Asp Arg Phe Phe Ala Ser Lys Ser Gly Asn Thr Ala Ser Leu Thr
    210                 215                 220

Val Ser Gly Leu Gln Ala Asp Asp Glu Ala Asp Tyr Tyr Cys Ala Ser
225                 230                 235                 240

Tyr Ala Gly Thr Tyr Ser Tyr Val Phe Gly Thr Gly Thr Gln Leu Thr
                245                 250                 255

Val Leu Gly Ala Ala Ala
            260

<210>  6
<211>  789
<212>  DNA
<213>  E. coli

<220>
<221>  CDS
<222>  (1)..(789)

<400>  6
gag gtg cag ctg gtg gag tct ggg gct gag gtg aag aag cct ggg gcc      48
Glu Val Gln Leu Val Glu Ser Gly Ala Glu Val Lys Lys Pro Gly Ala
1               5                   10                  15

tca gtg aag gtc tcc tgc aag gct tct gga tac acc ttc acc ggc tac      96
Ser Val Lys Val Ser Cys Lys Ala Ser Gly Tyr Thr Phe Thr Gly Tyr
            20                  25                  30

tat atg cac tgg gtg cga cag gcc cct gga caa ggg ctt gag tgg atg     144
Tyr Met His Trp Val Arg Gln Ala Pro Gly Gln Gly Leu Glu Trp Met
        35                  40                  45

gga tgg atc aac cct aac agt ggt ggc aca aac tat gca cag aag ttt     192
Gly Trp Ile Asn Pro Asn Ser Gly Gly Thr Asn Tyr Ala Gln Lys Phe
    50                  55                  60

cag ggc agg gtc acc atg acc agg gac acg tcc atc agc aca gcc tac     240
Gln Gly Arg Val Thr Met Thr Arg Asp Thr Ser Ile Ser Thr Ala Tyr
65                  70                  75                  80

atg gag ctg agc agg ctg aga tct gac gac acg gcc gtg tat tac tgt     288

Met Glu Leu Ser Arg Leu Arg Ser Asp Asp Thr Ala Val Tyr Tyr Cys
                85                  90                  95

gtg aga ggt tcg cca caa aat tgt act aat ggt gta tgc cac cgg ggg      336
Val Arg Gly Ser Pro Gln Asn Cys Thr Asn Gly Val Cys His Arg Gly
            100                 105                 110

agt cat gtc cac tac tac ggt atg gac gtc tgg ggc caa ggg acc acg      384
Ser His Val His Tyr Tyr Gly Met Asp Val Trp Gly Gln Gly Thr Thr
        115                 120                 125

gtc acc gtc tcc tca ggt ggg ggc ggt tca ggc gga ggt ggc tct ggc      432
Val Thr Val Ser Ser Gly Gly Gly Gly Ser Gly Gly Gly Gly Ser Gly
    130                 135                 140

ggt ggc gga tcg cag tct gcc ctg act cag cct gcc tcc gtg tct ggg      480
Gly Gly Gly Ser Gln Ser Ala Leu Thr Gln Pro Ala Ser Val Ser Gly
145                 150                 155                 160

tct cct gga cag tcg atc acc atc tcc tgc act gga acc agc agt gat      528
Ser Pro Gly Gln Ser Ile Thr Ile Ser Cys Thr Gly Thr Ser Ser Asp
                165                 170                 175

gtt ggg agt tat aac ctt gtc tcc tgg tac caa cag cac cca ggc aaa      576
Val Gly Ser Tyr Asn Leu Val Ser Trp Tyr Gln Gln His Pro Gly Lys
            180                 185                 190

gcc ccc aaa ctc atg att tat gag gtc agt aat cgg ccc tca ggg gtt      624
Ala Pro Lys Leu Met Ile Tyr Glu Val Ser Asn Arg Pro Ser Gly Val
        195                 200                 205

tgt aat cgc ttc tct ggc tcc aag tct ggc aac acg gcc tcc ctg acc      672
Cys Asn Arg Phe Ser Gly Ser Lys Ser Gly Asn Thr Ala Ser Leu Thr
    210                 215                 220

atc tct ggg ctc cag gct gag gac gag gct gat tat tac tgc agc tca      720
Ile Ser Gly Leu Gln Ala Glu Asp Glu Ala Asp Tyr Tyr Cys Ser Ser
225                 230                 235                 240

tat aca agc agc agc act ctc gag gtg ttc ggc gga ggg acc cag ctc      768
Tyr Thr Ser Ser Ser Thr Leu Glu Val Phe Gly Gly Gly Thr Gln Leu
                245                 250                 255

acc gtt tta ggt gcg gcc gca                                          789
Thr Val Leu Gly Ala Ala Ala
            260

<210>  7
<211>  263
<212>  PRT
<213>  E. coli

<400>  7

Glu Val Gln Leu Val Glu Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Val Arg Gly Ser Pro Gln Asn Cys Thr Asn Gly Val Cys His Arg Gly
            100                 105                 110

Ser His Val His Tyr Tyr Gly Met Asp Val Trp Gly Gln Gly Thr Thr
        115                 120                 125

Val Thr Val Ser Ser Gly Gly Gly Gly Ser Gly Gly Gly Gly Ser Gly
    130                 135                 140

Gly Gly Gly Ser Gln Ser Ala Leu Thr Gln Pro Ala Ser Val Ser Gly
145                 150                 155                 160

Ser Pro Gly Gln Ser Ile Thr Ile Ser Cys Thr Gly Thr Ser Ser Asp
                165                 170                 175

Val Gly Ser Tyr Asn Leu Val Ser Trp Tyr Gln Gln His Pro Gly Lys
            180                 185                 190

Ala Pro Lys Leu Met Ile Tyr Glu Val Ser Asn Arg Pro Ser Gly Val
        195                 200                 205

Cys Asn Arg Phe Ser Gly Ser Lys Ser Gly Asn Thr Ala Ser Leu Thr
    210                 215                 220

Ile Ser Gly Leu Gln Ala Glu Asp Glu Ala Asp Tyr Tyr Cys Ser Ser
225                 230                 235                 240

Tyr Thr Ser Ser Ser Thr Leu Glu Val Phe Gly Gly Gly Thr Gln Leu
                245                 250                 255

Thr Val Leu Gly Ala Ala Ala
            260

<210> 8
<211> 747
<212> DNA
<213> E. coli

<220>
<221> CDS
<222> (1)..(747)

<400> 8
cag gag gtg cag ctg gtg gag tct ggg ggt ggc ttg gtc cag cct ggg 48
Gln Glu Val Gln Leu Val Glu Ser Gly Gly Gly Leu Val Gln Pro Gly
1               5                   10                  15

ggg tcc ctg aga ctc tcc tgt gca gcc tct gga ttc acc ctc agt agc 96
Gly Ser Leu Arg Leu Ser Cys Ala Ala Ser Gly Phe Thr Leu Ser Ser
            20                  25                  30

tat gct atg cac tgg gtc cgc cag gct cca ggg aag ggg ctg gag tgg 144
Tyr Ala Met His Trp Val Arg Gln Ala Pro Gly Lys Gly Leu Glu Trp
        35                  40                  45

gtc tca act att agt ggt ggt ggt ggt agc aca tac tac gca gac tcc 192
Val Ser Thr Ile Ser Gly Gly Gly Gly Ser Thr Tyr Tyr Ala Asp Ser
    50                  55                  60

gtg aag ggc cgg ttc acc atc tcc aga gac aat tcc aag aac acg ctg 240
Val Lys Gly Arg Phe Thr Ile Ser Arg Asp Asn Ser Lys Asn Thr Leu
65                  70                  75                  80

tat ctg caa atg aac agc ctg aga gcc gag gac acg gcc gta tat tac 288
Tyr Leu Gln Met Asn Ser Leu Arg Ala Glu Asp Thr Ala Val Tyr Tyr
                85                  90                  95

tgt gcg aga cgg ggg cgg gct ttt gat atc tgg ggc caa ggg acc acg 336
Cys Ala Arg Arg Gly Arg Ala Phe Asp Ile Trp Gly Gln Gly Thr Thr
            100                 105                 110

gtc acc gtc tcc tta ggt gga ggc ggt tca ggc gga ggt ggc tct ggc 384
Val Thr Val Ser Leu Gly Gly Gly Gly Ser Gly Gly Gly Gly Ser Gly
        115                 120                 125

ggt ggc gga tcg cag tct gtg ttg acg cag ccg ccc tca gtg tct ggg 432
Gly Gly Gly Ser Gln Ser Val Leu Thr Gln Pro Pro Ser Val Ser Gly
    130                 135                 140

gcc cca ggg cag agg gtc acc atc tcc tgc act ggg agc agc tcc aac 480
Ala Pro Gly Gln Arg Val Thr Ile Ser Cys Thr Gly Ser Ser Ser Asn
145                 150                 155                 160

atc ggg gcg ggg tat gat gta cac tgg tac cag cag ctt cca gga aca 528
Ile Gly Ala Gly Tyr Asp Val His Trp Tyr Gln Gln Leu Pro Gly Thr
                165                 170                 175

gcc ccc aaa ctc ctc att tat ggt aac agc aat cgg ccc tca ggg gtc 576
Ala Pro Lys Leu Leu Ile Tyr Gly Asn Ser Asn Arg Pro Ser Gly Val
            180                 185                 190

cct gac cga ttc tct ggc tcc aag tct ggc acc tca gcc tcc ctg gcc 624

Pro Asp Arg Phe Ser Gly Ser Lys Ser Gly Thr Ser Ala Ser Leu Ala
        195                 200                 205

atc act ggg ctc cag gct gag gat gag gct gat tat tat tgc tcc agt 672
Ile Thr Gly Leu Gln Ala Glu Asp Glu Ala Asp Tyr Tyr Cys Ser Ser
    210                 215                 220

cct atg atc agc agc ctg agt ggt cat gtg gta ttc ggc gga ggg acc 720
Pro Met Ile Ser Ser Leu Ser Gly His Val Val Phe Gly Gly Gly Thr
225                 230                 235                 240

aag gtg acc gtc cta ggt gcg gcc gca 747
Lys Val Thr Val Leu Gly Ala Ala Ala
                245

<210> 9
<211> 249
<212> PRT
<213> E. coli

<400> 9

Gln Glu Val Gln Leu Val Glu Ser Gly Gly Gly Leu Val Gln Pro Gly
1               5                   10                  15

Gly Ser Leu Arg Leu Ser Cys Ala Ala Ser Gly Phe Thr Leu Ser Ser
            20                  25                  30

Tyr Ala Met His Trp Val Arg Gln Ala Pro Gly Lys Gly Leu Glu Trp
        35                  40                  45

Val Ser Thr Ile Ser Gly Gly Gly Gly Ser Thr Tyr Tyr Ala Asp Ser
    50                  55                  60

Val Lys Gly Arg Phe Thr Ile Ser Arg Asp Asn Ser Lys Asn Thr Leu
65                  70                  75                  80

Tyr Leu Gln Met Asn Ser Leu Arg Ala Glu Asp Thr Ala Val Tyr Tyr
                85                  90                  95

Cys Ala Arg Arg Gly Arg Ala Phe Asp Ile Trp Gly Gln Gly Thr Thr
            100                 105                 110

Val Thr Val Ser Leu Gly Gly Gly Gly Ser Gly Gly Gly Gly Ser Gly
        115                 120                 125

Gly Gly Gly Ser Gln Ser Val Leu Thr Gln Pro Pro Ser Val Ser Gly
    130                 135                 140

Ala Pro Gly Gln Arg Val Thr Ile Ser Cys Thr Gly Ser Ser Ser Asn
145                 150                 155                 160

Ile Gly Ala Gly Tyr Asp Val His Trp Tyr Gln Gln Leu Pro Gly Thr
                165                 170                 175

Ala Pro Lys Leu Leu Ile Tyr Gly Asn Ser Asn Arg Pro Ser Gly Val
            180                 185                 190

Pro Asp Arg Phe Ser Gly Ser Lys Ser Gly Thr Ser Ala Ser Leu Ala
        195                 200                 205

Ile Thr Gly Leu Gln Ala Glu Asp Glu Ala Asp Tyr Tyr Cys Ser Ser
    210                 215                 220

Pro Met Ile Ser Ser Leu Ser Gly His Val Val Phe Gly Gly Gly Thr
225                 230                 235                 240

Lys Val Thr Val Leu Gly Ala Ala Ala
                245

<210>  10
<211>  747
<212>  DNA
<213>  E. coli

<220>
<221>  CDS
<222>  (1)..(747)

<400>  10
cag gtg cag ctg gtg cag tct ggg gct gag gtg aag aag cct ggg gcc       48
Gln Val Gln Leu Val Gln Ser Gly Ala Glu Val Lys Lys Pro Gly Ala
1               5                   10                  15

tca gtg aag gtc tcc tgc aag gct tct gga tac acc ttc acc ggc tac       96
Ser Val Lys Val Ser Cys Lys Ala Ser Gly Tyr Thr Phe Thr Gly Tyr
            20                  25                  30

tat atg cac tgg gtg cga cag gcc cct gga caa ggg ctt gag tgg atg      144
Tyr Met His Trp Val Arg Gln Ala Pro Gly Gln Gly Leu Glu Trp Met
        35                  40                  45

gga tgg atc aac cct aac agt ggt ggc aca aac tat gca cag aag ttc      192
Gly Trp Ile Asn Pro Asn Ser Gly Gly Thr Asn Tyr Ala Gln Lys Phe
    50                  55                  60

cag ggc agg gtc acc atg acc agg gac acg tcc att ggc aca gtc tac      240
Gln Gly Arg Val Thr Met Thr Arg Asp Thr Ser Ile Gly Thr Val Tyr
65                  70                  75                  80

atg gag ttg agc agc ctg aca tct gac gac acg gcc atg tat tat tgt      288
Met Glu Leu Ser Ser Leu Thr Ser Asp Asp Thr Ala Met Tyr Tyr Cys
                85                  90                  95

gcg aga aac aat gtt gct atg ggt tat act atg gac gtc tgg ggc caa 336
Ala Arg Asn Asn Val Ala Met Gly Tyr Thr Met Asp Val Trp Gly Gln
            100                 105                 110

ggg aca atg gtc acc gtc tct tca ggt gga ggc ggt tca ggc gga ggt 384
Gly Thr Met Val Thr Val Ser Ser Gly Gly Gly Gly Ser Gly Gly Gly
        115                 120                 125

ggc tct ggc ggt ggc gga tcg cag tct gcc ctg act cag cct gcc tcc 432
Gly Ser Gly Gly Gly Gly Ser Gln Ser Ala Leu Thr Gln Pro Ala Ser
    130                 135                 140

gcg tcc ggg tct cct gga cag tca gtc acc atc tcc tgc act gga acc 480
Ala Ser Gly Ser Pro Gly Gln Ser Val Thr Ile Ser Cys Thr Gly Thr
145                 150                 155                 160

agc agt gac gtt ggt ggt tat aac tat gtc tcc tgg tac caa cag cac 528
Ser Ser Asp Val Gly Gly Tyr Asn Tyr Val Ser Trp Tyr Gln Gln His
                165                 170                 175

cca ggc aaa acc ccc aaa ctc ttg att tat gag gtc agt agt cgg ccc 576
Pro Gly Lys Thr Pro Lys Leu Leu Ile Tyr Glu Val Ser Ser Arg Pro
            180                 185                 190

tca ggg gtt tct aat cgc ttc tct ggc tcc aag cct ggc aac acg gcc 624
Ser Gly Val Ser Asn Arg Phe Ser Gly Ser Lys Pro Gly Asn Thr Ala
        195                 200                 205

tcc ctg acc atc tct ggt ctc cag gct gag gac gag gct gat tat tac 672
Ser Leu Thr Ile Ser Gly Leu Gln Ala Glu Asp Glu Ala Asp Tyr Tyr
    210                 215                 220

tgc atc tca tat aca agc agc aac act tgg gtg ttc ggc gga ggg acc 720
Cys Ile Ser Tyr Thr Ser Ser Asn Thr Trp Val Phe Gly Gly Gly Thr
225                 230                 235                 240

cag ctc acc gtt tta ggt gcg gcc gca 747
Gln Leu Thr Val Leu Gly Ala Ala Ala
                245

<210> 11
<211> 249
<212> PRT
<213> E. coli

<400> 11

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Gly Thr Asn Tyr Ala Gln Lys Phe

50                  55                  60

Gln Gly Arg Val Thr Met Thr Arg Asp Thr Ser Ile Gly Thr Val Tyr
65                  70                  75                  80

Met Glu Leu Ser Ser Leu Thr Ser Asp Asp Thr Ala Met Tyr Tyr Cys
                85                  90                  95

Ala Arg Asn Asn Val Ala Met Gly Tyr Thr Met Asp Val Trp Gly Gln
            100                 105                 110

Gly Thr Met Val Thr Val Ser Ser Gly Gly Gly Gly Ser Gly Gly Gly
        115                 120                 125

Gly Ser Gly Gly Gly Gly Ser Gln Ser Ala Leu Thr Gln Pro Ala Ser
    130                 135                 140

Ala Ser Gly Ser Pro Gly Gln Ser Val Thr Ile Ser Cys Thr Gly Thr
145                 150                 155                 160

Ser Ser Asp Val Gly Gly Tyr Asn Tyr Val Ser Trp Tyr Gln Gln His
                165                 170                 175

Pro Gly Lys Thr Pro Lys Leu Leu Ile Tyr Glu Val Ser Ser Arg Pro
            180                 185                 190

Ser Gly Val Ser Asn Arg Phe Ser Gly Ser Lys Pro Gly Asn Thr Ala
        195                 200                 205

Ser Leu Thr Ile Ser Gly Leu Gln Ala Glu Asp Glu Ala Asp Tyr Tyr
    210                 215                 220

Cys Ile Ser Tyr Thr Ser Ser Asn Thr Trp Val Phe Gly Gly Gly Thr
225                 230                 235                 240

Gln Leu Thr Val Leu Gly Ala Ala Ala
                245

<210>  12
<211>  733
<212>  DNA
<213>  E. coli

<220>
<221>  CDS
<222>  (1)..(732)

<400> 12
gag gtg cag ctg ttg cag tct ggg gcg gag gtg aag aag cct ggg gcc 48
Glu Val Gln Leu Leu Gln Ser Gly Ala Glu Val Lys Lys Pro Gly Ala
1               5                   10                  15

tca gtg aag gtc tcc tgc aag gct tct gga tac acc ttc acc ggc tac 96
Ser Val Lys Val Ser Cys Lys Ala Ser Gly Tyr Thr Phe Thr Gly Tyr
            20                  25                  30

tat atg cac tgg gtg cga cag gcc cct gga caa ggg ctt gag tgg atg 144
Tyr Met His Trp Val Arg Gln Ala Pro Gly Gln Gly Leu Glu Trp Met
        35                  40                  45

gga tgg atc aac cct aac agt ggt ggc aca aac tat gca cag aag ttt 192
Gly Trp Ile Asn Pro Asn Ser Gly Gly Thr Asn Tyr Ala Gln Lys Phe
    50                  55                  60

cag ggc aga gtc acc atg acc agg aac acc tcc ata agc aca gcc tac 240
Gln Gly Arg Val Thr Met Thr Arg Asn Thr Ser Ile Ser Thr Ala Tyr
65                  70                  75                  80

atg gag ctg agc agc ctg aga tct gag gac acg gcc gtg tat tac tgt 288
Met Glu Leu Ser Ser Leu Arg Ser Glu Asp Thr Ala Val Tyr Tyr Cys
                85                  90                  95

gcg ggt cag gag gca cat ggg gac ggt atg gac gtc tgg ggc caa ggg 336
Ala Gly Gln Glu Ala His Gly Asp Gly Met Asp Val Trp Gly Gln Gly
            100                 105                 110

acc acg gtc acc gtc tcc tcg gtg gag cga ggt ggc tct ggc ggt ggc 384
Thr Thr Val Thr Val Ser Ser Val Glu Arg Gly Gly Ser Gly Gly Gly
        115                 120                 125

gga tcg cag tct gcc ctg act cag cct gcc tcc gcg tcc ggg tct cct 432
Gly Ser Gln Ser Ala Leu Thr Gln Pro Ala Ser Ala Ser Gly Ser Pro
    130                 135                 140

gga cag tcg atc acc atc tcc tgc act gga acc agc ggt gac gtt ggt 480
Gly Gln Ser Ile Thr Ile Ser Cys Thr Gly Thr Ser Gly Asp Val Gly
145                 150                 155                 160

ggt tat aac tat gtc tcc tgg tac caa cag cac cca ggc aaa gcc ccc 528
Gly Tyr Asn Tyr Val Ser Trp Tyr Gln Gln His Pro Gly Lys Ala Pro
                165                 170                 175

aaa ctc atg att tat gaa gtc agt aat cgg ccc tca ggg gtt tct aat 576
Lys Leu Met Ile Tyr Glu Val Ser Asn Arg Pro Ser Gly Val Ser Asn
            180                 185                 190

cgc ttc tct ggc tcc aag tct ggc agc acg gcc tcc ctg acc atc tct 624
Arg Phe Ser Gly Ser Lys Ser Gly Ser Thr Ala Ser Leu Thr Ile Ser
        195                 200                 205

ggg ctc cag gct gag gac gag gct gat tat tac tgc gtc tca tat aca 672
Gly Leu Gln Ala Glu Asp Glu Ala Asp Tyr Tyr Cys Val Ser Tyr Thr
    210                 215                 220

agc aga aac act tat gtc ttc gga tcc ggg acc cag ctc acc gtt tta 720
Ser Arg Asn Thr Tyr Val Phe Gly Ser Gly Thr Gln Leu Thr Val Leu
225                 230                 235                 240

ggt gcg gcc gcg a                                                    733
Gly Ala Ala Ala

<210>  13
<211>  244
<212>  PRT
<213>  E. coli

<400>  13

Glu Val Gln Leu Leu Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asn Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Gly Gln Glu Ala His Gly Asp Gly Met Asp Val Trp Gly Gln Gly
            100                 105                 110

Thr Thr Val Thr Val Ser Ser Val Glu Arg Gly Gly Ser Gly Gly Gly
        115                 120                 125

Gly Ser Gln Ser Ala Leu Thr Gln Pro Ala Ser Ala Ser Gly Ser Pro
    130                 135                 140

Gly Gln Ser Ile Thr Ile Ser Cys Thr Gly Thr Ser Gly Asp Val Gly
145                 150                 155                 160

Gly Tyr Asn Tyr Val Ser Trp Tyr Gln Gln His Pro Gly Lys Ala Pro
                165                 170                 175

Lys Leu Met Ile Tyr Glu Val Ser Asn Arg Pro Ser Gly Val Ser Asn
            180                 185                 190

Arg Phe Ser Gly Ser Lys Ser Gly Ser Thr Ala Ser Leu Thr Ile Ser

195                 200                 205

Gly Leu Gln Ala Glu Asp Glu Ala Asp Tyr Tyr Cys Val Ser Tyr Thr
    210                 215                 220

Ser Arg Asn Thr Tyr Val Phe Gly Ser Gly Thr Gln Leu Thr Val Leu
225                 230                 235                 240

Gly Ala Ala Ala

<210>  14
<211>  738
<212>  DNA
<213>  E. coli

<220>
<221>  CDS
<222>  (1)..(738)

<220>
<221>  misc_feature
<222>  (274)..(274)
<223>  n is a, c, g, or t

<220>
<221>  misc_feature
<222>  (387)..(387)
<223>  n is a, c, g, or t

<400>  14
gag gtg cag ctg gtg gag tct gga gct gag gtg aag aag ccc ggg gcc      48
Glu Val Gln Leu Val Glu Ser Gly Ala Glu Val Lys Lys Pro Gly Ala
1               5                   10                  15

tca gtg aag gtc tcc tgc aag gct tct gga tac acc ttc acc gcc tcc      96
Ser Val Lys Val Ser Cys Lys Ala Ser Gly Tyr Thr Phe Thr Ala Ser
            20                  25                  30

tat atg cac tgg gtg cga cag gcc cct gga caa ggg ctt gag tgg atg      144
Tyr Met His Trp Val Arg Gln Ala Pro Gly Gln Gly Leu Glu Trp Met
        35                  40                  45

gga tgg ttc aac cct aat agt ggt ggc aca aac tat gca cag aag ttt      192
Gly Trp Phe Asn Pro Asn Ser Gly Gly Thr Asn Tyr Ala Gln Lys Phe
    50                  55                  60

cag ggc agg gtc acc atg acc ggg gac acg tcc acc agc aca ggc tat      240
Gln Gly Arg Val Thr Met Thr Gly Asp Thr Ser Thr Ser Thr Gly Tyr
65                  70                  75                  80

atg gag ctg agc agg ctg aca tct gac gac gcg ncc gtg tat tat tgt      288
Met Glu Leu Ser Arg Leu Thr Ser Asp Asp Ala Xaa Val Tyr Tyr Cys
                85                  90                  95

gcg aga gat cgg gcc tct gct atg ggc gtc tgg ggc caa ggc acc ctg      336

Ala Arg Asp Arg Ala Ser Ala Met Gly Val Trp Gly Gln Gly Thr Leu
            100                 105                 110

gtc acc gtc tcc tca ggt gga ggc ggt tca ggc gga ggt ggc tct ggc      384
Val Thr Val Ser Ser Gly Gly Gly Gly Ser Gly Gly Gly Gly Ser Gly
        115                 120                 125

ggn ggc gga tcg cag tct gcc ctg act cag cct gcc tcc gcg tcc ggg      432
Gly Gly Gly Ser Gln Ser Ala Leu Thr Gln Pro Ala Ser Ala Ser Gly
    130                 135                 140

tct cct gga cag tca gtc acc atc tcc tgc act gga acc agc agt gac      480
Ser Pro Gly Gln Ser Val Thr Ile Ser Cys Thr Gly Thr Ser Ser Asp
145                 150                 155                 160

gtt ggt ggt tat aac tat gtc tcc tgg tac caa cag cac cca ggc aaa      528
Val Gly Gly Tyr Asn Tyr Val Ser Trp Tyr Gln Gln His Pro Gly Lys
                165                 170                 175

gcc ccc aaa ctc atg att tat gac gtc aat aag cgg ccc tca ggg gtc      576
Ala Pro Lys Leu Met Ile Tyr Asp Val Asn Lys Arg Pro Ser Gly Val
            180                 185                 190

cct gat cgc ttc tct ggc tcc aag tct ggc aac acg gcc tcc ctg acc      624
Pro Asp Arg Phe Ser Gly Ser Lys Ser Gly Asn Thr Ala Ser Leu Thr
        195                 200                 205

gtc tct ggg ctc cag gct gag gat gag gct gat tat tac tgc agc tca      672
Val Ser Gly Leu Gln Ala Glu Asp Glu Ala Asp Tyr Tyr Cys Ser Ser
    210                 215                 220

tat gca ggt agt aac act ttc cta ttc ggc gga ggg acc cag ctc acc      720
Tyr Ala Gly Ser Asn Thr Phe Leu Phe Gly Gly Gly Thr Gln Leu Thr
225                 230                 235                 240

gtt tta ggt gcg gcc gca                                              738
Val Leu Gly Ala Ala Ala
                245


<210>  15
<211>  246
<212>  PRT
<213>  E. coli

<220>
<221>  misc_feature
<222>  (92)..(92)
<223>  The 'Xaa' at location 92 stands for Thr, Ala, Pro, or Ser.

<400>  15

Glu Val Gln Leu Val Glu Ser Gly Ala Glu Val Lys Lys Pro Gly Ala
1               5                   10                  15


Ser Val Lys Val Ser Cys Lys Ala Ser Gly Tyr Thr Phe Thr Ala Ser
            20                  25                  30


Tyr Met His Trp Val Arg Gln Ala Pro Gly Gln Gly Leu Glu Trp Met

35                  40                  45

Gly Trp Phe Asn Pro Asn Ser Gly Gly Thr Asn Tyr Ala Gln Lys Phe
    50                  55                  60

Gln Gly Arg Val Thr Met Thr Gly Asp Thr Ser Thr Ser Thr Gly Tyr
65                  70                  75                  80

Met Glu Leu Ser Arg Leu Thr Ser Asp Asp Ala Xaa Val Tyr Tyr Cys
                85                  90                  95

Ala Arg Asp Arg Ala Ser Ala Met Gly Val Trp Gly Gln Gly Thr Leu
            100                 105                 110

Val Thr Val Ser Ser Gly Gly Gly Gly Ser Gly Gly Gly Gly Ser Gly
        115                 120                 125

Gly Gly Gly Ser Gln Ser Ala Leu Thr Gln Pro Ala Ser Ala Ser Gly
    130                 135                 140

Ser Pro Gly Gln Ser Val Thr Ile Ser Cys Thr Gly Thr Ser Ser Asp
145                 150                 155                 160

Val Gly Gly Tyr Asn Tyr Val Ser Trp Tyr Gln Gln His Pro Gly Lys
                165                 170                 175

Ala Pro Lys Leu Met Ile Tyr Asp Val Asn Lys Arg Pro Ser Gly Val
            180                 185                 190

Pro Asp Arg Phe Ser Gly Ser Lys Ser Gly Asn Thr Ala Ser Leu Thr
        195                 200                 205

Val Ser Gly Leu Gln Ala Glu Asp Glu Ala Asp Tyr Tyr Cys Ser Ser
    210                 215                 220

Tyr Ala Gly Ser Asn Thr Phe Leu Phe Gly Gly Gly Thr Gln Leu Thr
225                 230                 235                 240

Val Leu Gly Ala Ala Ala
                245

<210>  16
<211>  372
<212>  DNA
<213>  E. coli

<220>
<221> CDS
<222> (1)..(372)

<400> 16
gag gtg cag ctg ttg cag tct ggg gct gag gtg aag aag cct ggg gcc 48
Glu Val Gln Leu Leu Gln Ser Gly Ala Glu Val Lys Lys Pro Gly Ala
1               5                   10                  15

tca gtg aag gtc tcc tgc aag gct tct gga tac acc ttc acc ggc tcc 96
Ser Val Lys Val Ser Cys Lys Ala Ser Gly Tyr Thr Phe Thr Gly Ser
            20                  25                  30

tat att cac tgg gtg cga cag gcc cct gga caa ggg ctt gag tgg atg 144
Tyr Ile His Trp Val Arg Gln Ala Pro Gly Gln Gly Leu Glu Trp Met
        35                  40                  45

gga cgg atg aac cct aac agt ggt gac aca aac tat gca cag aag ttt 192
Gly Arg Met Asn Pro Asn Ser Gly Asp Thr Asn Tyr Ala Gln Lys Phe
    50                  55                  60

cag ggc cgg gtc acc atg acc agg gac acg tcc atc agc aca gcc tac 240
Gln Gly Arg Val Thr Met Thr Arg Asp Thr Ser Ile Ser Thr Ala Tyr
65                  70                  75                  80

atg gag ctg agc agg ctg aga tct gac gac acg gcc gtg tac tac tgt 288
Met Glu Leu Ser Arg Leu Arg Ser Asp Asp Thr Ala Val Tyr Tyr Cys
                85                  90                  95

gcg acg gag gga gtg gct tta cgt ccc ggt gct ttt gat ttc tgg ggc 336
Ala Thr Glu Gly Val Ala Leu Arg Pro Gly Ala Phe Asp Phe Trp Gly
            100                 105                 110

caa ggg acc cag ctc acc gtt tta ggt gcg gcc gca 372
Gln Gly Thr Gln Leu Thr Val Leu Gly Ala Ala Ala
        115                 120

<210> 17
<211> 124
<212> PRT
<213> E. coli

<400> 17

Glu Val Gln Leu Leu Gln Ser Gly Ala Glu Val Lys Lys Pro Gly Ala
1               5                   10                  15

Ser Val Lys Val Ser Cys Lys Ala Ser Gly Tyr Thr Phe Thr Gly Ser
            20                  25                  30

Tyr Ile His Trp Val Arg Gln Ala Pro Gly Gln Gly Leu Glu Trp Met
        35                  40                  45

Gly Arg Met Asn Pro Asn Ser Gly Asp Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Thr Glu Gly Val Ala Leu Arg Pro Gly Ala Phe Asp Phe Trp Gly
            100                 105                 110

Gln Gly Thr Gln Leu Thr Val Leu Gly Ala Ala Ala
        115                 120

<210>  18
<211>  750
<212>  DNA
<213>  E. coli

<220>
<221>  CDS
<222>  (1)..(750)

<400>  18
gag gag gtg cag ctg gtg cag tct gga gga ggc ttg atc cag ccg ggg        48
Glu Glu Val Gln Leu Val Gln Ser Gly Gly Gly Leu Ile Gln Pro Gly
1               5                   10                  15

ggg tcc ctg aga ctc tct tgt gta gcc tct gag ttc aac gtc aga agc        96
Gly Ser Leu Arg Leu Ser Cys Val Ala Ser Glu Phe Asn Val Arg Ser
            20                  25                  30

aac tac atg agc tgg gtc cgc cag gct cca ggg aag ggg ctg gag tgg       144
Asn Tyr Met Ser Trp Val Arg Gln Ala Pro Gly Lys Gly Leu Glu Trp
        35                  40                  45

gtc tca gtt atg tat gac ggc ggt agt aca tac tac gca gac tcc gtg       192
Val Ser Val Met Tyr Asp Gly Gly Ser Thr Tyr Tyr Ala Asp Ser Val
    50                  55                  60

aag ggc cga ttc acc atc tcc aga gac aat tct aag aac acg gtg tat       240
Lys Gly Arg Phe Thr Ile Ser Arg Asp Asn Ser Lys Asn Thr Val Tyr
65                  70                  75                  80

ctt caa atg aac agc ctg aga gcc gag gac acg gcc gtc tat tac tgt       288
Leu Gln Met Asn Ser Leu Arg Ala Glu Asp Thr Ala Val Tyr Tyr Cys
                85                  90                  95

gcg aga ggc gga ttg ggg ttg cct aca atc gcg tct tgg gag atc tgg       336
Ala Arg Gly Gly Leu Gly Leu Pro Thr Ile Ala Ser Trp Glu Ile Trp
            100                 105                 110

ggc caa ggg aca atg gtc acc gtc tct tca ggt gga ggc ggt tct ggc       384
Gly Gln Gly Thr Met Val Thr Val Ser Ser Gly Gly Gly Gly Ser Gly
        115                 120                 125

gga ggt ggc tct ggc ggt ggc gga tcg tcc tat gtg ctg act cag cca       432
Gly Gly Gly Ser Gly Gly Gly Gly Ser Ser Tyr Val Leu Thr Gln Pro

130                 135                 140

ccc tcg gtg tca gtg gcc cca gga aag acg gcc acg att acc tgt gcg        480
Pro Ser Val Ser Val Ala Pro Gly Lys Thr Ala Thr Ile Thr Cys Ala
145                 150                 155                 160

gga aac aat ata gga agt aac agt gta tac tgg tac cag cag aaa cca        528
Gly Asn Asn Ile Gly Ser Asn Ser Val Tyr Trp Tyr Gln Gln Lys Pro
                165                 170                 175

ggc ctg gcc cct gta ctg gtc gtc tat gat gat aga gac cgg ccc tca        576
Gly Leu Ala Pro Val Leu Val Val Tyr Asp Asp Arg Asp Arg Pro Ser
            180                 185                 190

ggg atc cct gag cga ttc tct ggc tcc aaa tcc ggg aac acg gcc acc        624
Gly Ile Pro Glu Arg Phe Ser Gly Ser Lys Ser Gly Asn Thr Ala Thr
        195                 200                 205

ctg acc atc agc agg gtc gag gcc ggg gat gag gcc gac tat tct tgt        672
Leu Thr Ile Ser Arg Val Glu Ala Gly Asp Glu Ala Asp Tyr Ser Cys
    210                 215                 220

cag gtg tgg gat cct agt agt gat cac ctc tat gtc ttc gga act ggg        720
Gln Val Trp Asp Pro Ser Ser Asp His Leu Tyr Val Phe Gly Thr Gly
225                 230                 235                 240

acc cag ctc acc gtt tta ggt gcg gcc gca                                750
Thr Gln Leu Thr Val Leu Gly Ala Ala Ala
                245                 250

<210>  19
<211>  250
<212>  PRT
<213>  E. coli

<400>  19

Glu Glu Val Gln Leu Val Gln Ser Gly Gly Gly Leu Ile Gln Pro Gly
1               5                   10                  15

Gly Ser Leu Arg Leu Ser Cys Val Ala Ser Glu Phe Asn Val Arg Ser
            20                  25                  30

Asn Tyr Met Ser Trp Val Arg Gln Ala Pro Gly Lys Gly Leu Glu Trp
        35                  40                  45

Val Ser Val Met Tyr Asp Gly Gly Ser Thr Tyr Tyr Ala Asp Ser Val
    50                  55                  60

Lys Gly Arg Phe Thr Ile Ser Arg Asp Asn Ser Lys Asn Thr Val Tyr
65                  70                  75                  80

Leu Gln Met Asn Ser Leu Arg Ala Glu Asp Thr Ala Val Tyr Tyr Cys
                85                  90                  95

Ala Arg Gly Gly Leu Gly Leu Pro Thr Ile Ala Ser Trp Glu Ile Trp
            100                 105                 110

Gly Gln Gly Thr Met Val Thr Val Ser Ser Gly Gly Gly Gly Ser Gly
        115                 120                 125

Gly Gly Gly Ser Gly Gly Gly Gly Ser Ser Tyr Val Leu Thr Gln Pro
    130                 135                 140

Pro Ser Val Ser Val Ala Pro Gly Lys Thr Ala Thr Ile Thr Cys Ala
145                 150                 155                 160

Gly Asn Asn Ile Gly Ser Asn Ser Val Tyr Trp Tyr Gln Gln Lys Pro
                165                 170                 175

Gly Leu Ala Pro Val Leu Val Val Tyr Asp Asp Arg Asp Arg Pro Ser
            180                 185                 190

Gly Ile Pro Glu Arg Phe Ser Gly Ser Lys Ser Gly Asn Thr Ala Thr
        195                 200                 205

Leu Thr Ile Ser Arg Val Glu Ala Gly Asp Glu Ala Asp Tyr Ser Cys
    210                 215                 220

Gln Val Trp Asp Pro Ser Ser Asp His Leu Tyr Val Phe Gly Thr Gly
225                 230                 235                 240

Thr Gln Leu Thr Val Leu Gly Ala Ala Ala
                245                 250

<210>  20
<211>  756
<212>  DNA
<213>  E. coli

<220>
<221>  CDS
<222>  (1)..(756)

<400>  20
gag gag gtg cag ctg gtg gag tct ggg gga ggc ttg gtc cag cct ggg      48
Glu Glu Val Gln Leu Val Glu Ser Gly Gly Gly Leu Val Gln Pro Gly
1               5                   10                  15

ggg tcc ctg aga ctc tcc tgt gca gcc tct gga ttc acc gtc agt agc      96
Gly Ser Leu Arg Leu Ser Cys Ala Ala Ser Gly Phe Thr Val Ser Ser
            20                  25                  30

aac tac atg agc tgg gtc cgc cag gct cca ggg aag ggg ctg gag tgg      144

Asn Tyr Met Ser Trp Val Arg Gln Ala Pro Gly Lys Gly Leu Glu Trp
        35                  40                  45

gtc tca gtt gtt tat agc ggt ggt agc aca tac tac gca gac tcc gtg        192
Val Ser Val Val Tyr Ser Gly Gly Ser Thr Tyr Tyr Ala Asp Ser Val
    50                  55                  60

aag ggc cga ttc acc atc tcc aga gac aat tcc aag aac acg ctg tat        240
Lys Gly Arg Phe Thr Ile Ser Arg Asp Asn Ser Lys Asn Thr Leu Tyr
65                  70                  75                  80

ctt caa atg aac agc ctg aga gct gag gac acg gct gtg tat tac tgt        288
Leu Gln Met Asn Ser Leu Arg Ala Glu Asp Thr Ala Val Tyr Tyr Cys
                85                  90                  95

gcg aga gac cta ggg ggg act aca gtt tgg cgc tac tac ggt atg gac        336
Ala Arg Asp Leu Gly Gly Thr Thr Val Trp Arg Tyr Tyr Gly Met Asp
            100                 105                 110

gtc tgg ggc caa ggg acc acg gtc acc gtc tcc tca ggt gga ggc ggt        384
Val Trp Gly Gln Gly Thr Thr Val Thr Val Ser Ser Gly Gly Gly Gly
        115                 120                 125

tca ggc gga ggt ggc tct ggc ggt ggc gga tcg tcc tat gtg ctg act        432
Ser Gly Gly Gly Gly Ser Gly Gly Gly Gly Ser Ser Tyr Val Leu Thr
    130                 135                 140

cag cca ccc tcg gtg tca gtg gcc cca gga aag acg gcc acg att acc        480
Gln Pro Pro Ser Val Ser Val Ala Pro Gly Lys Thr Ala Thr Ile Thr
145                 150                 155                 160

tgt gcg gga aac aat ata gga agt aac agt gta tac tgg tac cag cag        528
Cys Ala Gly Asn Asn Ile Gly Ser Asn Ser Val Tyr Trp Tyr Gln Gln
                165                 170                 175

aaa cca ggc ctg gcc cct gta ctg gtc gtc tat gat gat aga gac cgg        576
Lys Pro Gly Leu Ala Pro Val Leu Val Val Tyr Asp Asp Arg Asp Arg
            180                 185                 190

ccc tca ggg atc cct ggg cga ttc tct ggc tcc aaa tcc ggg aac acg        624
Pro Ser Gly Ile Pro Gly Arg Phe Ser Gly Ser Lys Ser Gly Asn Thr
        195                 200                 205

gcc acc ctg acc atc agc agg gtc gag gcc ggg gat gag gcc gac tat        672
Ala Thr Leu Thr Ile Ser Arg Val Glu Ala Gly Asp Glu Ala Asp Tyr
    210                 215                 220

tct tgt cag gtg tgg gat cct agt agt gat cac ctc tat gtc ttc gga        720
Ser Cys Gln Val Trp Asp Pro Ser Ser Asp His Leu Tyr Val Phe Gly
225                 230                 235                 240

act ggg acc cag ctc acc gtt tta ggt gcg gcc gca                        756
Thr Gly Thr Gln Leu Thr Val Leu Gly Ala Ala Ala
                245                 250

<210>  21
<211>  252
<212>  PRT
<213>  E. coli

<400> 21

Glu Glu Val Gln Leu Val Glu Ser Gly Gly Gly Leu Val Gln Pro Gly
1               5                   10                  15

Gly Ser Leu Arg Leu Ser Cys Ala Ala Ser Gly Phe Thr Val Ser Ser
            20                  25                  30

Asn Tyr Met Ser Trp Val Arg Gln Ala Pro Gly Lys Gly Leu Glu Trp
        35                  40                  45

Val Ser Val Val Tyr Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Leu Gly Gly Thr Thr Val Trp Arg Tyr Tyr Gly Met Asp
            100                 105                 110

Val Trp Gly Gln Gly Thr Thr Val Thr Val Ser Ser Gly Gly Gly Gly
        115                 120                 125

Ser Gly Gly Gly Gly Ser Gly Gly Gly Gly Ser Ser Tyr Val Leu Thr
    130                 135                 140

Gln Pro Pro Ser Val Ser Val Ala Pro Gly Lys Thr Ala Thr Ile Thr
145                 150                 155                 160

Cys Ala Gly Asn Asn Ile Gly Ser Asn Ser Val Tyr Trp Tyr Gln Gln
                165                 170                 175

Lys Pro Gly Leu Ala Pro Val Leu Val Val Tyr Asp Asp Arg Asp Arg
            180                 185                 190

Pro Ser Gly Ile Pro Gly Arg Phe Ser Gly Ser Lys Ser Gly Asn Thr
        195                 200                 205

Ala Thr Leu Thr Ile Ser Arg Val Glu Ala Gly Asp Glu Ala Asp Tyr
    210                 215                 220

Ser Cys Gln Val Trp Asp Pro Ser Ser Asp His Leu Tyr Val Phe Gly
225                 230                 235                 240

Thr Gly Thr Gln Leu Thr Val Leu Gly Ala Ala Ala
                245                 250

<210>  22
<211>  735
<212>  DNA
<213>  E. coli

<220>
<221>  CDS
<222>  (1)..(735)

<400>  22
gag gag gtg cag ctg gtg gag tct gga gga gac ttg atc cag cct ggg        48
Glu Glu Val Gln Leu Val Glu Ser Gly Gly Asp Leu Ile Gln Pro Gly
1               5                   10                  15

ggg tcc ctg aga ctc tcc tgt gca gcc tct ggg ttt acc gtc ggt agc        96
Gly Ser Leu Arg Leu Ser Cys Ala Ala Ser Gly Phe Thr Val Gly Ser
            20                  25                  30

aac tac atg agc tgg gtc cgc cag gct cca ggg aag ggg ctg gaa tgg       144
Asn Tyr Met Ser Trp Val Arg Gln Ala Pro Gly Lys Gly Leu Glu Trp
        35                  40                  45

gtc tca gtt att tat agc ggt ggt agt aca tac tac gca gac tcc gtg       192
Val Ser Val Ile Tyr Ser Gly Gly Ser Thr Tyr Tyr Ala Asp Ser Val
    50                  55                  60

aag ggc cga ttc acc atc tcc aga gac aat tcc aag aac acg ctg tat       240
Lys Gly Arg Phe Thr Ile Ser Arg Asp Asn Ser Lys Asn Thr Leu Tyr
65                  70                  75                  80

ctt caa atg aac agc ctg aga gcc gag gac acg gcc gtg tat tac tgt       288
Leu Gln Met Asn Ser Leu Arg Ala Glu Asp Thr Ala Val Tyr Tyr Cys
                85                  90                  95

gtg aga gat agg ggt gat gct ttt gat atc tgg ggc caa ggg aca atg       336
Val Arg Asp Arg Gly Asp Ala Phe Asp Ile Trp Gly Gln Gly Thr Met
            100                 105                 110

gtc acc gtc tct tca ggt gga ggc gtt cca ggc gga ggt ggc tct ggc       384
Val Thr Val Ser Ser Gly Gly Gly Val Pro Gly Gly Gly Gly Ser Gly
        115                 120                 125

ggt ggc gga tcg tcc tat gcg ctg act cag cca ccc tcg gtg tca gtg       432
Gly Gly Gly Ser Ser Tyr Ala Leu Thr Gln Pro Pro Ser Val Ser Val
    130                 135                 140

gcc cca gga aag acg gcc acg att acc tgt gcg gga aac aat ata gga       480
Ala Pro Gly Lys Thr Ala Thr Ile Thr Cys Ala Gly Asn Asn Ile Gly
145                 150                 155                 160

agt aac agt gta tac tgg tac cag cag aaa cca ggc ctg gcc cct gta       528
Ser Asn Ser Val Tyr Trp Tyr Gln Gln Lys Pro Gly Leu Ala Pro Val
                165                 170                 175

ctg gtc gtc tat gat gat agc gac cgg ccc tca ggg atg tct gag cga       576

Leu Val Val Tyr Asp Asp Ser Asp Arg Pro Ser Gly Met Ser Glu Arg
            180                 185                 190

ttc tct ggc tcc aaa tcc ggg aac acg gcc acc ctg acc atc agc agg 624
Phe Ser Gly Ser Lys Ser Gly Asn Thr Ala Thr Leu Thr Ile Ser Arg
        195                 200                 205

gtc gag gcc ggg gat gag gcc gac tat tct tgt cag gtg tgg gat cct 672
Val Glu Ala Gly Asp Glu Ala Asp Tyr Ser Cys Gln Val Trp Asp Pro
    210                 215                 220

agt agt gat cac ctc tat gtc ttc gga act ggg acc cag ctc acc gtt 720
Ser Ser Asp His Leu Tyr Val Phe Gly Thr Gly Thr Gln Leu Thr Val
225                 230                 235                 240

tta ggt gcg gcc gca 735
Leu Gly Ala Ala Ala
                245

<210> 23
<211> 245
<212> PRT
<213> E. coli

<400> 23

Glu Glu Val Gln Leu Val Glu Ser Gly Gly Asp Leu Ile Gln Pro Gly
1               5                   10                  15

Gly Ser Leu Arg Leu Ser Cys Ala Ala Ser Gly Phe Thr Val Gly Ser
            20                  25                  30

Asn Tyr Met Ser Trp Val Arg Gln Ala Pro Gly Lys Gly Leu Glu Trp
        35                  40                  45

Val Ser Val Ile Tyr Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Val Arg Asp Arg Gly Asp Ala Phe Asp Ile Trp Gly Gln Gly Thr Met
            100                 105                 110

Val Thr Val Ser Ser Gly Gly Gly Val Pro Gly Gly Gly Gly Ser Gly
        115                 120                 125

Gly Gly Gly Ser Ser Tyr Ala Leu Thr Gln Pro Pro Ser Val Ser Val
    130                 135                 140

Ala Pro Gly Lys Thr Ala Thr Ile Thr Cys Ala Gly Asn Asn Ile Gly
145                 150                 155                 160

Ser Asn Ser Val Tyr Trp Tyr Gln Gln Lys Pro Gly Leu Ala Pro Val
                165                 170                 175

Leu Val Val Tyr Asp Asp Ser Asp Arg Pro Ser Gly Met Ser Glu Arg
            180                 185                 190

Phe Ser Gly Ser Lys Ser Gly Asn Thr Ala Thr Leu Thr Ile Ser Arg
        195                 200                 205

Val Glu Ala Gly Asp Glu Ala Asp Tyr Ser Cys Gln Val Trp Asp Pro
    210                 215                 220

Ser Ser Asp His Leu Tyr Val Phe Gly Thr Gly Thr Gln Leu Thr Val
225                 230                 235                 240

Leu Gly Ala Ala Ala
                245

<210>  24
<211>  741
<212>  DNA
<213>  E. coli

<220>
<221>  CDS
<222>  (1)..(741)

<400>  24
gag cag gtg cag ctg cag gag tct ggg gga ggc ttg gta cag cct ggg       48
Glu Gln Val Gln Leu Gln Glu Ser Gly Gly Gly Leu Val Gln Pro Gly
1               5                   10                  15

ggg tcc ctg aga ctc tcc tgt gca gcc tct gga ttc acc ttt agt act       96
Gly Ser Leu Arg Leu Ser Cys Ala Ala Ser Gly Phe Thr Phe Ser Thr
            20                  25                  30

tat gcc atg agc tgg gtc cgc cag gct cca ggg aag ggg ctg gag tgg      144
Tyr Ala Met Ser Trp Val Arg Gln Ala Pro Gly Lys Gly Leu Glu Trp
        35                  40                  45

gtc tca gtt att agt ggt agt ggt cat aca aca aac tac gcc gac tcc      192
Val Ser Val Ile Ser Gly Ser Gly His Thr Thr Asn Tyr Ala Asp Ser
    50                  55                  60

gtg aag ggc cgc gtc acc ata tcc aga gac aat tcc aag aac aca cta      240
Val Lys Gly Arg Val Thr Ile Ser Arg Asp Asn Ser Lys Asn Thr Leu
65                  70                  75                  80

tat ctg caa atc aac agc ctg aga gcc gac gac acg gcc gtg tat tac 288
Tyr Leu Gln Ile Asn Ser Leu Arg Ala Asp Asp Thr Ala Val Tyr Tyr
                85                  90                  95

tgt gcg aga gat gtg tta gtc cta cag aat gct ttt gat atc tgg ggc 336
Cys Ala Arg Asp Val Leu Val Leu Gln Asn Ala Phe Asp Ile Trp Gly
            100                 105                 110

caa ggg acc acg gtc acc gtc tcc tca ggt gga ggt ggt tca ggc gga 384
Gln Gly Thr Thr Val Thr Val Ser Ser Gly Gly Gly Gly Ser Gly Gly
        115                 120                 125

ggt ggc tct ggc ggt ggc gga tcg gat gtt gtg atg acc cag tct cca 432
Gly Gly Ser Gly Gly Gly Gly Ser Asp Val Val Met Thr Gln Ser Pro
    130                 135                 140

tcc tca ctg tct gca tct gta gga gac aga gtc acc atc act tgt cgg 480
Ser Ser Leu Ser Ala Ser Val Gly Asp Arg Val Thr Ile Thr Cys Arg
145                 150                 155                 160

gcg agt cag ggt att agc agg tgg tta gcc tgg tat caa cag aaa cca 528
Ala Ser Gln Gly Ile Ser Arg Trp Leu Ala Trp Tyr Gln Gln Lys Pro
                165                 170                 175

ggg aaa gcc cct aag ctc ctg atc tac gct gca tcc agt ttg caa agt 576
Gly Lys Ala Pro Lys Leu Leu Ile Tyr Ala Ala Ser Ser Leu Gln Ser
            180                 185                 190

ggg gtc cca tca agg ttc agt ggc agt gga tct ggg aca gat ttc act 624
Gly Val Pro Ser Arg Phe Ser Gly Ser Gly Ser Gly Thr Asp Phe Thr
        195                 200                 205

ctc acc atc agc agt ctg caa cct gaa gat ttt gca act tac atc tgt 672
Leu Thr Ile Ser Ser Leu Gln Pro Glu Asp Phe Ala Thr Tyr Ile Cys
    210                 215                 220

caa cag agt tac agt agg ccg ctc act ttc ggc gga ggg acc aag gtg 720
Gln Gln Ser Tyr Ser Arg Pro Leu Thr Phe Gly Gly Gly Thr Lys Val
225                 230                 235                 240

gaa atc aaa cgt gcg gcc gca 741
Glu Ile Lys Arg Ala Ala Ala
                245

<210> 25
<211> 247
<212> PRT
<213> E. coli

<400> 25

Glu Gln Val Gln Leu Gln Glu Ser Gly Gly Gly Leu Val Gln Pro Gly
1               5                   10                  15

Gly Ser Leu Arg Leu Ser Cys Ala Ala Ser Gly Phe Thr Phe Ser Thr
            20                  25                  30

Tyr Ala Met Ser Trp Val Arg Gln Ala Pro Gly Lys Gly Leu Glu Trp

35                  40                  45

Val Ser Val Ile Ser Gly Ser Gly His Thr Thr Asn Tyr Ala Asp Ser
    50                  55                  60

Val Lys Gly Arg Val Thr Ile Ser Arg Asp Asn Ser Lys Asn Thr Leu
65                  70                  75                  80

Tyr Leu Gln Ile Asn Ser Leu Arg Ala Asp Asp Thr Ala Val Tyr Tyr
                85                  90                  95

Cys Ala Arg Asp Val Leu Val Leu Gln Asn Ala Phe Asp Ile Trp Gly
            100                 105                 110

Gln Gly Thr Thr Val Thr Val Ser Ser Gly Gly Gly Gly Ser Gly Gly
        115                 120                 125

Gly Gly Ser Gly Gly Gly Gly Ser Asp Val Val Met Thr Gln Ser Pro
    130                 135                 140

Ser Ser Leu Ser Ala Ser Val Gly Asp Arg Val Thr Ile Thr Cys Arg
145                 150                 155                 160

Ala Ser Gln Gly Ile Ser Arg Trp Leu Ala Trp Tyr Gln Gln Lys Pro
                165                 170                 175

Gly Lys Ala Pro Lys Leu Leu Ile Tyr Ala Ala Ser Ser Leu Gln Ser
            180                 185                 190

Gly Val Pro Ser Arg Phe Ser Gly Ser Gly Ser Gly Thr Asp Phe Thr
        195                 200                 205

Leu Thr Ile Ser Ser Leu Gln Pro Glu Asp Phe Ala Thr Tyr Ile Cys
    210                 215                 220

Gln Gln Ser Tyr Ser Arg Pro Leu Thr Phe Gly Gly Gly Thr Lys Val
225                 230                 235                 240

Glu Ile Lys Arg Ala Ala Ala
                245

<210>  26
<211>  771
<212>  DNA
<213>  E. coli

<220>
<221> CDS
<222> (1)..(771)

<400> 26
gag cag gtg cag ctg gtg cag tct ggg gcg gag gtg aag aag cct ggg 48
Glu Gln Val Gln Leu Val Gln Ser Gly Ala Glu Val Lys Lys Pro Gly
1               5                   10                  15

gcc tca gtg aga gtt tcc tgc cag gca tct gga tac aca ttc agc agg 96
Ala Ser Val Arg Val Ser Cys Gln Ala Ser Gly Tyr Thr Phe Ser Arg
            20                  25                  30

tac cat atg cac tgg gtg cga cag gcc cct gga caa ggg ctt gag tgg 144
Tyr His Met His Trp Val Arg Gln Ala Pro Gly Gln Gly Leu Glu Trp
        35                  40                  45

atg gga gtg atc gac ccc aat agt ggt aga gta agt tac tca cag aag 192
Met Gly Val Ile Asp Pro Asn Ser Gly Arg Val Ser Tyr Ser Gln Lys
    50                  55                  60

ttc cag gac aga gtt acc atg acc agg gac acg tcc acg agc aca gta 240
Phe Gln Asp Arg Val Thr Met Thr Arg Asp Thr Ser Thr Ser Thr Val
65                  70                  75                  80

tac atg gag ctg aac agc ccg aga tct gag gac acg gcc gtt tat tat 288
Tyr Met Glu Leu Asn Ser Pro Arg Ser Glu Asp Thr Ala Val Tyr Tyr
                85                  90                  95

tgt gcg aga gat cga gga tat tgt aat ggt ggc agg tgc ttt atg gat 336
Cys Ala Arg Asp Arg Gly Tyr Cys Asn Gly Gly Arg Cys Phe Met Asp
            100                 105                 110

gca ttt gac tac tgg ggc cag ggg aca atg gtc acc gtc tct tca ggt 384
Ala Phe Asp Tyr Trp Gly Gln Gly Thr Met Val Thr Val Ser Ser Gly
        115                 120                 125

gga ggc ggt tta ggc gga ggt ggc tct ggc ggt ggc gga tcg tcc tat 432
Gly Gly Gly Leu Gly Gly Gly Gly Ser Gly Gly Gly Gly Ser Ser Tyr
    130                 135                 140

gtg ctg act cac cca ccc tca ttg tct ggg gcc cca ggg cag agc atc 480
Val Leu Thr His Pro Pro Ser Leu Ser Gly Ala Pro Gly Gln Ser Ile
145                 150                 155                 160

acc atc tcc tgc act ggg agc agt tcc aac atc ggg gca ggt ttt cat 528
Thr Ile Ser Cys Thr Gly Ser Ser Ser Asn Ile Gly Ala Gly Phe His
                165                 170                 175

ata cac tgg tac cag cag ttt cca aaa aca gcc ccc aaa ctc ctt atc 576
Ile His Trp Tyr Gln Gln Phe Pro Lys Thr Ala Pro Lys Leu Leu Ile
            180                 185                 190

tat ggt agt agt aat cga ccc tca ggg gtc cct gac cgc ttc tct ggc 624
Tyr Gly Ser Ser Asn Arg Pro Ser Gly Val Pro Asp Arg Phe Ser Gly
        195                 200                 205

tcc agg tct ggc tcc tca ggc tcc ctg gcc atc act ggg ctc cag gca 672
Ser Arg Ser Gly Ser Ser Gly Ser Leu Ala Ile Thr Gly Leu Gln Ala
    210                 215                 220

gac gat gag gct gat tat tac tgt gtg gga tgg gat ggc agc ctg agt 720
Asp Asp Glu Ala Asp Tyr Tyr Cys Val Gly Trp Asp Gly Ser Leu Ser
225                 230                 235                 240

ggt tat gtc ttc gga act ggg acc cag ctc acc gtt tta ggt gcg gcc 768
Gly Tyr Val Phe Gly Thr Gly Thr Gln Leu Thr Val Leu Gly Ala Ala
                245                 250                 255

gca 771
Ala

<210> 27
<211> 257
<212> PRT
<213> E. coli

<400> 27

Glu Gln Val Gln Leu Val Gln Ser Gly Ala Glu Val Lys Lys Pro Gly
1               5                   10                  15

Ala Ser Val Arg Val Ser Cys Gln Ala Ser Gly Tyr Thr Phe Ser Arg
            20                  25                  30

Tyr His Met His Trp Val Arg Gln Ala Pro Gly Gln Gly Leu Glu Trp
        35                  40                  45

Met Gly Val Ile Asp Pro Asn Ser Gly Arg Val Ser Tyr Ser Gln Lys
    50                  55                  60

Phe Gln Asp Arg Val Thr Met Thr Arg Asp Thr Ser Thr Ser Thr Val
65                  70                  75                  80

Tyr Met Glu Leu Asn Ser Pro Arg Ser Glu Asp Thr Ala Val Tyr Tyr
                85                  90                  95

Cys Ala Arg Asp Arg Gly Tyr Cys Asn Gly Gly Arg Cys Phe Met Asp
            100                 105                 110

Ala Phe Asp Tyr Trp Gly Gln Gly Thr Met Val Thr Val Ser Ser Gly
        115                 120                 125

Gly Gly Gly Leu Gly Gly Gly Gly Ser Gly Gly Gly Gly Ser Ser Tyr
    130                 135                 140

Val Leu Thr His Pro Pro Ser Leu Ser Gly Ala Pro Gly Gln Ser Ile
145                 150                 155                 160

Thr Ile Ser Cys Thr Gly Ser Ser Ser Asn Ile Gly Ala Gly Phe His

165                 170                 175

Ile His Trp Tyr Gln Gln Phe Pro Lys Thr Ala Pro Lys Leu Leu Ile
        180                 185                 190

Tyr Gly Ser Ser Asn Arg Pro Ser Gly Val Pro Asp Arg Phe Ser Gly
    195                 200                 205

Ser Arg Ser Gly Ser Ser Gly Ser Leu Ala Ile Thr Gly Leu Gln Ala
    210                 215                 220

Asp Asp Glu Ala Asp Tyr Tyr Cys Val Gly Trp Asp Gly Ser Leu Ser
225                 230                 235                 240

Gly Tyr Val Phe Gly Thr Gly Thr Gln Leu Thr Val Leu Gly Ala Ala
                245                 250                 255

Ala

<210>  28
<211>  765
<212>  DNA
<213>  E. coli

<220>
<221>  CDS
<222>  (1)..(765)

<400>  28
gag cag gtg cag ctg gtg cag tct ggg gga ggc gtg gtc cag cct ggg      48
Glu Gln Val Gln Leu Val Gln Ser Gly Gly Gly Val Val Gln Pro Gly
1               5                   10                  15

agg tcc ctg aga ctc tcc tgt gca gcc tct gga ttc agc ttc agt aac      96
Arg Ser Leu Arg Leu Ser Cys Ala Ala Ser Gly Phe Ser Phe Ser Asn
            20                  25                  30

tat gtt atg cac tgg gtc cgc cag gct cca ggc aag ggg ctg gag tgg      144
Tyr Val Met His Trp Val Arg Gln Ala Pro Gly Lys Gly Leu Glu Trp
        35                  40                  45

gtg gca gtt ata tca cat gat gga agc aat aaa tac tac gca gac tcc      192
Val Ala Val Ile Ser His Asp Gly Ser Asn Lys Tyr Tyr Ala Asp Ser
    50                  55                  60

gtg aag ggc cga ttc acc atc tcc aga gac aat tcc aag aac acg cta      240
Val Lys Gly Arg Phe Thr Ile Ser Arg Asp Asn Ser Lys Asn Thr Leu
65                  70                  75                  80

tat ctg caa atg aaa agc ctg aga cct gag gac acg gct gtg tat tac      288
Tyr Leu Gln Met Lys Ser Leu Arg Pro Glu Asp Thr Ala Val Tyr Tyr
                85                  90                  95

tgt gcg aga agt agt ggc tgg tac ctt ctc ttt gat gct ttt gat atc     336
Cys Ala Arg Ser Ser Gly Trp Tyr Leu Leu Phe Asp Ala Phe Asp Ile
            100                 105                 110

tgg ggc caa ggg aca atg gtc acc gtc tct tca ggt gga ggc ggt tca     384
Trp Gly Gln Gly Thr Met Val Thr Val Ser Ser Gly Gly Gly Gly Ser
        115                 120                 125

ggc gga ggt ggc tct ggc ggt ggc gga tcg gac atc cag atg acc cag     432
Gly Gly Gly Gly Ser Gly Gly Gly Gly Ser Asp Ile Gln Met Thr Gln
    130                 135                 140

tct cca gac tcc ctg cct gtg tct ctg ggc gag agg gcc acc atc aac     480
Ser Pro Asp Ser Leu Pro Val Ser Leu Gly Glu Arg Ala Thr Ile Asn
145                 150                 155                 160

tgc agg tcc agc cag agt gtt tta tac agc tcc aac aat aag aac tac     528
Cys Arg Ser Ser Gln Ser Val Leu Tyr Ser Ser Asn Asn Lys Asn Tyr
                165                 170                 175

tta gct tgg tac cag cag aaa cca gga cag cct cct aag ctg ctc att     576
Leu Ala Trp Tyr Gln Gln Lys Pro Gly Gln Pro Pro Lys Leu Leu Ile
            180                 185                 190

tac tgg gca tct acc cgg gaa tcc ggt gtc cct gac cga ttc agt ggc     624
Tyr Trp Ala Ser Thr Arg Glu Ser Gly Val Pro Asp Arg Phe Ser Gly
        195                 200                 205

agc ggg tct ggg aca gat ttc act ctc acc atc agc agc ctg cag gct     672
Ser Gly Ser Gly Thr Asp Phe Thr Leu Thr Ile Ser Ser Leu Gln Ala
    210                 215                 220

gaa gat gtg gca gtt tat tac tgt cag caa tat tat agg att ccg tgg     720
Glu Asp Val Ala Val Tyr Tyr Cys Gln Gln Tyr Tyr Arg Ile Pro Trp
225                 230                 235                 240

acg ttc ggc caa ggg acg aag gtg gaa atc aaa cgt gcg gcc gca         765
Thr Phe Gly Gln Gly Thr Lys Val Glu Ile Lys Arg Ala Ala Ala
                245                 250                 255


<210>  29
<211>  255
<212>  PRT
<213>  E. coli

<400>  29

Glu Gln Val Gln Leu Val Gln Ser Gly Gly Gly Val Val Gln Pro Gly
1               5                   10                  15


Arg Ser Leu Arg Leu Ser Cys Ala Ala Ser Gly Phe Ser Phe Ser Asn
            20                  25                  30


Tyr Val Met His Trp Val Arg Gln Ala Pro Gly Lys Gly Leu Glu Trp
        35                  40                  45

Val Ala Val Ile Ser His Asp Gly Ser Asn Lys Tyr Tyr Ala Asp Ser
    50                  55                  60

Val Lys Gly Arg Phe Thr Ile Ser Arg Asp Asn Ser Lys Asn Thr Leu
65                  70                  75                  80

Tyr Leu Gln Met Lys Ser Leu Arg Pro Glu Asp Thr Ala Val Tyr Tyr
                85                  90                  95

Cys Ala Arg Ser Ser Gly Trp Tyr Leu Leu Phe Asp Ala Phe Asp Ile
            100                 105                 110

Trp Gly Gln Gly Thr Met Val Thr Val Ser Ser Gly Gly Gly Gly Ser
        115                 120                 125

Gly Gly Gly Gly Ser Gly Gly Gly Gly Ser Asp Ile Gln Met Thr Gln
    130                 135                 140

Ser Pro Asp Ser Leu Pro Val Ser Leu Gly Glu Arg Ala Thr Ile Asn
145                 150                 155                 160

Cys Arg Ser Ser Gln Ser Val Leu Tyr Ser Ser Asn Asn Lys Asn Tyr
                165                 170                 175

Leu Ala Trp Tyr Gln Gln Lys Pro Gly Gln Pro Pro Lys Leu Leu Ile
            180                 185                 190

Tyr Trp Ala Ser Thr Arg Glu Ser Gly Val Pro Asp Arg Phe Ser Gly
        195                 200                 205

Ser Gly Ser Gly Thr Asp Phe Thr Leu Thr Ile Ser Ser Leu Gln Ala
    210                 215                 220

Glu Asp Val Ala Val Tyr Tyr Cys Gln Gln Tyr Tyr Arg Ile Pro Trp
225                 230                 235                 240

Thr Phe Gly Gln Gly Thr Lys Val Glu Ile Lys Arg Ala Ala Ala
                245                 250                 255

<210>  30
<211>  759
<212>  DNA
<213>  E. coli

<220>
<221>  CDS
<222>  (1)..(759)

<400> 30
gag gag gtg cag ctg ttg cag tct ggg gga ggt gtg gta cgg cct ggg 48
Glu Glu Val Gln Leu Leu Gln Ser Gly Gly Gly Val Val Arg Pro Gly
1               5                   10                  15

ggg tcc ctg aga ctc tcc tgt gca gcc tct gga ttc acc ttt gat gat 96
Gly Ser Leu Arg Leu Ser Cys Ala Ala Ser Gly Phe Thr Phe Asp Asp
            20                  25                  30

tat ggc atg acc tgg gtc cgc cag gct cca ggg aag ggg ctg gag tgg 144
Tyr Gly Met Thr Trp Val Arg Gln Ala Pro Gly Lys Gly Leu Glu Trp
        35                  40                  45

gtc tca gct att agt ggt agt ggt ggt agc aca tac tac gca gac tcc 192
Val Ser Ala Ile Ser Gly Ser Gly Gly Ser Thr Tyr Tyr Ala Asp Ser
    50                  55                  60

gtg aag ggc cgg ttc gcc atc tcc aga gac aat tcc aag aac acg ctg 240
Val Lys Gly Arg Phe Ala Ile Ser Arg Asp Asn Ser Lys Asn Thr Leu
65                  70                  75                  80

tat ctg caa atg aac agc ctg aga gcc gag gac acg gcc gta tat tac 288
Tyr Leu Gln Met Asn Ser Leu Arg Ala Glu Asp Thr Ala Val Tyr Tyr
                85                  90                  95

tgt gcg aaa tct cgc tac tat gat agt agt ggt tat tac tac acc gtg 336
Cys Ala Lys Ser Arg Tyr Tyr Asp Ser Ser Gly Tyr Tyr Tyr Thr Val
            100                 105                 110

cga cct gat gct ttt gat atc tgg ggc caa ggg gca atg gtc acc gtc 384
Arg Pro Asp Ala Phe Asp Ile Trp Gly Gln Gly Ala Met Val Thr Val
        115                 120                 125

tct tca ggt gga ggc ggt gga ggt ggc tct ggc ggt ggc gga tcg tct 432
Ser Ser Gly Gly Gly Gly Gly Gly Gly Ser Gly Gly Gly Gly Ser Ser
    130                 135                 140

tct gag ctg act caa cca ccc tca gtg tcc gtg tcc cca gga cag aca 480
Ser Glu Leu Thr Gln Pro Pro Ser Val Ser Val Ser Pro Gly Gln Thr
145                 150                 155                 160

gcc atc atc acc tgc tct gga gat aaa ttg ggg gat aaa tat gct tcc 528
Ala Ile Ile Thr Cys Ser Gly Asp Lys Leu Gly Asp Lys Tyr Ala Ser
                165                 170                 175

tgg tat cag cac agg cca ggc cag tcg cct gtc ttg gtc atc tat cag 576
Trp Tyr Gln His Arg Pro Gly Gln Ser Pro Val Leu Val Ile Tyr Gln
            180                 185                 190

gat tcc agg cgg ccc tca gac atc cct gag cga ttc tct ggc tcc aac 624
Asp Ser Arg Arg Pro Ser Asp Ile Pro Glu Arg Phe Ser Gly Ser Asn
        195                 200                 205

tct ggg aac aca gcc act ctg acc atc acc gag gcc cag gct ttg gat 672
Ser Gly Asn Thr Ala Thr Leu Thr Ile Thr Glu Ala Gln Ala Leu Asp
    210                 215                 220

gag gct gac tat tat tgt cag gcc tgg gcc ggc aga tct gtg gtc ttc 720
Glu Ala Asp Tyr Tyr Cys Gln Ala Trp Ala Gly Arg Ser Val Val Phe
225                 230                 235                 240

ggc ggg ggg acc cag ctc acc gtt tta ggt gcg gcc gca                759
Gly Gly Gly Thr Gln Leu Thr Val Leu Gly Ala Ala Ala
                245                 250

<210>  31
<211>  253
<212>  PRT
<213>  E. coli

<400>  31

Glu Glu Val Gln Leu Leu Gln Ser Gly Gly Gly Val Val Arg Pro Gly
1               5                   10                  15

Gly Ser Leu Arg Leu Ser Cys Ala Ala Ser Gly Phe Thr Phe Asp Asp
            20                  25                  30

Tyr Gly Met Thr Trp Val Arg Gln Ala Pro Gly Lys Gly Leu Glu Trp
        35                  40                  45

Val Ser Ala Ile Ser Gly Ser Gly Gly Ser Thr Tyr Tyr Ala Asp Ser
    50                  55                  60

Val Lys Gly Arg Phe Ala Ile Ser Arg Asp Asn Ser Lys Asn Thr Leu
65                  70                  75                  80

Tyr Leu Gln Met Asn Ser Leu Arg Ala Glu Asp Thr Ala Val Tyr Tyr
                85                  90                  95

Cys Ala Lys Ser Arg Tyr Tyr Asp Ser Ser Gly Tyr Tyr Tyr Thr Val
            100                 105                 110

Arg Pro Asp Ala Phe Asp Ile Trp Gly Gln Gly Ala Met Val Thr Val
        115                 120                 125

Ser Ser Gly Gly Gly Gly Gly Gly Gly Ser Gly Gly Gly Gly Ser Ser
    130                 135                 140

Ser Glu Leu Thr Gln Pro Pro Ser Val Ser Val Ser Pro Gly Gln Thr
145                 150                 155                 160

Ala Ile Ile Thr Cys Ser Gly Asp Lys Leu Gly Asp Lys Tyr Ala Ser
                165                 170                 175

Trp Tyr Gln His Arg Pro Gly Gln Ser Pro Val Leu Val Ile Tyr Gln
            180                 185                 190

Asp Ser Arg Arg Pro Ser Asp Ile Pro Glu Arg Phe Ser Gly Ser Asn
        195                 200                 205

Ser Gly Asn Thr Ala Thr Leu Thr Ile Thr Glu Ala Gln Ala Leu Asp
    210                 215                 220

Glu Ala Asp Tyr Tyr Cys Gln Ala Trp Ala Gly Arg Ser Val Val Phe
225                 230                 235                 240

Gly Gly Gly Thr Gln Leu Thr Val Leu Gly Ala Ala Ala
                245                 250

<210> 32
<211> 768
<212> DNA
<213> E. coli

<220>
<221> CDS
<222> (1)..(768)

<400> 32
gag gag gtg cag ctg ttg cag tct ggg gcg gag gtg aag aag cct ggg 48
Glu Glu Val Gln Leu Leu Gln Ser Gly Ala Glu Val Lys Lys Pro Gly
1               5                   10                  15

gcc tca gtg aga gtt tcc tgc cag gca tct gga tac aca ttc agc agg 96
Ala Ser Val Arg Val Ser Cys Gln Ala Ser Gly Tyr Thr Phe Ser Arg
            20                  25                  30

tac cat atg cac tgg gtg cga cag gcc cct gga caa ggg ctt gag tgg 144
Tyr His Met His Trp Val Arg Gln Ala Pro Gly Gln Gly Leu Glu Trp
        35                  40                  45

atg gga gtg atc gac ccc aat agt ggt aga gta agt tac tca cag aag 192
Met Gly Val Ile Asp Pro Asn Ser Gly Arg Val Ser Tyr Ser Gln Lys
    50                  55                  60

ttc cag gac aga gtc acc atg acc agg gac acg ttc acg agc aca gta 240
Phe Gln Asp Arg Val Thr Met Thr Arg Asp Thr Phe Thr Ser Thr Val
65                  70                  75                  80

tac atg gag ctg aac agc ctg aga tct gag gac acg gcc gtt tat tat 288
Tyr Met Glu Leu Asn Ser Leu Arg Ser Glu Asp Thr Ala Val Tyr Tyr
                85                  90                  95

tgt gcg aga gat cga gga tat tgt aat ggt ggc agg tgc ttt atg gat 336
Cys Ala Arg Asp Arg Gly Tyr Cys Asn Gly Gly Arg Cys Phe Met Asp
            100                 105                 110

gca ttt gac tac tgg ggc cag ggg acc acg gtc acc gtc tcc tca ggt 384
Ala Phe Asp Tyr Trp Gly Gln Gly Thr Thr Val Thr Val Ser Ser Gly
        115                 120                 125

gga ggc ggt tca ggc gga ggt ggc cct ggc ggt ggc gga tcg tcc tat 432
Gly Gly Gly Ser Gly Gly Gly Gly Pro Gly Gly Gly Gly Ser Ser Tyr

130                 135                 140

gtg ctg act cag cca ccc tca gcg tct ggg gcc ccc gga cag agg gtc        480
Val Leu Thr Gln Pro Pro Ser Ala Ser Gly Ala Pro Gly Gln Arg Val
145                 150                 155                 160

acc atc tct tgt tct gga agc aac tcc aac atc gga cgt aat tgg gta        528
Thr Ile Ser Cys Ser Gly Ser Asn Ser Asn Ile Gly Arg Asn Trp Val
                165                 170                 175

tac tgg tac cag caa ctc cca gga acg gcc ccc aaa ctc ctc atg ttt        576
Tyr Trp Tyr Gln Gln Leu Pro Gly Thr Ala Pro Lys Leu Leu Met Phe
            180                 185                 190

agg aat aat gaa cgg tcc tca ggg gtc cct gac cga ttc tct ggc tcc        624
Arg Asn Asn Glu Arg Ser Ser Gly Val Pro Asp Arg Phe Ser Gly Ser
        195                 200                 205

aag act ggc acc tca gcc tcc ctg gcc atc agt ggg ctc cgg tct gag        672
Lys Thr Gly Thr Ser Ala Ser Leu Ala Ile Ser Gly Leu Arg Ser Glu
    210                 215                 220

gat gag ggt gat tac tac tgt gca tca tgg gat gac agt ctg cat gct        720
Asp Glu Gly Asp Tyr Tyr Cys Ala Ser Trp Asp Asp Ser Leu His Ala
225                 230                 235                 240

tgg gtg ttc ggc ggg ggg acc cag ctc acc gtt tta ggt gcg gcc gca        768
Trp Val Phe Gly Gly Gly Thr Gln Leu Thr Val Leu Gly Ala Ala Ala
                245                 250                 255

<210>  33
<211>  256
<212>  PRT
<213>  E. coli

<400>  33

Glu Glu Val Gln Leu Leu Gln Ser Gly Ala Glu Val Lys Lys Pro Gly
1               5                   10                  15

Ala Ser Val Arg Val Ser Cys Gln Ala Ser Gly Tyr Thr Phe Ser Arg
            20                  25                  30

Tyr His Met His Trp Val Arg Gln Ala Pro Gly Gln Gly Leu Glu Trp
        35                  40                  45

Met Gly Val Ile Asp Pro Asn Ser Gly Arg Val Ser Tyr Ser Gln Lys
    50                  55                  60

Phe Gln Asp Arg Val Thr Met Thr Arg Asp Thr Phe Thr Ser Thr Val
65                  70                  75                  80

Tyr Met Glu Leu Asn Ser Leu Arg Ser Glu Asp Thr Ala Val Tyr Tyr
                85                  90                  95

Cys Ala Arg Asp Arg Gly Tyr Cys Asn Gly Gly Arg Cys Phe Met Asp
            100                 105                 110

Ala Phe Asp Tyr Trp Gly Gln Gly Thr Thr Val Thr Val Ser Ser Gly
        115                 120                 125

Gly Gly Gly Ser Gly Gly Gly Gly Pro Gly Gly Gly Gly Ser Ser Tyr
    130                 135                 140

Val Leu Thr Gln Pro Pro Ser Ala Ser Gly Ala Pro Gly Gln Arg Val
145                 150                 155                 160

Thr Ile Ser Cys Ser Gly Ser Asn Ser Asn Ile Gly Arg Asn Trp Val
                165                 170                 175

Tyr Trp Tyr Gln Gln Leu Pro Gly Thr Ala Pro Lys Leu Leu Met Phe
            180                 185                 190

Arg Asn Asn Glu Arg Ser Ser Gly Val Pro Asp Arg Phe Ser Gly Ser
        195                 200                 205

Lys Thr Gly Thr Ser Ala Ser Leu Ala Ile Ser Gly Leu Arg Ser Glu
    210                 215                 220

Asp Glu Gly Asp Tyr Tyr Cys Ala Ser Trp Asp Asp Ser Leu His Ala
225                 230                 235                 240

Trp Val Phe Gly Gly Gly Thr Gln Leu Thr Val Leu Gly Ala Ala Ala
                245                 250                 255

<210>  34
<211>  735
<212>  DNA
<213>  E. coli

<220>
<221>  CDS
<222>  (1)..(735)

<400>  34
gag cag gtg cag ctg gtg cag tct ggg gga ggc ttg gta cag cct ggg        48
Glu Gln Val Gln Leu Val Gln Ser Gly Gly Gly Leu Val Gln Pro Gly
1               5                   10                  15

ggg tcc ctg aga ctc tcc tgt gca gcc tct gga ttc acc ttt agc agc        96
Gly Ser Leu Arg Leu Ser Cys Ala Ala Ser Gly Phe Thr Phe Ser Ser
            20                  25                  30

tat gcc atg agc tgg gtc cgc cag gct cca ggg aag ggg ctg gag tgg       144

Tyr Ala Met Ser Trp Val Arg Gln Ala Pro Gly Lys Gly Leu Glu Trp
        35                  40                  45

gtc tca gct att agt ggt agt ggt ggt agc aca tac tac gca gac tcc 192
Val Ser Ala Ile Ser Gly Ser Gly Gly Ser Thr Tyr Tyr Ala Asp Ser
    50                  55                  60

gtg aag ggc cgg ttc acc atc tcc aga gag aat tcc aag aac acg cta 240
Val Lys Gly Arg Phe Thr Ile Ser Arg Glu Asn Ser Lys Asn Thr Leu
65                  70                  75                  80

tat ctg caa atg aat agc ctg aga gcc gag gac acg gct gtg tat tac 288
Tyr Leu Gln Met Asn Ser Leu Arg Ala Glu Asp Thr Ala Val Tyr Tyr
                85                  90                  95

tgt gcg aga caa aca aga gtc cgt gct ttt gat atc tgg ggc caa ggg 336
Cys Ala Arg Gln Thr Arg Val Arg Ala Phe Asp Ile Trp Gly Gln Gly
            100                 105                 110

aca atg gtc acc gtc tct tca ggt gga ggc ggt tca ggc gga ggt ggc 384
Thr Met Val Thr Val Ser Ser Gly Gly Gly Gly Ser Gly Gly Gly Gly
        115                 120                 125

tct ggc ggt ggc gga tcg gac atc cag atg acc cag tct cct tcc gcc 432
Ser Gly Gly Gly Gly Ser Asp Ile Gln Met Thr Gln Ser Pro Ser Ala
    130                 135                 140

ctg tct gca tct gta gga ggc aga gtc acc atc act tgc cgg gca agt 480
Leu Ser Ala Ser Val Gly Gly Arg Val Thr Ile Thr Cys Arg Ala Ser
145                 150                 155                 160

cag agc act agt agc gat tta aat tgg tat cag caa aga cca ggg aaa 528
Gln Ser Thr Ser Ser Asp Leu Asn Trp Tyr Gln Gln Arg Pro Gly Lys
                165                 170                 175

gcc cct aaa ctc ctg atc tct gtt gca tcc act tta caa agt gac gtc 576
Ala Pro Lys Leu Leu Ile Ser Val Ala Ser Thr Leu Gln Ser Asp Val
            180                 185                 190

cca tca agg ttc agt ggc agt ggt tct ggg aca gat ttc agt ctc acc 624
Pro Ser Arg Phe Ser Gly Ser Gly Ser Gly Thr Asp Phe Ser Leu Thr
        195                 200                 205

atc agc agt ctg caa cct gaa gac ttt gca act tac ttc tgt caa cag 672
Ile Ser Ser Leu Gln Pro Glu Asp Phe Ala Thr Tyr Phe Cys Gln Gln
    210                 215                 220

agt tac agc acc ccg tac act ttt ggc cag ggg acc aaa gtg gat atc 720
Ser Tyr Ser Thr Pro Tyr Thr Phe Gly Gln Gly Thr Lys Val Asp Ile
225                 230                 235                 240

aaa cgt gcg gcc gca 735
Lys Arg Ala Ala Ala
                245

<210> 35
<211> 245
<212> PRT
<213> E. coli

<400>  35

Glu Gln Val Gln Leu Val Gln Ser Gly Gly Gly Leu Val Gln Pro Gly
1               5                   10                  15

Gly Ser Leu Arg Leu Ser Cys Ala Ala Ser Gly Phe Thr Phe Ser Ser
            20                  25                  30

Tyr Ala Met Ser Trp Val Arg Gln Ala Pro Gly Lys Gly Leu Glu Trp
        35                  40                  45

Val Ser Ala Ile Ser Gly Ser Gly Gly Ser Thr Tyr Tyr Ala Asp Ser
    50                  55                  60

Val Lys Gly Arg Phe Thr Ile Ser Arg Glu Asn Ser Lys Asn Thr Leu
65                  70                  75                  80

Tyr Leu Gln Met Asn Ser Leu Arg Ala Glu Asp Thr Ala Val Tyr Tyr
                85                  90                  95

Cys Ala Arg Gln Thr Arg Val Arg Ala Phe Asp Ile Trp Gly Gln Gly
            100                 105                 110

Thr Met Val Thr Val Ser Ser Gly Gly Gly Gly Ser Gly Gly Gly Gly
        115                 120                 125

Ser Gly Gly Gly Gly Ser Asp Ile Gln Met Thr Gln Ser Pro Ser Ala
    130                 135                 140

Leu Ser Ala Ser Val Gly Gly Arg Val Thr Ile Thr Cys Arg Ala Ser
145                 150                 155                 160

Gln Ser Thr Ser Ser Asp Leu Asn Trp Tyr Gln Gln Arg Pro Gly Lys
                165                 170                 175

Ala Pro Lys Leu Leu Ile Ser Val Ala Ser Thr Leu Gln Ser Asp Val
            180                 185                 190

Pro Ser Arg Phe Ser Gly Ser Gly Ser Gly Thr Asp Phe Ser Leu Thr
        195                 200                 205

Ile Ser Ser Leu Gln Pro Glu Asp Phe Ala Thr Tyr Phe Cys Gln Gln
    210                 215                 220

Ser Tyr Ser Thr Pro Tyr Thr Phe Gly Gln Gly Thr Lys Val Asp Ile
225                 230                 235                 240

Lys Arg Ala Ala Ala
                245

<210> 36
<211> 765
<212> DNA
<213> E. coli

<220>
<221> CDS
<222> (1)..(765)

<400> 36
gag gag gtg cag ctg gtg gag tct ggg gga aac ttg gtt cag cct ggg      48
Glu Glu Val Gln Leu Val Glu Ser Gly Gly Asn Leu Val Gln Pro Gly
1               5                   10                  15

ggg tcc ctg aga ctc tcc tgt gca gcc tct gga ttc acc ttt agc agt      96
Gly Ser Leu Arg Leu Ser Cys Ala Ala Ser Gly Phe Thr Phe Ser Ser
            20                  25                  30

tat gcc atg agc tgg gtc cgc cag gct cca ggg aag ggg ctg gaa tgg      144
Tyr Ala Met Ser Trp Val Arg Gln Ala Pro Gly Lys Gly Leu Glu Trp
        35                  40                  45

gtc tca gct att agt gct agt ggt ggc acc aca tac tac gca gat tcc      192
Val Ser Ala Ile Ser Ala Ser Gly Gly Thr Thr Tyr Tyr Ala Asp Ser
    50                  55                  60

gtg aag ggc cgg ttc acc atc tcc aga gac aat tcc aag aac acg ctg      240
Val Lys Gly Arg Phe Thr Ile Ser Arg Asp Asn Ser Lys Asn Thr Leu
65                  70                  75                  80

tat ctt caa atg aac agc ctg aga act gag gac acg gct gtg tat tac      288
Tyr Leu Gln Met Asn Ser Leu Arg Thr Glu Asp Thr Ala Val Tyr Tyr
                85                  90                  95

tgt gcg aga gac agc cgt gca tac agc tat ggt tac ctc tac gtc ttt      336
Cys Ala Arg Asp Ser Arg Ala Tyr Ser Tyr Gly Tyr Leu Tyr Val Phe
            100                 105                 110

gac tac tgg ggc cag ggc acc ctg gtc acc gtc tcc tca ggt gga ggc      384
Asp Tyr Trp Gly Gln Gly Thr Leu Val Thr Val Ser Ser Gly Gly Gly
        115                 120                 125

ggt tca ggc gga ggt ggc tct ggc ggt ggc gga tcg cag tct gcc ctg      432
Gly Ser Gly Gly Gly Gly Ser Gly Gly Gly Gly Ser Gln Ser Ala Leu
    130                 135                 140

act cag cct gcc tcc gtg tct ggg tct cct gga cag tcg atc acc atc      480
Thr Gln Pro Ala Ser Val Ser Gly Ser Pro Gly Gln Ser Ile Thr Ile
145                 150                 155                 160

tcc tgc act gga acc agc aat gat gtt ggg agt tat aac ctt gtc tcc      528
Ser Cys Thr Gly Thr Ser Asn Asp Val Gly Ser Tyr Asn Leu Val Ser
                165                 170                 175

tgg tac caa caa cac cca ggc aaa gcc ccc aaa ctc ctg att tat gag      576

Trp Tyr Gln Gln His Pro Gly Lys Ala Pro Lys Leu Leu Ile Tyr Glu
        180                 185                 190

ggc agt aag cgg ccc tca ggg att tct aat cgc ttc tct ggc tcc aag        624
Gly Ser Lys Arg Pro Ser Gly Ile Ser Asn Arg Phe Ser Gly Ser Lys
    195                 200                 205

tct ggc aac acg gcc tcc ctg acc atc tct ggg ctc cag gct gag gac        672
Ser Gly Asn Thr Ala Ser Leu Thr Ile Ser Gly Leu Gln Ala Glu Asp
    210                 215                 220

gag gct gat tat tac tgc atg tca tat acg agc agt ggc act cct tat        720
Glu Ala Asp Tyr Tyr Cys Met Ser Tyr Thr Ser Ser Gly Thr Pro Tyr
225                 230                 235                 240

gtc ttc gga act ggg acc cag ctc acc gtt tta ggt gcg gcc gca            765
Val Phe Gly Thr Gly Thr Gln Leu Thr Val Leu Gly Ala Ala Ala
                245                 250                 255

<210>  37
<211>  255
<212>  PRT
<213>  E. coli

<400>  37

Glu Glu Val Gln Leu Val Glu Ser Gly Gly Asn Leu Val Gln Pro Gly
1               5                   10                  15

Gly Ser Leu Arg Leu Ser Cys Ala Ala Ser Gly Phe Thr Phe Ser Ser
            20                  25                  30

Tyr Ala Met Ser Trp Val Arg Gln Ala Pro Gly Lys Gly Leu Glu Trp
        35                  40                  45

Val Ser Ala Ile Ser Ala Ser Gly Gly Thr Thr Tyr Tyr Ala Asp Ser
    50                  55                  60

Val Lys Gly Arg Phe Thr Ile Ser Arg Asp Asn Ser Lys Asn Thr Leu
65                  70                  75                  80

Tyr Leu Gln Met Asn Ser Leu Arg Thr Glu Asp Thr Ala Val Tyr Tyr
                85                  90                  95

Cys Ala Arg Asp Ser Arg Ala Tyr Ser Tyr Gly Tyr Leu Tyr Val Phe
            100                 105                 110

Asp Tyr Trp Gly Gln Gly Thr Leu Val Thr Val Ser Ser Gly Gly Gly
        115                 120                 125

Gly Ser Gly Gly Gly Gly Ser Gly Gly Gly Gly Ser Gln Ser Ala Leu
    130                 135                 140

Thr Gln Pro Ala Ser Val Ser Gly Ser Pro Gly Gln Ser Ile Thr Ile
145                 150                 155                 160

Ser Cys Thr Gly Thr Ser Asn Asp Val Gly Ser Tyr Asn Leu Val Ser
                165                 170                 175

Trp Tyr Gln Gln His Pro Gly Lys Ala Pro Lys Leu Leu Ile Tyr Glu
            180                 185                 190

Gly Ser Lys Arg Pro Ser Gly Ile Ser Asn Arg Phe Ser Gly Ser Lys
        195                 200                 205

Ser Gly Asn Thr Ala Ser Leu Thr Ile Ser Gly Leu Gln Ala Glu Asp
    210                 215                 220

Glu Ala Asp Tyr Tyr Cys Met Ser Tyr Thr Ser Ser Gly Thr Pro Tyr
225                 230                 235                 240

Val Phe Gly Thr Gly Thr Gln Leu Thr Val Leu Gly Ala Ala Ala
                245                 250                 255

<210>  38
<211>  768
<212>  DNA
<213>  E. coli

<220>
<221>  CDS
<222>  (1)..(768)

<400>  38
gag gag gtg cag ctg gtg gag tct ggg gct gag gtg aag aag cct ggg      48
Glu Glu Val Gln Leu Val Glu Ser Gly Ala Glu Val Lys Lys Pro Gly
1               5                   10                  15

gcc tca gtg aga gtt tcc tgc cag gca tct gga tac aca ttc acc agg      96
Ala Ser Val Arg Val Ser Cys Gln Ala Ser Gly Tyr Thr Phe Thr Arg
            20                  25                  30

tac cat ata cac tgg gtg cga cag gcc cct gga caa ggg ctt gag tgg      144
Tyr His Ile His Trp Val Arg Gln Ala Pro Gly Gln Gly Leu Glu Trp
        35                  40                  45

atg gga gtg atc gac ccc aat agt ggt aga ata agt tac tca cag aag      192
Met Gly Val Ile Asp Pro Asn Ser Gly Arg Ile Ser Tyr Ser Gln Lys
    50                  55                  60

ttc cag gac aga gtc acc atg acc agg gac acg tcc acg agc aca gtc      240
Phe Gln Asp Arg Val Thr Met Thr Arg Asp Thr Ser Thr Ser Thr Val
65                  70                  75                  80

tac atg gag ctg aac agc ctg aga tct gag gac aca gcc att tat tac 288
Tyr Met Glu Leu Asn Ser Leu Arg Ser Glu Asp Thr Ala Ile Tyr Tyr
                85                  90                  95

tgt gcg aga gat cga gga tat tgt aat ggt ggc agg tgc ttt atg gat 336
Cys Ala Arg Asp Arg Gly Tyr Cys Asn Gly Gly Arg Cys Phe Met Asp
            100                 105                 110

gca ttt gac tac tgg ggc cag ggg acc acg gtc acc gtc tcc tca ggt 384
Ala Phe Asp Tyr Trp Gly Gln Gly Thr Thr Val Thr Val Ser Ser Gly
        115                 120                 125

gga ggc ggt tca ggc gga ggt ggc tct ggc ggt ggc gga tcg cag tct 432
Gly Gly Gly Ser Gly Gly Gly Gly Ser Gly Gly Gly Gly Ser Gln Ser
    130                 135                 140

gtg ttg acg cag ccg ccc tca gcg tct ggg acc ccc ggg cag agg gtc 480
Val Leu Thr Gln Pro Pro Ser Ala Ser Gly Thr Pro Gly Gln Arg Val
145                 150                 155                 160

acc atc gct tgt tct gga agc agc tcc aac atc gga att aat act gta 528
Thr Ile Ala Cys Ser Gly Ser Ser Ser Asn Ile Gly Ile Asn Thr Val
                165                 170                 175

aac tgg tac cag cag atc cca gga acg gcc ccc aaa ctc ctc atc tat 576
Asn Trp Tyr Gln Gln Ile Pro Gly Thr Ala Pro Lys Leu Leu Ile Tyr
            180                 185                 190

aat aat gat cag cgg ccc tca ggg gtc cct gac cga ttc tct ggc tcc 624
Asn Asn Asp Gln Arg Pro Ser Gly Val Pro Asp Arg Phe Ser Gly Ser
        195                 200                 205

aag tct gcc acc tca gcc tcc ctg gcc atc act ggg ctc cag gtt gac 672
Lys Ser Ala Thr Ser Ala Ser Leu Ala Ile Thr Gly Leu Gln Val Asp
    210                 215                 220

gat gag gct gat tat tac tgc cag tcc tat gac agc agc ctg ggt ggt 720
Asp Glu Ala Asp Tyr Tyr Cys Gln Ser Tyr Asp Ser Ser Leu Gly Gly
225                 230                 235                 240

tat gtc ttc gga act ggg acc cag ctc acc gtt tta ggt gcg gcc gca 768
Tyr Val Phe Gly Thr Gly Thr Gln Leu Thr Val Leu Gly Ala Ala Ala
                245                 250                 255

<210> 39
<211> 256
<212> PRT
<213> E. coli

<400> 39

Glu Glu Val Gln Leu Val Glu Ser Gly Ala Glu Val Lys Lys Pro Gly
1               5                   10                  15

Ala Ser Val Arg Val Ser Cys Gln Ala Ser Gly Tyr Thr Phe Thr Arg
            20                  25                  30

Tyr His Ile His Trp Val Arg Gln Ala Pro Gly Gln Gly Leu Glu Trp

35                  40                  45

Met Gly Val Ile Asp Pro Asn Ser Gly Arg Ile Ser Tyr Ser Gln Lys
    50                  55                  60

Phe Gln Asp Arg Val Thr Met Thr Arg Asp Thr Ser Thr Ser Thr Val
65                  70                  75                  80

Tyr Met Glu Leu Asn Ser Leu Arg Ser Glu Asp Thr Ala Ile Tyr Tyr
                85                  90                  95

Cys Ala Arg Asp Arg Gly Tyr Cys Asn Gly Gly Arg Cys Phe Met Asp
            100                 105                 110

Ala Phe Asp Tyr Trp Gly Gln Gly Thr Thr Val Thr Val Ser Ser Gly
        115                 120                 125

Gly Gly Gly Ser Gly Gly Gly Gly Ser Gly Gly Gly Gly Ser Gln Ser
    130                 135                 140

Val Leu Thr Gln Pro Pro Ser Ala Ser Gly Thr Pro Gly Gln Arg Val
145                 150                 155                 160

Thr Ile Ala Cys Ser Gly Ser Ser Ser Asn Ile Gly Ile Asn Thr Val
                165                 170                 175

Asn Trp Tyr Gln Gln Ile Pro Gly Thr Ala Pro Lys Leu Leu Ile Tyr
            180                 185                 190

Asn Asn Asp Gln Arg Pro Ser Gly Val Pro Asp Arg Phe Ser Gly Ser
        195                 200                 205

Lys Ser Ala Thr Ser Ala Ser Leu Ala Ile Thr Gly Leu Gln Val Asp
    210                 215                 220

Asp Glu Ala Asp Tyr Tyr Cys Gln Ser Tyr Asp Ser Ser Leu Gly Gly
225                 230                 235                 240

Tyr Val Phe Gly Thr Gly Thr Gln Leu Thr Val Leu Gly Ala Ala Ala
                245                 250                 255

<210>  40
<211>  765
<212>  DNA
<213>  E. coli

<220>
<221> CDS
<222> (1)..(765)

<400> 40
gag gag gtg cag ctg ttg cag tct ggg gga ggc gtg gtc cag cct ggg 48
Glu Glu Val Gln Leu Leu Gln Ser Gly Gly Gly Val Val Gln Pro Gly
1               5                   10                  15

agg tcc ctg aga ctc tcc tgt gca gcc tct gga ttc agc ttc agt aac 96
Arg Ser Leu Arg Leu Ser Cys Ala Ala Ser Gly Phe Ser Phe Ser Asn
            20                  25                  30

tat gtt atg cac tgg gtc cgc cag gct cca ggc aag ggg ctg gag tgg 144
Tyr Val Met His Trp Val Arg Gln Ala Pro Gly Lys Gly Leu Glu Trp
        35                  40                  45

gtg gca gtt ata tca tat gat gga agc aat aaa tac tac gca gac tcc 192
Val Ala Val Ile Ser Tyr Asp Gly Ser Asn Lys Tyr Tyr Ala Asp Ser
    50                  55                  60

gtg aag ggc cga ttc acc atc tcc aga gac aat tcc aag aac acg cta 240
Val Lys Gly Arg Phe Thr Ile Ser Arg Asp Asn Ser Lys Asn Thr Leu
65                  70                  75                  80

tat ctg caa atg aaa ggc ctg aga cct gag gac acg gct gtg tat tac 288
Tyr Leu Gln Met Lys Gly Leu Arg Pro Glu Asp Thr Ala Val Tyr Tyr
                85                  90                  95

tgt gcg aga agt agt ggc tgg tac ctt ctc ttt gat gct ttt gat atc 336
Cys Ala Arg Ser Ser Gly Trp Tyr Leu Leu Phe Asp Ala Phe Asp Ile
            100                 105                 110

tgg ggc caa ggg aca atg gtc acc gtc tct tca ggt gga ggc ggt tca 384
Trp Gly Gln Gly Thr Met Val Thr Val Ser Ser Gly Gly Gly Gly Ser
        115                 120                 125

ggc gga ggt ggc tct ggc ggt ggc gga tcg gat gtt gtg atg aca cag 432
Gly Gly Gly Gly Ser Gly Gly Gly Gly Ser Asp Val Val Met Thr Gln
    130                 135                 140

tct cca gac tcc ctg gct gtg tcg ctg ggc gag agg gcc acc atc aac 480
Ser Pro Asp Ser Leu Ala Val Ser Leu Gly Glu Arg Ala Thr Ile Asn
145                 150                 155                 160

tgc gag tcc agc cag agt gtt tta ttc agc tcc aac aat aag aac tac 528
Cys Glu Ser Ser Gln Ser Val Leu Phe Ser Ser Asn Asn Lys Asn Tyr
                165                 170                 175

tta gct tgg tac cag cag aaa cca gga cag cct cct aag ctg ctc att 576
Leu Ala Trp Tyr Gln Gln Lys Pro Gly Gln Pro Pro Lys Leu Leu Ile
            180                 185                 190

tac tgg gca tct acc cgg gaa tcc ggg gtc cct gac cga ttc agt ggc 624
Tyr Trp Ala Ser Thr Arg Glu Ser Gly Val Pro Asp Arg Phe Ser Gly
        195                 200                 205

agc ggg tct gag aca gat ttc act ctc acc atc agc agc ctg cag gct 672
Ser Gly Ser Glu Thr Asp Phe Thr Leu Thr Ile Ser Ser Leu Gln Ala
    210                 215                 220

gaa gat gtg gca gtt tat tac tgt cag caa tat tat agg att ccg tgg     720
Glu Asp Val Ala Val Tyr Tyr Cys Gln Gln Tyr Tyr Arg Ile Pro Trp
225                 230                 235                 240

acg ttc ggc caa ggg acc aaa gtg gat atc aaa cgt gcg gcc gca         765
Thr Phe Gly Gln Gly Thr Lys Val Asp Ile Lys Arg Ala Ala Ala
                245                 250                 255

<210>  41
<211>  255
<212>  PRT
<213>  E. coli

<400>  41

Glu Glu Val Gln Leu Leu Gln Ser Gly Gly Gly Val Val Gln Pro Gly
1               5                   10                  15

Arg Ser Leu Arg Leu Ser Cys Ala Ala Ser Gly Phe Ser Phe Ser Asn
            20                  25                  30

Tyr Val Met His Trp Val Arg Gln Ala Pro Gly Lys Gly Leu Glu Trp
        35                  40                  45

Val Ala Val Ile Ser Tyr Asp Gly Ser Asn Lys Tyr Tyr Ala Asp Ser
    50                  55                  60

Val Lys Gly Arg Phe Thr Ile Ser Arg Asp Asn Ser Lys Asn Thr Leu
65                  70                  75                  80

Tyr Leu Gln Met Lys Gly Leu Arg Pro Glu Asp Thr Ala Val Tyr Tyr
                85                  90                  95

Cys Ala Arg Ser Ser Gly Trp Tyr Leu Leu Phe Asp Ala Phe Asp Ile
            100                 105                 110

Trp Gly Gln Gly Thr Met Val Thr Val Ser Ser Gly Gly Gly Gly Ser
        115                 120                 125

Gly Gly Gly Gly Ser Gly Gly Gly Gly Ser Asp Val Val Met Thr Gln
    130                 135                 140

Ser Pro Asp Ser Leu Ala Val Ser Leu Gly Glu Arg Ala Thr Ile Asn
145                 150                 155                 160

Cys Glu Ser Ser Gln Ser Val Leu Phe Ser Ser Asn Asn Lys Asn Tyr
                165                 170                 175

Leu Ala Trp Tyr Gln Gln Lys Pro Gly Gln Pro Pro Lys Leu Leu Ile

            180                 185                 190

Tyr Trp Ala Ser Thr Arg Glu Ser Gly Val Pro Asp Arg Phe Ser Gly
        195                 200                 205

Ser Gly Ser Glu Thr Asp Phe Thr Leu Thr Ile Ser Ser Leu Gln Ala
    210                 215                 220

Glu Asp Val Ala Val Tyr Tyr Cys Gln Gln Tyr Tyr Arg Ile Pro Trp
225                 230                 235                 240

Thr Phe Gly Gln Gly Thr Lys Val Asp Ile Lys Arg Ala Ala Ala
                245                 250                 255

4